(19) Europäisches Patentamt
European Patent Office
Office européen des brevets

(11) EP 0 374 510 B1

(12) # EUROPEAN PATENT SPECIFICATION

(45) Date of publication and mention of the grant of the patent: **15.01.1997 Bulletin 1997/03**

(51) Int. Cl.$^6$: **A61K 39/395** // (A61K39/395, 31:395, 31:38, 31:675, 31:66)

(21) Application number: **89121661.6**

(22) Date of filing: **23.11.1989**

(54) **Products for the treatment of endotoxic shock in a mammal**

Produkte zur Behandlung des Endotoxin -Schocks bei einem Säugetier

Produits pour le traitement de choc endotoxique chez les mammifères

(84) Designated Contracting States:
**AT BE CH DE ES FR GB GR IT LI NL SE**

(30) Priority: **19.12.1988 US 286201**

(43) Date of publication of application: **27.06.1990 Bulletin 1990/26**

(73) Proprietor: **AMERICAN CYANAMID COMPANY**
**Wayne, NJ 07470-8426 (US)**

(72) Inventors:
- **Wissner, Allan**
  **Ardsley, NY 10502 (US)**
- **Kerwar, Suresh S.**
  **Ossining, NY 10562 (US)**
- **Kohler, Constance**
  **Old Tappan, NJ 07675 (US)**

(74) Representative: **Wächtershäuser, Günter, Prof. Dr. et al**
**Patentanwalt,**
**Tal 29**
**80331 München (DE)**

(56) References cited:
**US-A- 4 148 879**
- **JOURNAL OF IMMUNOLOGY, vol. 141, no. 11 01 December 1988, American Association of Immunologists, US; F.H. VALONE et al., pp. 3945-3950**

EP 0 374 510 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

This invention relates to products containing, in therapeutically effective amounts, Platelet Activating Factor antagonist and one or more monoclonal or polyclonal antibodies directed to either Tumor Necrosis Factor α, interleukin-Iβ, gamma interferon or bacterial cell wall lipopolysaccharide/s as a combined preparation for simultaneous, separate or sequential use in the treatment of endotoxic shock in mammals.

DETAILED DESCRIPTION OF THE INVENTION

Platelet Activating Factor (PAF) is a potent mediator of inflammation in mammals and it is synthesized by a variety of cells including macrophages, neutrophils, endothelial cells and platelets (Cammussi, G. 1986, Kidney Int. 29, 469). When injected into mammals, PAF induces hemodynamic and hematological changes including hypotension, platelet aggregation, neutropenia, disseminated intravascular coagulation, increases in vascular permeability, bronchoconstriction, tissue injury (hypoxia and necrosis) and eventually death (reviewed by Cammussi, G. Kidney Int. 29, 469, 1986). In recent years, it has been postulated that PAF is the mediator of tissue injury in mammals undergoing endotoxic shock due to bacterial sepsis (Terashita, Z., Y. Imura, K. Nishikawa and S. Sumida 1985, Eur. J. Pharmacol. 109:257-261; Doebber, T. W., M.S.Wu, J. C. Robbins, B. M. Choy, M. N. Chang and T. Y. Shen 1985, Biochem. Biophys. Res. Comm 127:799-808; Inarrea, P., Gomez-Cambronero, J. Pascual, M. del Carmen Ponte, L. Hernando and M. Sanchez-Crespo. 1985, Immunopharmacology, 9:45-52). These studies, in mammals, have shown that PAF is produced in large amounts when the mammal has been treated with endotoxin. In addition, mammals undergoing endotoxic shock exhibit all of the clinical symptoms associated with the administration of PAF.

Studies (Tracey, K. J., B. Beutler, S. F. Lowry, J. Merryweather, S. Wolpe, I. W. Milsark, R. J. Hairi, T. J. Fahey, A. Zentalla, J. D. Albert and A. Cerami 1986, Science (Wash., D.C. 234;47-473) have shown that the clinical symptoms associated with endotoxic shock or with PAF can be reproduced in mammals by the administration of the macrophage cytokine, Tumor Necrosis Factor α (TNFα; also known as cachectin). Since macrophages can be activated in vitro and in vivo, to synthesize and secrete TNFα (Carswell, E. A., L. J. Old, R. L. Kassel, S. Green, D. Fiore and B. Williamson 1975, Proc. Natl. Acad. Sci. USA 72;:3666-3671), it has been suggested that TNFα may be one of the mediators synthesized after endotoxin shock. Evidence supporting this claim is derived from the studies of Beutler et al. (Beutler, B., I. W. Milsark and A. C. Cerami 1985, Science (Wash. D.C.) 229;869-872). These studies have shown that if mice are pretreated with polyclonal antisera directed against TNFα (antiTNFα), they were protected from the lethal effects of endotoxin. However, if antiTNFα was administered at the time of endotoxin treatment or if antiTNFα was administered after endotoxin treatment, no significant protection was detected. Thus, the time of administration of antiTNFα was critical in the protection against endotoxin induced lethality.

Recent studies by Camussi et al. (Camussi, G., F. Bussolino, G. Salvidio and C. Baglioni 1987, J. Exp. Med. 166, 1390-1404) have shown that when TNFα is added to cultures of rat peritoneal macrophages, neutrophils or human endothelial cells, large amounts of PAF are synthesized and released. These observations indicate that the clinical symptoms associated with the administration of TNFα to mammals are due in part to PAF that is synthesized by a variety of cells when exposed to TNFα.

Since endotoxin shock is initiated by the cell wall lipopolysaccharide (LPS) a monoclonal or a polyclonal antibody directed against LPS in combination with a PAF antagonist can also be a desired form of treatment for endotoxic shock in mammals.

Since macrophages can be activated by gamma interferon (A. G. Morris, Interferons, Immunology 1988 Supplement 1,43-45), a monoclonal or polyclonal antibody directed to human gamma interferon in combination with a PAF antagonist can also be a desired form of treatment for endotoxic shock in mammals.

Another cytokine that is synthesized and released by macrophages exposed to endotoxin is interleukin-1β (IL-1β). Studies conducted by Okusawa et al. (S. Okusawa, J. A. Gelfand, T. Ikejima, R. J. Connolly and C. A. Dinarello, 1988, Clin. Invest. 81, 1162-1172) have shown that the administration of IL-1β to mammals induces a shock like state (hemodynamic and hematological changes) that is similar to that seen when TNFα is administered. When IL-1β was combined with TNFα, the shock syndrome in rabbits was of a greater intensity than if either agent was used alone. Thus, it is likely that IL-1β is an additional mediator that can induce the synthesis and release of PAF by a variety of cells.

Thus, endotoxin shock due to bacterial sepsis involves the synthesis and release of IL-1β and TNFα by macrophages. The released IL-1β and TNFα can interact with a variety of cells such as macrophages, endothelial cells, neutrophils and platelets to stimulate the synthesis and release of PAF. The released PAF induces hemodynamic and hematological changes, causes tissue damage (hypoxia), increased vascular permeability and organ failure. Due to these induced changes in the mammal, PAF is lethal.

Concurrently, with the realization that PAF is an important mediator of the clinical effects in mammals undergoing endotoxic shock, a number structurally different antagonists of PAF have been developed. References to some of these antagonists are listed hereinbelow.

Terashita, z., et. al., Life Sci. 32, 1975 (1983).
Miyamoto, T., et. al., In Advances in Prostaglandin, Thromboxane and Leukotriene Research, Vol 15 pp. 719-720, Raven Press, New York.
Barner, R., et. al., Eur. patent 147768 (10.07.1985), 41 pp.
Burri, K., et. al., Prostaglandins 30, 691 (1985).
Handley, D.A., et al., Proceedings of the first Sandoz Research Symposium. "New Horizons in Platelet-activating Factor", pp 335-342, J. Wiley, London, 1987.
VanValen, R.G., et al., ibid., pp 123-130.
Winslow, C.M., et. al., ibid., pp 152-164.
Lee, M. L., et. al., ibid.
Handley, D.A., et. al., Immunopharmacology 11, 175-182 (1986).
Winslow, C.M. et. al., Prostaglandins 30m 697 (1985).
Steiner, M., et. al., Biochem. Biophys. Res. Commun. 133, 851 (1985).
Wichrowski, B., et. al., In Sixth Int's Conf. on Prostaglandins and Related Compounds, Florence, Italy, June 3-6, 1986, p. 309.
Lee, M.L., et. al., Prostaglandins 30, 690 (1985).
Jaeggi, C., et. al., Eur. patent appl. EP 178,261 (April 16, 1986), U.S. Appln. 659,249 (Oct. 10, 1984).
Stenzel, H., et. al., In Third Int'l. Symposium, Davos, Switzerland, Sept. 7-9, 1986, p 50.
Braquet, P., In Adv. in Prostaglandin, Thromboxane and Leukotriene Res., Vol. 16, pp. 179-198, Raven Press, New York, 1986.
Dupont, L., et. al., Actz. Crystallog. C42:1759 (1986).
Braquet, P., GB Patent 84/18424 (July 19, 1984), Belg. BE 901,915.
Braquet, P., et. al., Blood Vessels 16, 559 (1985).
Korth, R., et. al., Eur. J. Pharmacol., in press, 1987.
Kuster, L.J., et. al., Thromb. Res. 43:425 (1986).
Nunez, D., et. al., Eur. J. Pharmacol. 123: 197 (1986).
Baroggi, N., et. al., Agents Actions (suppl.) 20, 87 (1986).
Simon, A.F., et. al., Thromb. Res. 1987.
Chung, K.F., et. al., Lancet 2, 248 (1987).
Rao, C.B.S.:Chemistry of LIgnans, Anhdra Univ. Press, India, p. 377, 1978.
Godfroid, J.J., et. al., J. Med. Chem. 30:792-797, 1987.
Comez-Cambrenero, J., et. al., Biochem. Biophys. Acta 845:511-515, 1985.
Hwang, S.B., et. al., J. Biol. Chem. 261:13720 (1986).
Ponpipom, M.M., et. al., J. Med. Chem. 30, 136 (1987).
Shen, T.Y., et. al., Proc. Natl. Acad. Sci. USA 82:672 (1985).
Hwang, S.B., et. al., Lab. Invest. 52:617 (1985).
Doebber, T.W., et. al., Biochem. Biophys. Res. Commun. 29:799 (1985).
Biftu, T., et. al., Eur. Patent Appln. EP 154887 (18.091985), 34 pp.
Biftu, T., et. al., J. Med. Chem. 29:1917 (1986).
Braquet, P., et. al., Trends Pharmacol. Sci. 7:397 (1986).
Hwang, S.B., et. al., J. Biol. Chem. 260:15639 (1985).
Shen, T.Y., et. al., Second World Conf. on Inflammation, Monte Carlo, 1986.
Shen, T.Y., et. al., Second Int'l. Conf. on Platelat-activating Factor and Structurally related Alkyl Ether Lipids, Gatlinburgh, TN, Oct. 1986, p. 34.
Setchell, K.D., et. al., Nature (Lond) 287:740 (1980).
Stitch, S.R., et. al., Nature (Lond) 287:738 (1980)
Okamoto, M., et. al., J. Antiobiot. (Tokyo) 39:198 (1986).
Okamoto, M., et. al., Chem. Pharm. Bull. (Tokyo) 34:345 (1986).
Okamoto, M., et. al., ibid, 34:340 (1986).
Lefort, J., et. al., Eur. J. Pharmacol., in press, 1987.
Sedivy, P., et. al., Adv. in Inflammation Res., vol. 10 pp. 171-173, Raven Press, New York, 1985.
Sedivy, P., et. al., Prostaglandins 30:688 (1985).
Cavero, L., et. al., Br. J. Pharmacol. 90:116 (1987).
Hwang, S.B., et. al., Thromb. Res. 34:519 (1984) Kornecki, E., et. al., Science (Wash., D.C.) 226:1454 (1985).
Casais-Stenzel, J., et. al., Ger. Offen. DE 3435974 (10.041986), 22 pp.
Casals-Stenzel, J., et. al., Ger. Offen. DE 3435973 (10.04.1986), 26 pp.
Casals-Stenzel, J., et. al., Ger. Offen. DE 3435972A1 (10.04.1986), 24 pp.
Weber, K.H. et. al., Second Int'l. Conf. on Platelet Activating Factor and Structurally Related Alkyl Ether Lipids,

Gatlinburg, TN, Oc. 1986, p. 29.
Tuffin, D.P., et. al., Prostaglandins 30:702 (1985).
Hwang, M.N., et. al., Eur. J. Pharmacol., in press, 1987.
Hadvary, P., et. al., Helv. Chimica Acta 69, 1862 (1986).
Kagari, F., et. al., Chem. Pharm. Bull. 35, 647 (1987).
Grue-Sorensen, G., et. al., submitted J. Org. Chem., 1987.
Bittman, R., et. al., J. Lipid Res., 28, 733 (1987).
Shimazaki, N., et. al., J. Med. Chem. 30, 1706 (1987).
O'Flaherty, J.T., et. al., Biochem. and Biophys. Res. Commun. 147, 18 (1987).
Bitterman, H., et. al., Meth and Find Exptl. Clin. Pharmacol. 9, 341 (1987).
Hwang, S., et. al., Eur. J. Pharmacol. 141, 269 (1987).

Other PAF antagonists useful in our invention are of the formula: wherein:

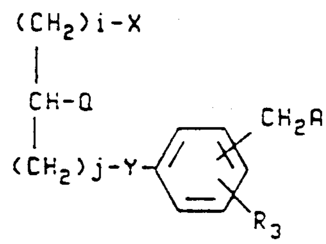

Formula II

(A) X is selected from the group consisting of

(i) $C_1$-$C_{24}$ alkyl;
(ii) $C_1$-$C_{24}$ alkoxy;
(iii) $C_1$-$C_{24}$ carboamoyloxy;

(iiii) 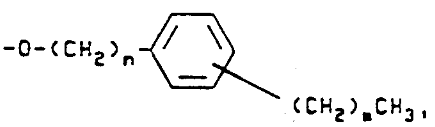 , 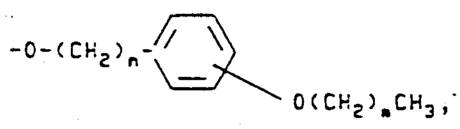 ,

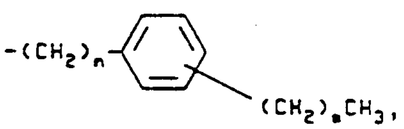 , 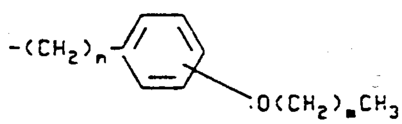

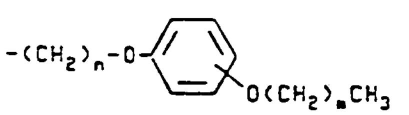 , 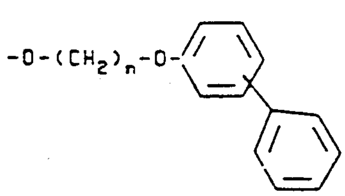

and 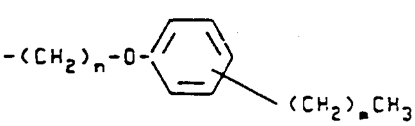

wherein n is an integer from 1 to 25 and m is an integer from 0 to 24 and the sum of n and m is less than or equal to 25;

(v) phenyl;

(vi) mono-or polysubstituted phenyl wherein the substituents are selected from the group consisting of $C_1$-$C_{20}$ alkyl, $C_1$-$C_{20}$ alkoxy, halogen, trifluoromethyl, phenyl, substituted phenyl and benzyloxy;

(vii) phenoxy;

(viii) mono-or polysubstituted phenoxy wherein the substituents are selected form the group consisting of $C_1$-$C_{20}$ alkyl, $C_1$-$C_{20}$ alkoxy, halogen, trifluoromethyl, phenyl, substituted phenyl and benzyloxy;

(ix) naphthaloxy;

(x) mono-or polysubstituted naphthaloxy wherein the substituents are selected from the group consisting of $C_1$-$C_{20}$ alkyl, $C_1$-$C_{20}$ alkoxy and halogen;

(xi) $-0-(CH_2)_r-O-((CH_2)_pO)_t-(CH_2)_a-W$

wherein W is selected from the group consisting of methyl and phenyl optionally substituted with $C_1$-$C_3$ alkyl, $C_1$-$C_3$ alkoxy and phenyl, r, p, t and a are integers such that the expression $r+(p+1)t+a$ is also an integer and has a value of 3 to 20; r is greater than or equal to 2; p is greater than or equal to 2; t is greater than or equal to zero; and a is greater than or equal to zero;

(B) i is an integer from 1 to 3 and j is an integer from 1 to 6;

(C) Q is selected from the group consisting of $-OR_2$,

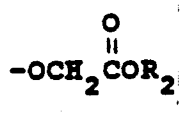 and 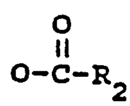,

wherein $R_2$ is hydrogen, $C_1$-$C_6$ alkyl or $C_1$-$C_6$ alkenyl;

(D) Y is a divalent radical selected from the group consisting of

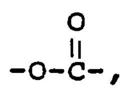,

$-OCH_2-$, and

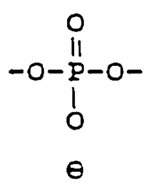;

(E) the moiety $R_3$ represents one or more substituents of the aromatic ring selected from the group consisting of $C_1$-$C_5$ alkyl, $C_1$-$C_5$ alkoxy and halogen;

(F) the moiety $-CH_2A$ represents a substituent of the aromatic ring which may be in the <u>ortho</u>, <u>meta</u> or <u>para</u> position wherein A is selected from the group consisting of:

(i) a 5-7 member aromatic heterocyclic ring containing at least one nitrogen atom and optionally one or more nitrogen or sulfur atoms;

and PAF antagonists of the formula:

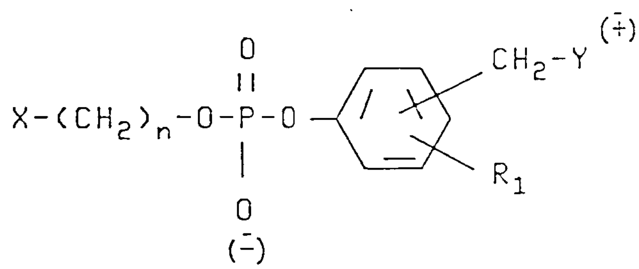

Formula I

wherein:

(A) X is a phenyl or naphthyl ring optionally substituted in any position with one or more substituents

(i) $-R_2$, wherein $R_2$ is $C_1$-$C_{25}$ alkyl, $C_1$-$C_{25}$ alkenyl, $C_1$-$C_{25}$ alkoxy, $C_1$-$C_{25}$ alkenyloxy, $C_1$-$C_{25}$ thioalkyl, phenyl, phenoxy, substituted phenyl or substituted phenoxy wherein the substituents are $C_1$-$C_{20}$ alkyl, $C_1$-$C_{20}$ alkoxy, halogen or trifluoromethyl;
(ii) hydrogen, halogen, trifluoromethyl, cyano, or nitro;
(iii) $-CO_2R_3$, $-CONHR_3$, -CHO, $-OCONHR_3$, or $-NHCOR_3$ wherein $R_3$ is $C_1$-$C_{25}$ alkyl, $C_1$-$C_{25}$ alkenyl, phenyl, or substituted phenyl, wherein the substituents are $C_1$-$C_{20}$ alkyl, $C_1$-$C_{20}$ alkoxy, halogen, or trifluoromethyl;

(B) $R_1$ represents one or more substituents of the aromatic ring which may be in any position and is $C_1$-$C_5$ alkyl, $C_1$-$C_5$ alkoxy or halogen.

$-CH_2$-Y represents a single substituent of the aromatic ring which may occupy any position wherein Y is a mono or bicyclic aromatic heterocycle with 5-7 membered rings containing at least one nitrogen atom which is bonded to the methylene group and optionally one or more other nitrogen or sulfur atoms: and (C) n is the integer 0 or 1.

A preferred embodiment is compounds of formula I, above, wherein Y is

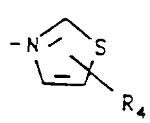 , 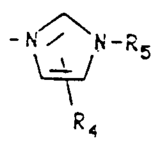 , 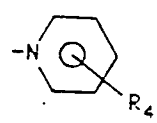

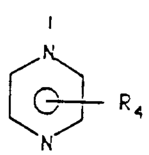 , 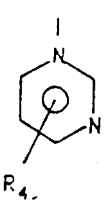 , 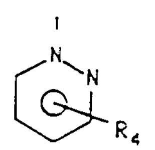

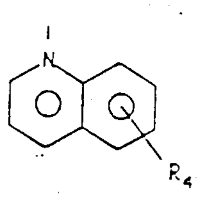 , 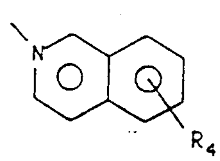

wherein $R_4$ represents one or more substituents of the heterocyclic ring which may occupy and non-hetero atom position and is $C_1$-$C_5$ alkyl, $C_1$-$C_5$ alkoxy, hydrogen or halogen; the moiety $R_5$ is $C_1$-$C_5$ alkyl or hydrogen.

Our invention relates to the unexpected discovery that when a PAF antagonist is administered in combination with a monoclonal or polyclonal antibody directed toward either Tumor Necrosis Factor α, Interleukin-1β, gamma interferon,

or bacterial cell wall lipopolysaccharides, the animals can be protected from the effects of endotoxin in a manner better than if the PAF antagonist or the antibody are administered alone; this combination therapy represents a new method for the treatment of endotoxic shock in mammals.

Antibody directed toward Tumor Necrosis Factor α can be obtained from Endogen Inc., 451 D Street, Boston, MA 02210. Antibody directed toward Interleukin-1β can be obtained from Genzyme, 75 Kneeland St., Boston, MA 02114. Antibody directed toward gamma interferon can be obtain from Alpha Therapeutic Corp., 5555 Valley Road, Los Angeles, CA 90032. Antibody directed toward bacterial cell wall lipopolysaccharides can be obtained from Oxoid Ltd., Wade Road, Basingstoke, Hampshire, RG 24, OPN, England.

The PAF antagonists used to implement the present invention may be orally administered, for example, with an inert diluent, or with an assimilable edible carrier, or they may be enclosed in hard or soft shell capsules, or they may be compressed into tablets, or they may be incorporated directly with the food of the diet. For oral therapeutic administration, these antagonists may be incorporated with excipients and used in the form of tablets, capsules, elixirs, suspensions, syrups and the like. Such compositions and preparations should contain at least 0.1% of active compound. The percentage of the compound in these compositions may, of course, be varied and may conveniently be between about 2% to about 60% of the weight of the unit. The amount of active compound in such therapeutically useful compositions is such that a suitable dosage will be obtained. Preferred compositions according to this invention are prepared so that an oral dosage unit contains between about 1 and 500 mg of active compound.

The tablets, capsules and the like may also contain a binder such as gum tragacanth, acacia, corn starch or gelatin; excipients such as dicalcium phosphate; a disintegrating agent such as corn starch, potato starch, alginic acid; a lubricant such as magnesium stearate; and a sweetening agent such as sucrose, lactose or saccharin. When the dosage unit form is a capsule, it may contain, in addition to materials of the above type, a liquid carrier such as a fatty oil.

Various other materials may be present as coatings or to modify the physical form of the dosage unit. For instance, tablets may be coated with shellac, sugar or both. A syrup or elixir may contain, in addition to active ingredient, sucrose as a sweetening agent, methyl and propylparabens as preservatives, a dye and flavoring such as cherry or orange flavor.

These PAF antagonists may also be administered parenterally. Solutions or suspensions of these active compounds can be prepared in water suitably mixed with a surfactant such as hydroxypropylcellulose. Dispersions can also be prepared in glycerol, liquid polyethylene glycols and mixtures thereof in oils. Under ordinary conditions of storage and use, these preparations contain a preservative to prevent the growth of microorganisms.

The pharmaceutical forms of PAF antagonists suitable for injectable use include sterile aqueous solutions or dispersions and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (e.g. glycerol, propylene glycol and liquid polyethylene glycol), suitable mixtures thereof, and vegetable oils.

The PAF antagonists of this invention may also be administered directly to the airways in the form of an aerosol.

The monoclonal or polyclonal antibodies directed towards Tumor Necrosis Factor α (also known as Cachectin), Interleukin-1β, Gamma Interferon, and bacterial cell wall lipopolysaccharides which are needed to implement this invention and are best administered parenterally in a dosage range of 2 to 500 mg/kg.

Solutions or suspensions of these antibody compounds can be prepared in water suitably mixed with a surfactant such as hydroxypropylcellulose. Dispersions can also be prepared in glycerol, liquid polyethylene glycols and mixtures thereof in oils. Under ordinary conditions of storage and use, these preparations contain a preservative to prevent the growth of microorganisms.

The pharmaceutical forms of the antibodies suitable for injectable use include sterile aqueous solutions or dispersions and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, saline, water, ethanol, polyol (e.g. glycerol, propylene glycol and liquid polyethylene glycol), suitable mixtures thereof, and vegetable oils.

The PAF antagonists and antibodies may be administered in the same vehicle or different vehicles. The PAF antagonists and antibodies may be administered at the same time or at different times during the course of therapy.

The invention will be further described by the following examples.

Example 1

Balb/c mice (male, 20 g) were obtained from Charles River Laboratories, Wilmington, Massachusetts. They were housed in groups of 5 in individual cages and fed ad libitum. E. coli endotoxin was obtained from Sigma Chemical Co., St. Louis, MO. The endotoxin was dissolved in Phosphate buffered saline (GIBCO) and administered to mice intravenously at a dose of 50 mg/kg. Fifteen to twenty minutes after the administration of endotoxin, the mice were divided into

several groups. One group received intravenous saline (Control). The second group received 3-[[3-[[hydroxy[2-methoxy-3-(tetradecyloxy)phenoxy]-phosphinyl]oxy]phenyl]methyl]-5-methyl-thiazolium, hydroxide, inner salt (PAF antagonist) intravenously at a dose of 20 mg/kg (Compound suspended in phosphate buffered saline and sonicated for approximately 15 seconds to provide a flocculent or cloudy solution). The third group received a monoclonal antibody directed against murine TNFα administered intravenously at a dose of 25 mg/kg. The fourth group received intravenously 3-[[3-[[hydroxy[2-methoxy-3-(tetradecyloxy)phenoxy]-phosphinyl]oxy]phenyl]methyl]-5-methyl-thiazolium, hydroxide, inner salt (20 mg/kg) and the monoclonal antibody directed against murine TNFα (25 mg/kg). The monoclonal antibody used in these studies was provided by Dr. Robert Schreiber, Department of Pathology, Washington University School of Medicine, St. Louis, MO and Cell-Tech, Great Britain. The mice were monitored for survival for up to 72 hours post endotoxin treatment. The results of these studies are shown in Table I.

Control mice that had received saline died within 24 hours after endotoxin challenge. Consistent with the observations of Beutler et al. (see reference above), mice that had received endotoxin followed by antiTNFα (antiTNFα administered 15 to 20 minutes after endotoxin) were not protected. By 72 hours, all of the mice had succumbed to the lethal effects of endotoxin. Mice treated with 3-[[3-[[hydroxy[2-methoxy-3-(tetradecyloxy) phenoxy]-phosphinyl]oxy]phenyl]methyl]-5-methyl-thiazolium, hydroxide, inner salt were also not protected from lethality induced by endotoxin. However, mice that had received both 3-[[3-[[hydroxy[2-methoxy-3-(tetradecyloxy)phenoxy] phosphinyl]oxy]phenyl]methyl]-5-methyl-thiazolium, hydroxide, inner salt and antiTNFα were completely protected from endotoxin induced lethality. No mortality was observed in this group at 24 hours or 72 hours post endotoxin treatment. They appeared healthy and for up to 13 days post endotoxin treatment. The experiment was terminated at this time.

## Example 2

Several Balb/c mice were treated intravenously with either 3-[[3-[[hydroxy[2-methoxy-3-(tetradecyloxy)phenoxy]phosphinyl]oxy]phenyl]methyl]-5-methyl-thiazolium, hydroxide, inner salt (20 mg/kg) or with saline. After two hours, all of these mice were treated intravenously with endotoxin at a dose of 50 mg/kg. One hour after endotoxin treatment, the mice were divided into several groups. Some mice that had received saline followed by endotoxin were treated intravenously with various doses of antiTNFα (doses ranging from 2.5 to 25 mg/kg). Some mice that had been treated with 3-[[3-[[hydroxy[2-methoxy-3-(tetradecyloxy)phenoxy]-phosphinyl]oxy]phenyl]methyl]-5-methyl-thiazolium, hydroxide, inner salt followed by endotoxin were also treated intravenously with various doses of antiTNFα (doses ranging from 2.5 to 25 mg/kg). Two types of controls were included. One control consisted of mice that were treated with 3-[[3-[[hydroxy[2-methoxy-3-(tetradecyloxy)phenoxy]-phosphinyl]oxy]phenyl]methyl]-5-methyl-thiazolium, hydroxide, inner salt followed by endotoxin and saline (saline administered 1 hour post endotoxin treatment). Another control consisted of mice treated with saline followed by endotoxin and saline (saline administered 1 hour post endotoxin). The mice were monitored for 48 hours. The results of these experiments are shown in Table II.

Control mice that had been pretreated either with 3-[[3-[[hydroxy[2-methoxy-3-(tetradecyloxy)phenoxy]-phosphinyl]oxy]phenyl]methyl]-5-methyl-thiazolium, hydroxide, inner salt or saline followed by endotoxin died within 24 or 48 hours. All mice treated with saline followed by endotoxin and various doses of antiTNFα (antiTNFα administered 1 hour post endotoxin treatment) also died within 48 hours. The only survivors at 48 hours (five out of ten treated mice survived) belonged to the group that had been treated with 3-[[3-[[hydroxy[2-methoxy-3-(tetradecyloxy)phenoxy]-phosphinyl]oxy]phenyl]methyl]-5-methyl-thiazolium, hydroxide, inner salt followed by endotoxin and anti-TNFα (25 mg/kg). These observations indicate that mammals can be pretreated in a prophylactic manner with a PAF antagonist followed by antiTNFα to prevent any potential endotoxin toxicity.

Table I

| Combination Effect of 3-[[3-[[hydroxy[2-methoxy-3-(tetradecyloxy)phenoxy]-phosphinyl]oxy]phenyl]methyl]- 5-methyl-thiazolium, hydroxide, inner salt (PAF antagonist)[1] and antiTNFα on Endotoxin Induced Lethality | | | |
|---|---|---|---|
| Treatment | Alive/Treated | | |
| | 24 hours | 48 hours | 72 hours |
| TDOPT (PAF antagonist) 20 mg/kg 20' after LPS | 0/5 | 0/5 | 0/5 |
| TDOPT (PAF antagonist) (20 mg/kg) + antiTNF (25 mg/kg) 20' after LPS | 5/5 | 5/5 | 5/5[2] |
| AntiTNF (25 mg/kg) 20' after LPS | 2/5 | 2/5 | 0/5 |
| PBS Control 20' after LPS | 0/5 | 0/5 | 0/5 |
| Drug and antibody were given by i.v. route at indicated doses 15-20' after 50 mg/kg i.v. endotoxin in male Balb/c mice. | | | |

1 3-[[3-[[hydroxy[2-methoxy-3-(tetradecyloxy)phenoxy]-phosphinyl]oxy]phenyl]methyl]-5-methyl-thiazolium, hydroxide, inner salt (PAF antagonist) will be called
TDOPT (PAF antagonist).
2 Still 5/5 by day 12.

**Table II**

**Prophylaxis Protocol: PAF Antagonist 3-[[3-[[hydroxy[2-methoxy-3-(tetradecyloxy)phenoxy]-phosphinyl]oxy]-phenyl]methyl]-5-methyl-thiazolium, hydroxide, inner salt 20 mg/kg (iv) and anti-TNFα (iv)[1]**

| TDOPT or PBS(iv) | LPS (iv) | antiTNFα (iv) TDOPT(iv) | | observation period | |
|---|---|---|---|---|---|
| HOURS | | | | | |
| | treatment | | | live/treated | |
| 0 hrs. | 2 hrs. | 3 hrs. | 24 hrs. | 48 hrs. | 72 hrs. |
| PBS | LPS | PBS | 0/20 | | |
| TDOPT | LPS | PBS | 1/10 | 0/10 | |
| TDOPT | LPS | TDOPT | 0/10 | | |
| PBS | LPS | antiTNFα (500) | 0/10 | | |
| | | (250) | 2/10 | 0/10 | |
| | | (100) | 0/10 | | |
| | | ( 50) | 0/10 | | |
| TDOPT | LPS | antiTNFα (500) | 6/10 | 5/10 | 5/10 |
| | | (250) | 3/10 | 0/10 | |
| | | (100) | 4/10 | 0/10 | |
| | | ( 50) | 2/10 | 0/10 | |

1 The (PAF antagonist) will be called TDOPT (PAF antagonist) in the above table

The preparation of compounds of this invention encompassed by formulas IA-C is described hereinbelow in Flowsheet A wherein X, i, j, $R_3$, $R_6$ and A are as described hereinabove; $Q^1$ is selected from the group

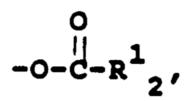

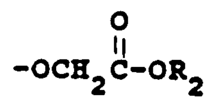

and $-OR^1_2$, wherein $R^1_2$ is $C_1$-$C_6$ alkyl or $C_1$-$C_6$ alkenyl; the moieties $-CH_2$-J and $-CH_2$-$A^1$ are substituents of the aromatic ring coming off the meta and para positions, wherein $A^1$ is a nitrogen containing heterocycle, a sulfide, thiourea, or a phosphine selected from the group:

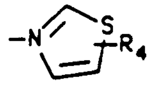 , 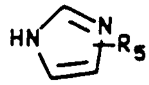 , 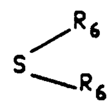 , 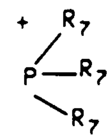 ,

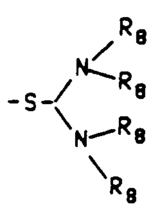 , 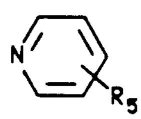 , 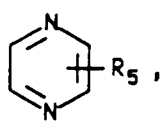 , 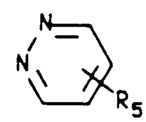 ,

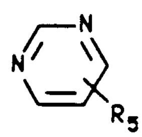 , 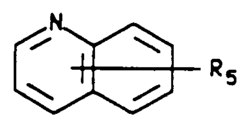

and 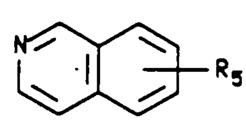

wherein $R_4$ represents one or more substituents of the heterocyclic ring which may occupy any non-hetero atom position and is selected from the group consisting of $C_1$-$C_5$ alkyl, $C_1$-$C_5$ alkoxy, hydrogen, halogen and $-CH_2CH_2OH$; $R_5$ represents one or more substituents of the heterocyclic ring which may occupy any non-hetero atom position and is selected from the group consisting of $C_1$-$C_5$ alkyl, $C_1$-$C_5$ alkoxy, hydrogen, and halogen; $R_6$ is $C_1$ to $C_5$ alkyl; $R_7$ is $C_1$-$C_5$ alkyl, phenyl and substituted phenyl $R_8$ is hydrogen or $C_1$-$C_6$ alkyl and J is a leaving group such as a chlorine, bromine or iodine atom.

According to the sequence of reactions outlined in Flowchart A, the alcohol 2 is treated with an equivalent of the phosphorus reagent 3 in the presence of a base such as triethylamine in an inert solvent such as carbon tetrachloride to give, after hydrolysis of the resulting intermediate in a buffered solvent system such as tetrahydrofuran-water-sodium acetate, the phosphate 4. The reaction of 4 with a large excess of a nitrogen containing heterocycle, a sulfide, thiourea, or a phosphine in an inert solvent such as toluene at 50°-150°C gives the compounds of this invention represented by formula IA. These compounds are obtained as internal salts except when A is an imidazole moiety.

In those cases where the compounds contain an imidazole ring (Formula IB), the imidazole moiety can be further

alkylated with an excess of alkyl halide by heating in an inert solvent such as toluene to give the internal salts IC.

**Flowsheet A**

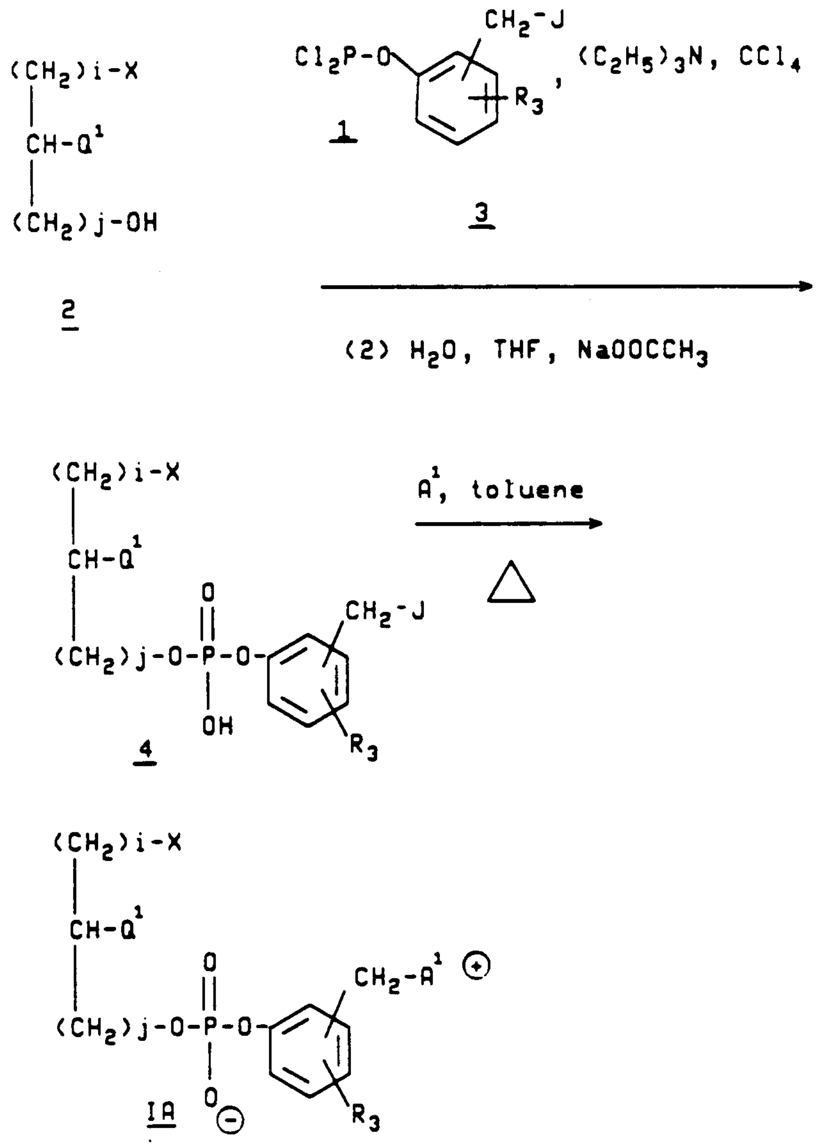


2 3 4 IA

**Flowchart A (continued)**

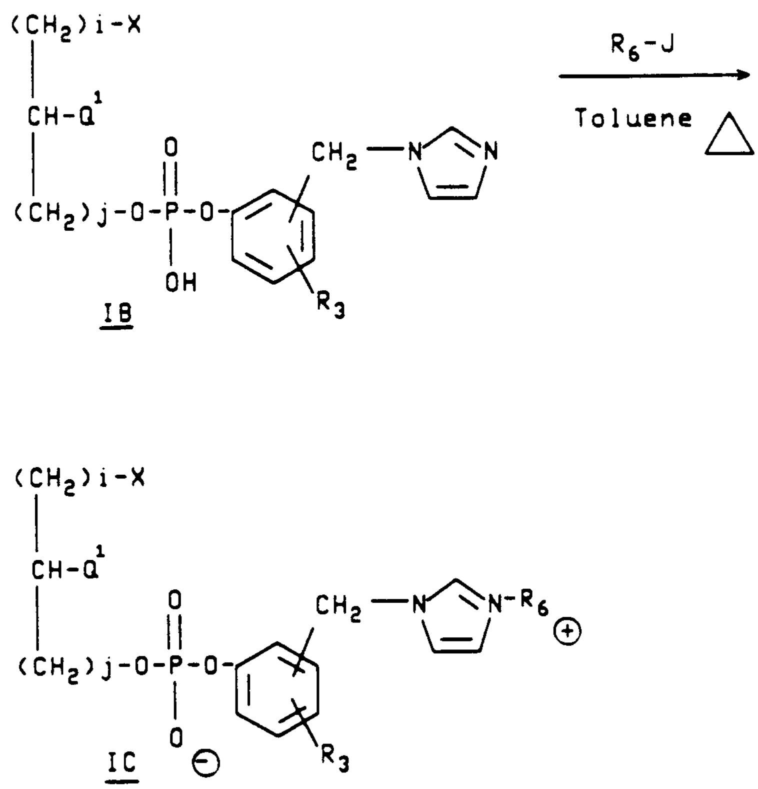


IB

IC

The preparation of compounds of this invention encompassed by formulas ID-IF is described hereinbelow in Flowchart B wherein X, i, j, $R_3$, $R_6$, A, $A^1$, $Q^1$ and J are as defined hereinabove.

According to the sequence of reactions outlined in Flowchart B, the alcohol 2 is treated with and equivalent of the phosphorus reagent 5 in the presence of a base such as triethylamine in an inert solvent such as carbon tetrachloride to give the cyclic phosphate 6. The reaction of 6 with a large excess of a nitrogen containing heterocycle, a sulfide, thiourea or a phosphine in an inert solvent such as toluene at 50°-150°C gives the compounds of this invention represented by formula ID. These compounds are obtained as internal salts except when A is an imidazole moiety. In those cases where the compounds contain an imidazole ring (formula IE), the imidazole moiety can be further alkylated with an excess of alkyl halide by heating in an inert solvent such as toluene to give the internal salts IF.

**Flowsheet B**

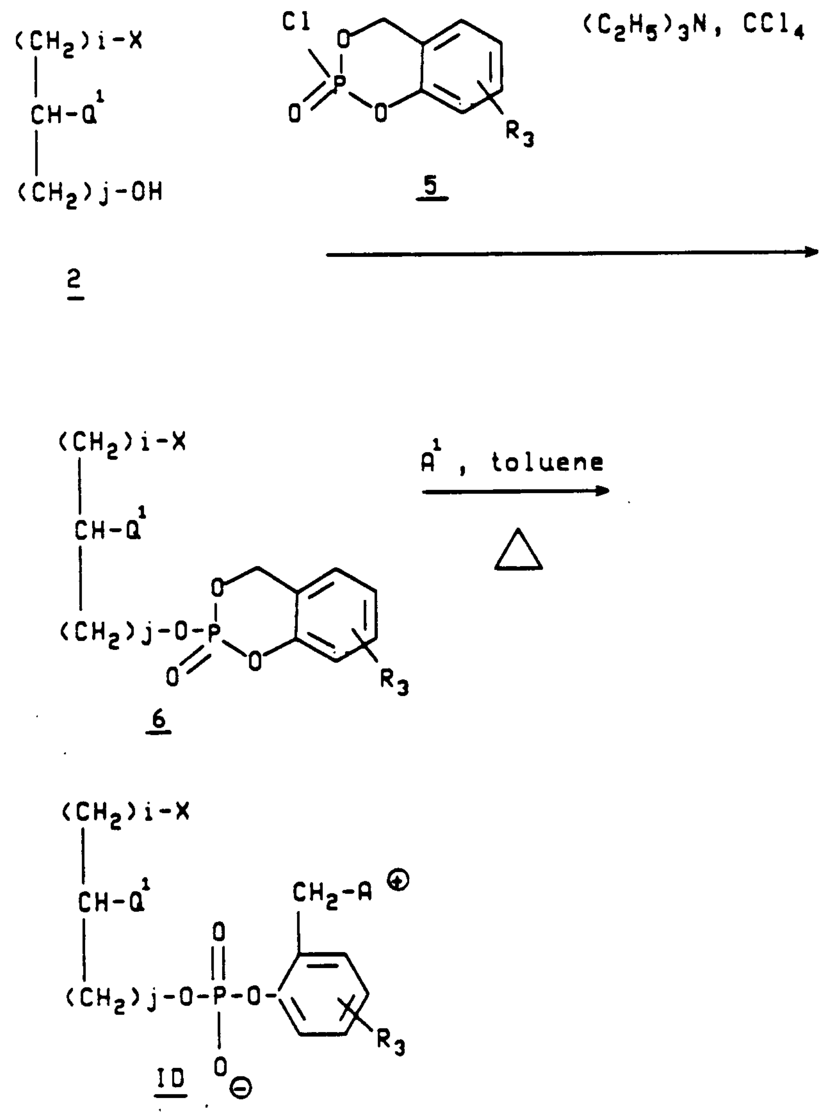


2

5

6

ID

## Flowchart B (continued)

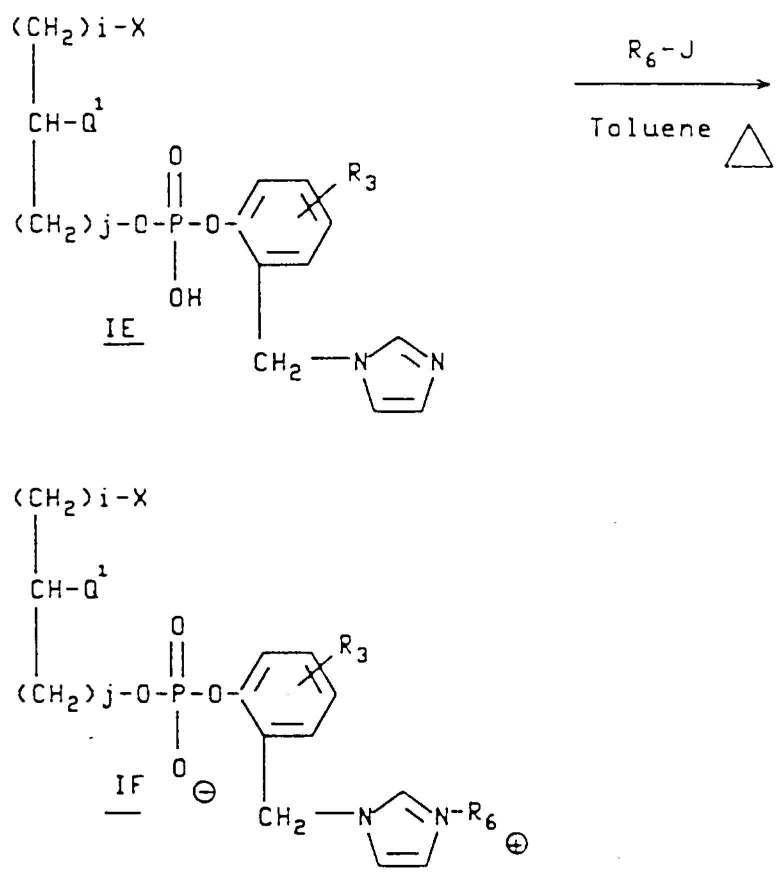


IE

IF

The phosphorus reagents represented by formulas 7a and 7b needed to prepare some of the compounds of this invention are prepared as described hereinbelow in Flowsheet C and in copending application Serial No. 679,788, filed December 10, 1984 (Case 29,706) (US-A-4 762 942) wherein $R_3$ is as defined hereinabove and $J^1$ is chlorine or bromine.

According to the reaction outlined in Flowsheet C, the phenols 8a or 8b are reacted with phosphorusoxychloride and at least one equivalent of an amine base such as triethylamine in an inert solvent such as carbon tetrachloride to give the phosphorus reagents 7a and 7b.

## Flowchart C

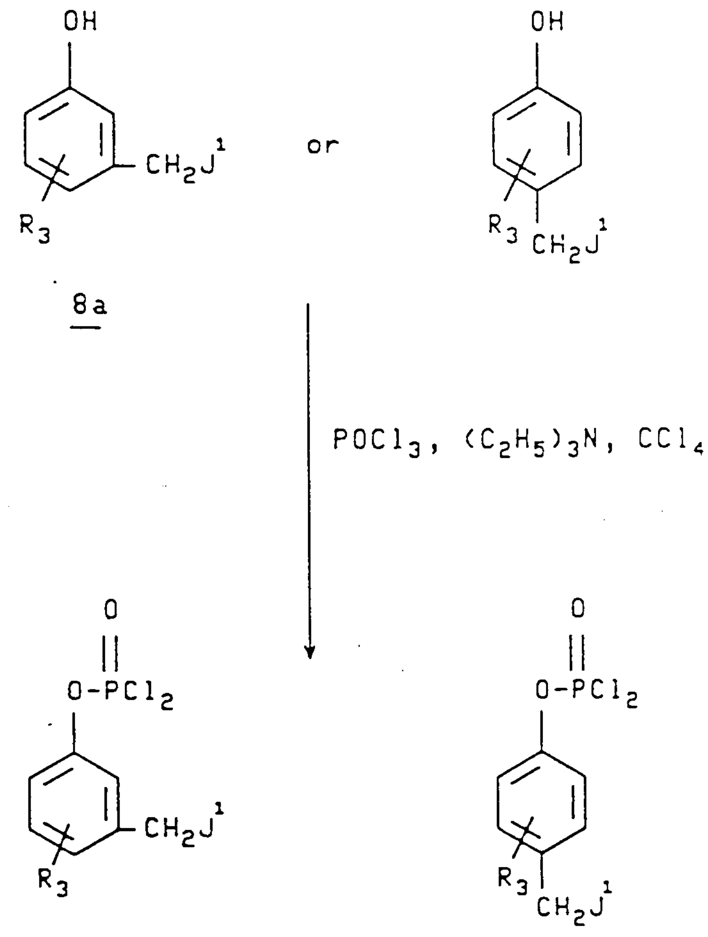


8a or

7a

7b

The cyclic phosphorus reagents represented by formula 9 needed to prepare some of the compounds of this invention are prepared as described hereinbelow in flowsheet D wherein $R_3$ is as defined hereinabove.

According to the reactions outlined in flowsheet D, the hydroxy phenol 10 is treated with an equivalent of phosphorus trichloride in an inert solvent such as ether in the presence of at least two equivalents of pyridine to give compound 11. Oxidation of 11 with a dry stream of oxygen in benzene then furnishes the phosphorus reagent 9.

## Flowsheet D

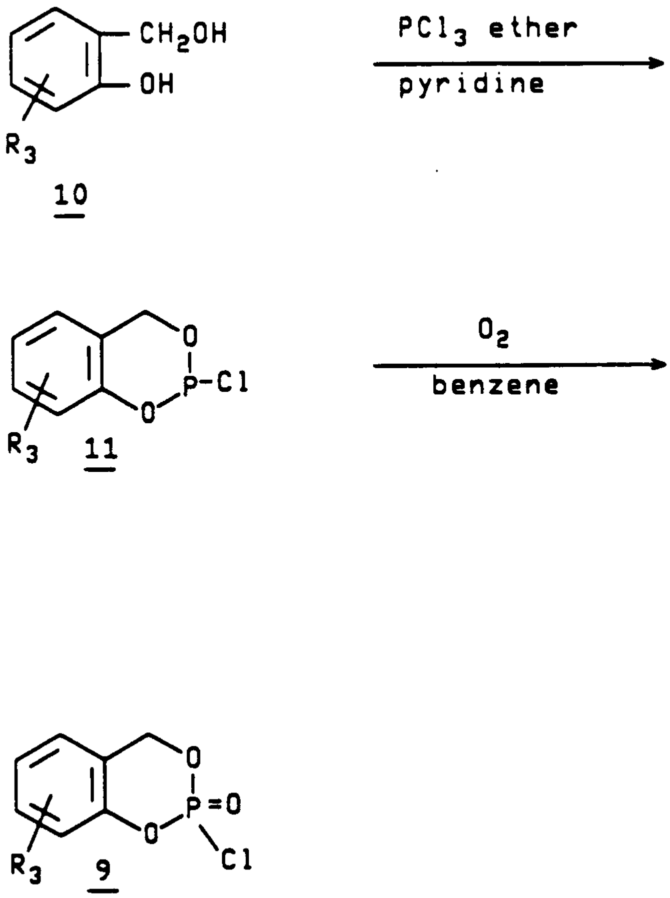


10

11

9

Compounds of this invention represented by formulas 16a-c are prepared as described hereinbelow in flowsheet E, wherein $R_3$, J′, J, X, Q′, i, j, A′ and A are as defined hereinabove.

According to the reactions outlined in flowchart E the carboxylic acid 13 is converted to the acid chloride 14 by the reaction with oxalyl chloride in dichloromethane in the presence of a catalytic amount of dimethylformamide. The acid chloride 14 is then treated with an equivalent of the alcohol 2 in an inert solvent such as tetrahydrofuran in the presence of at least one equivalent of an amine base such as pyridine to give the ester 15. The reaction of 15 with a large excess of a nitrogen containing heterocycle, a sulfide, thiourea, or a phosphine in an inert solvent such as toluene at 50°-150°C gives the compounds of this invention represented by formula 16a. These compounds are obtained as salts except when A is an imidazole moiety. In those cases where the compound contains an imidazole ring (formula 16b), the imidazole moiety can be further alkylated with an excess of alkyl halide by heating in an inert solvent such as toluene to give the salts 16c.

## Flowsheet E

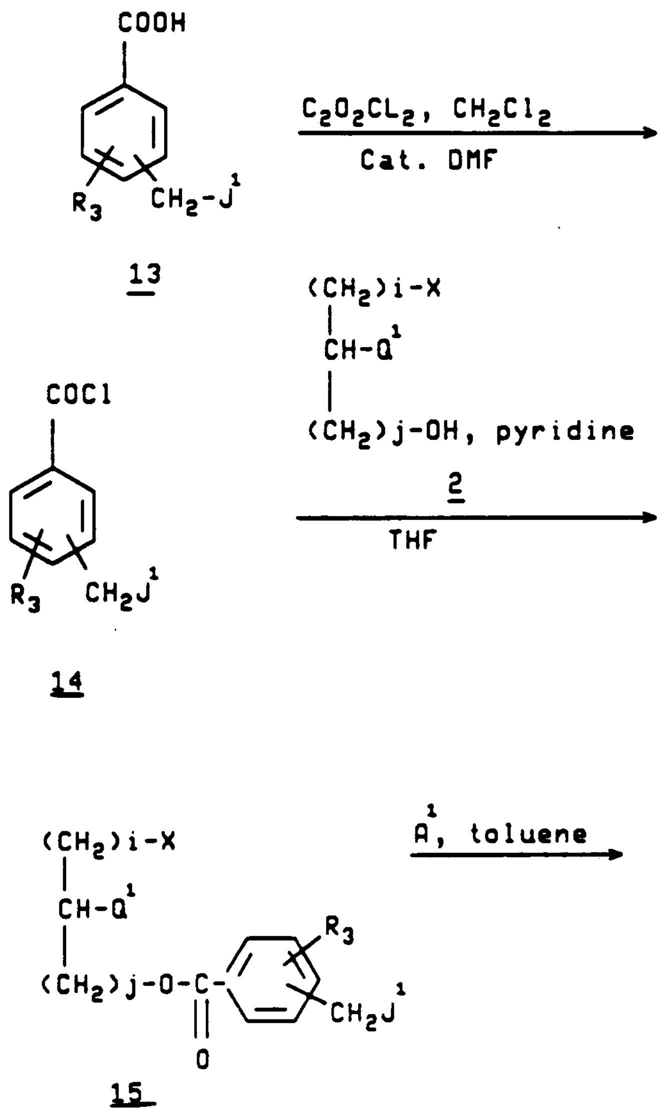

C2O2CL2, CH2Cl2
Cat. DMF
13
(CH2)i-X
CH-Q1
(CH2)j-OH, pyridine
2
THF
14
A1, toluene
15

## Flowchart E (continued)

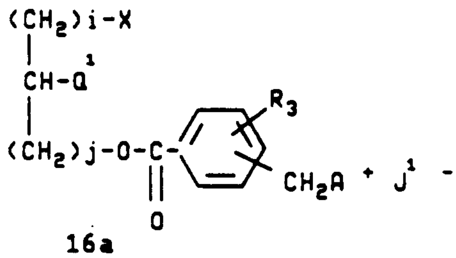

16a

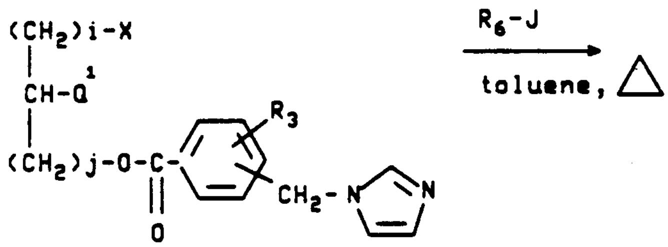


16b

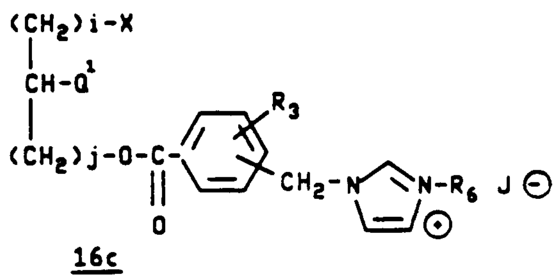

16c

Compounds of this invention represented by formulas 22a-c are prepared as described hereinbelow in flowsheet F wherein $R_2$, $R_3$, J′, J, X, i, j, A′ and A are as defined hereinabove and Q″ is $OR_2$‴, where $R_2$‴ is selected from the group consisting of $C_1$-$C_6$alkyl, $C_1$-$C_6$ alkenyl, or tetrahydropyranyl.

According to the reactions outlined in flowsheet F, the alcohol 17 is converted to the corresponding tosylate using p-toluensulfonyl chloride in pyridine. Addition of 18 to a dimethylformamide solution of the sodium salt of diol 19 gives the monoalkylated product 20 which is separated from the dialkylated product and unreacted 19 by chromatography on silica gel. The reaction of 20 with methane sulfonyl chloride and triethylamine in tetrahydrofuran in the presence of an excess of lithium chloride or lithium bromide gives the halide 21. The reaction of 21 with a large excess of a nitrogen containing heterocycle, a sulfide, thiourea, or a phosphine in an inert solvent such as toluene at 50°-150°C gives the compounds of this invention represented by formula 22a. These compounds are obtained as salts except when A is an imidazole moiety. In those cases where the compounds contain an imidazole ring (formula 22b), the imidazole moiety can be further alkylated with an excess of alkyl halide by heating in an inert solvent such as toluene to give the salts 22e.

In those cases where Q″ is tetrahydropyranyl (formula 23), the tetrahydropyranyl group can be hydrolyzed using a

hydrochloric acid-tetrahydrofuran mixture. The resulting alcohols 24 are useful to prepare the esters 25 of this invention by refluxing 24 with an excess of the anhydride 26 in the presence of an excess of an amine base such as triethylamine in an inert solvent such as chloroform.

## Flowsheet F

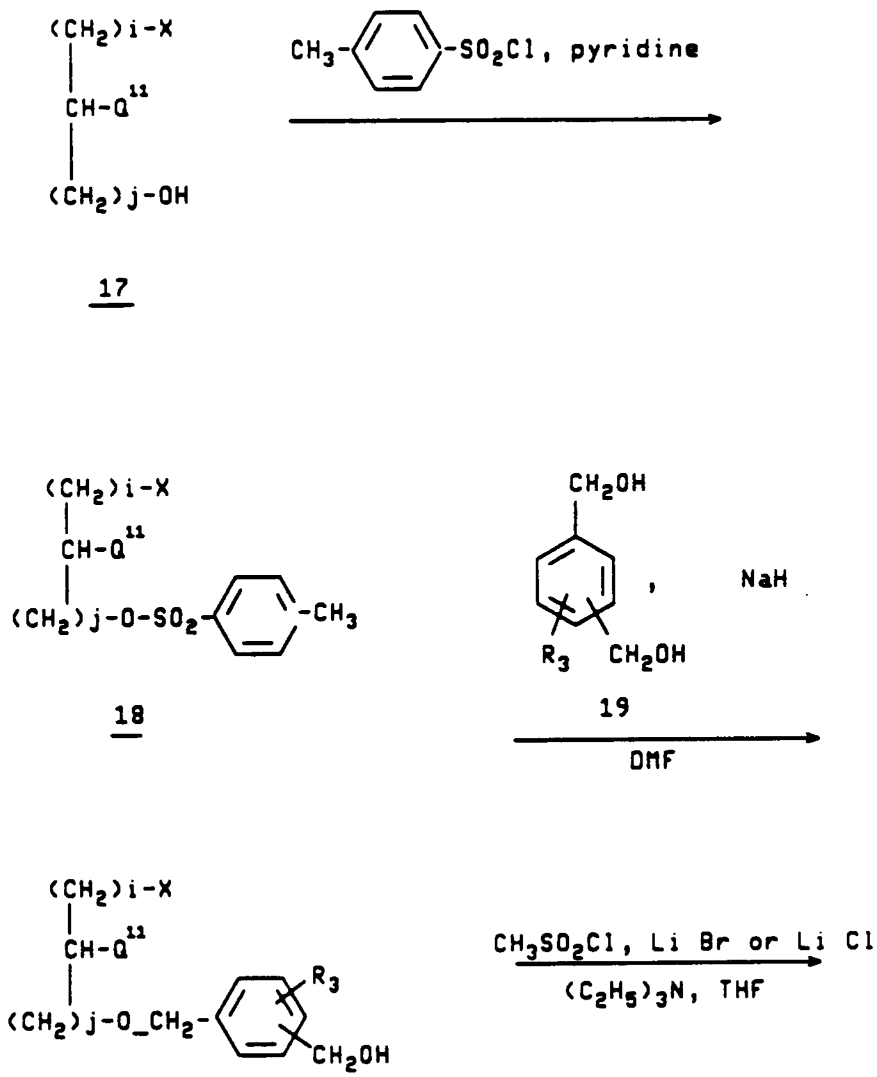


17

18

19

20

## Flowchart F (continued)

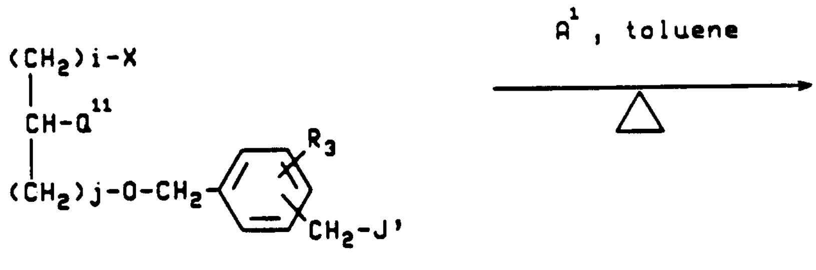


21

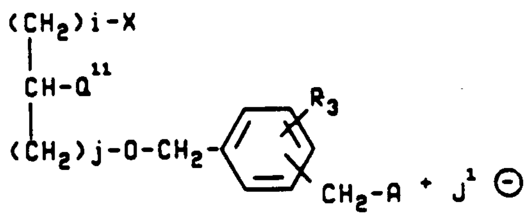

22a

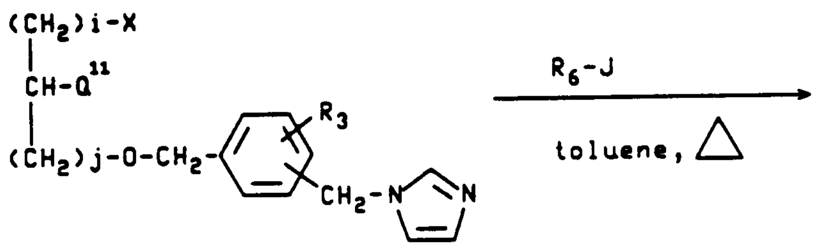


22b

**Flowchart F (continued)**

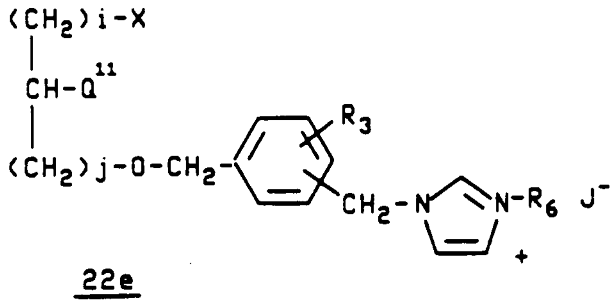

22e

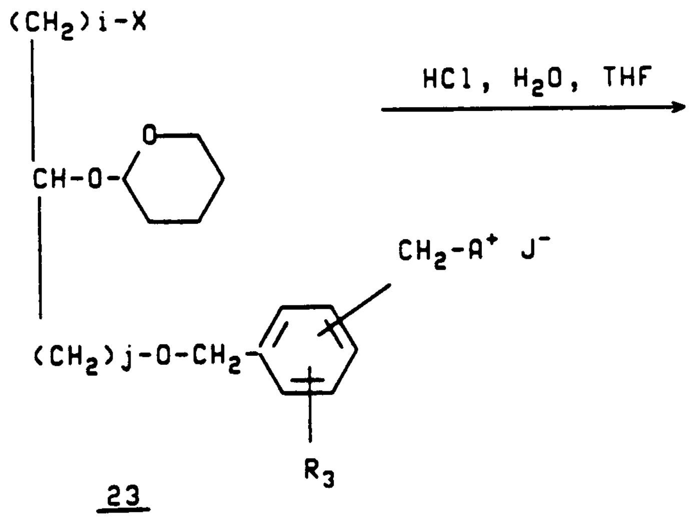


23

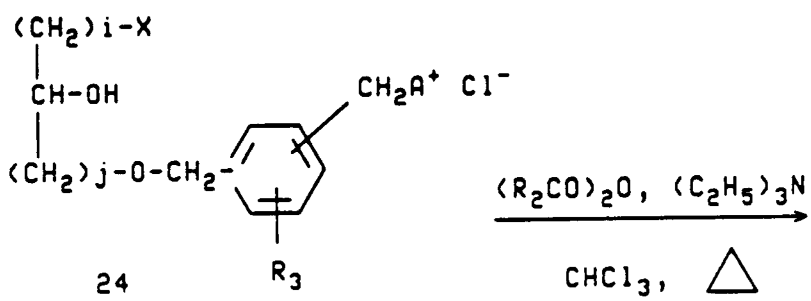


24

**Flowchart F (continued)**

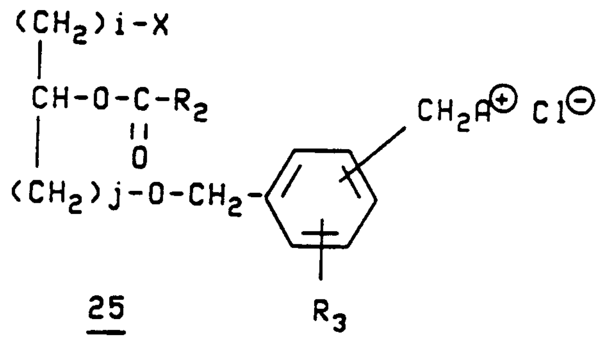

25

The preparations of the various alcohols for formulas 2 and 17 which are needed to prepared the compounds of this invention have been described in U.S. Patents 4,640,913, 4,697,031, 4,703,130 and 4,699,990, copending applications Serial No. 679,788, filed December 10, 1984 (Case 29,706) (US-A-4 762 942), and Serial No. 679,790, filed December 10, 1984 (Case 29,617) (US-A-4 827 011), and Terashita, Z., et. al., Life Sci., 32, 1975 (1983). The above patent and applications describe the preparation of alcohols of formulas 2 and 17 in both the racemic and optically active forms. Consequently, the compounds of this invention can be prepared both as the racemates and as the individual R and S enantiomers.

In addition to the synthetic methods discussed hereinabove, many of the methods and procedures described in the aforementioned U.S. Patent and copending applications are applicable to the preparation of the compounds of this invention by one skilled in the art.

The preparation of compounds of this invention encompassed by Formulas IA-C is described hereinbelow in Flowsheet A, wherein n, X, $R_1$ and $R_5$ are described hereinabove. The moieties -$CH_2$-J and -$CH_2$-Y′ represent substituents of the aromatic ring which are meta or para to the phosphate group wherein J is a leaving group such as the halogens chlorine or bromine and Y′ is a nitrogen containing heterocycle which can be

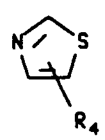 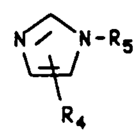 , 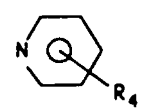

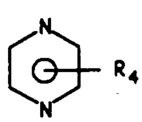 , 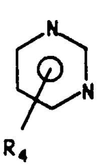 , 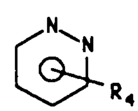

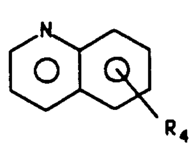 , and 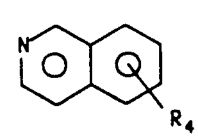

wherein $R_4$ and $R_5$ are as described hereinabove.

According to the sequence of reactions outlined in Flowchart A, the phenol or alcohol 2 is treated with an equivalent of the phosphorus reagent 3 in the presence of a base such as triethylamine in an inert solvent such as carbon tetrachloride to give, after hydrolysis of the resulting intermediate in a buffered solvent system such as tetrahydrofuran-water-sodium acetate, the phosphate 4. The reaction of 4 with a large excess of a nitrogen containing heterocycle in an inert solvent such as toluene at 50°-150°C gives the compounds of this invention represented by formula IA. These compounds are obtained as internal salts except when A is an imidazole moiety.

In those cases where the compounds contain an imidazole ring (Formula IB), the imidazole moiety can be further

alkylated with an excess of alkyl halide by heating in an inert solvent such as toluene to give the internal salts IC.

Flowsheet A

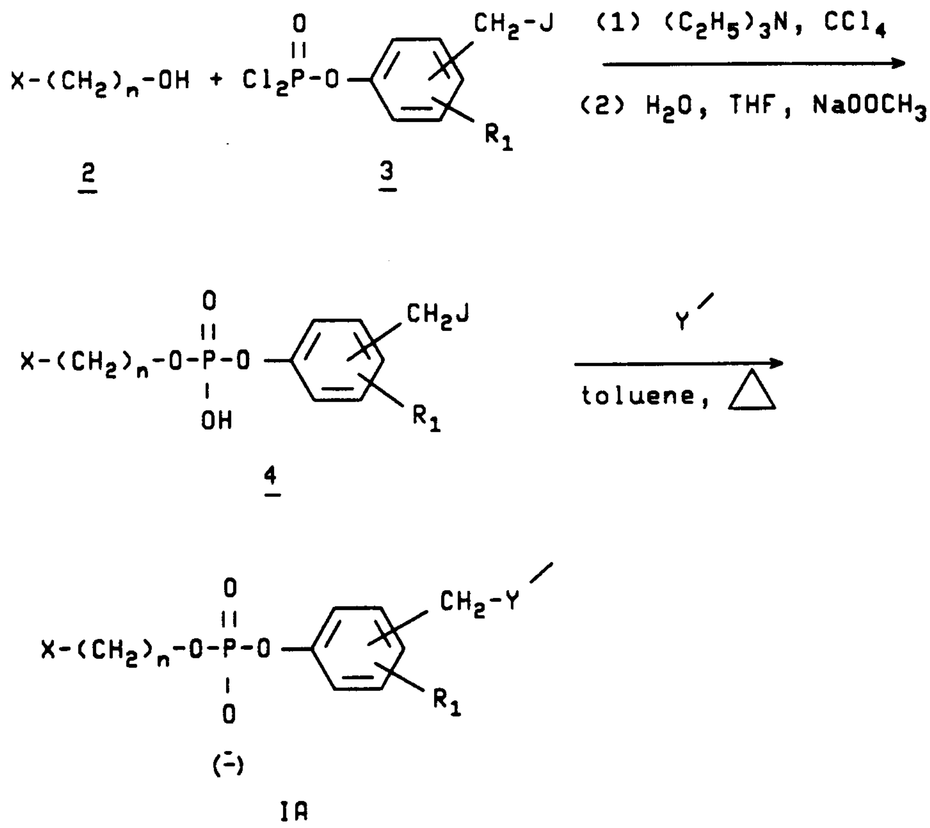


2 3

4

IA

## Flowsheet A (Cont'd)

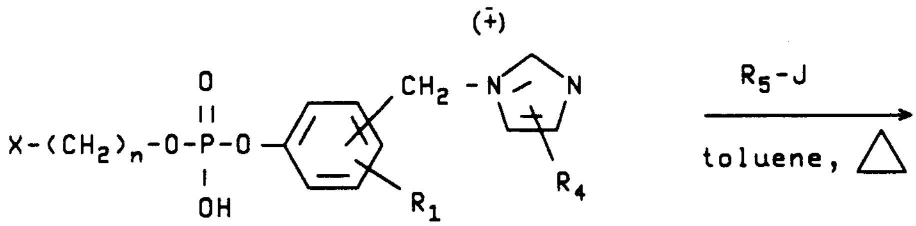


IB

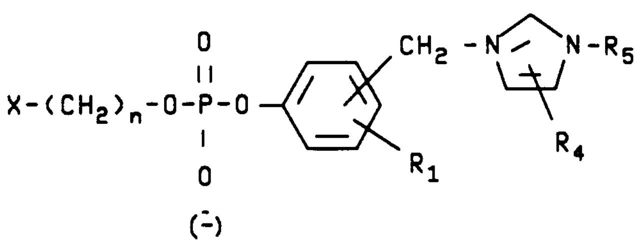

IC

The preparation of compounds of this invention encompassed by formulas ID-IF is described hereinbelow in Flowchart B wherein $R_4$, Y', X, $R_1$, and $R_5$, and J are as defined hereinabove.

According to the sequence of reactions outlined in Flowchart B, the phenol 2 is treated with an equivalent of the phosphorus reagent 5 in the presence of a base such as triethylamine in an inert solvent such as carbon tetrachloride to give the cyclic phosphate 6. The reaction of 6 with a large excess of a nitrogen containing heterocycle in an inert solvent such as toluene at 50°-150°C gives the compounds of this invention represented by formula ID. These compounds are obtained as internal salts except when Y' is an imidazole moiety. In those cases where the compounds contain an imidazole ring (formula IE), the imidazole moiety can be further alkylated with an excess of alkyl halide by heating in an inert solvent such as toluene to give the internal salts IF.

Flowsheet B

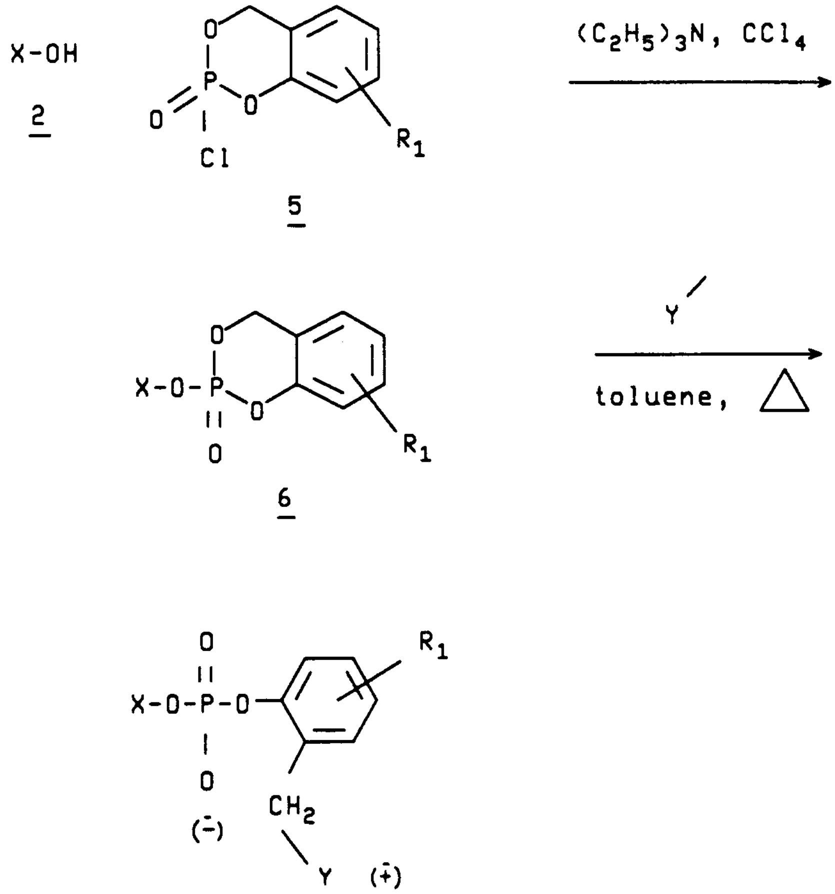


2

5

6

ID

Flowsheet B (Cont'd)

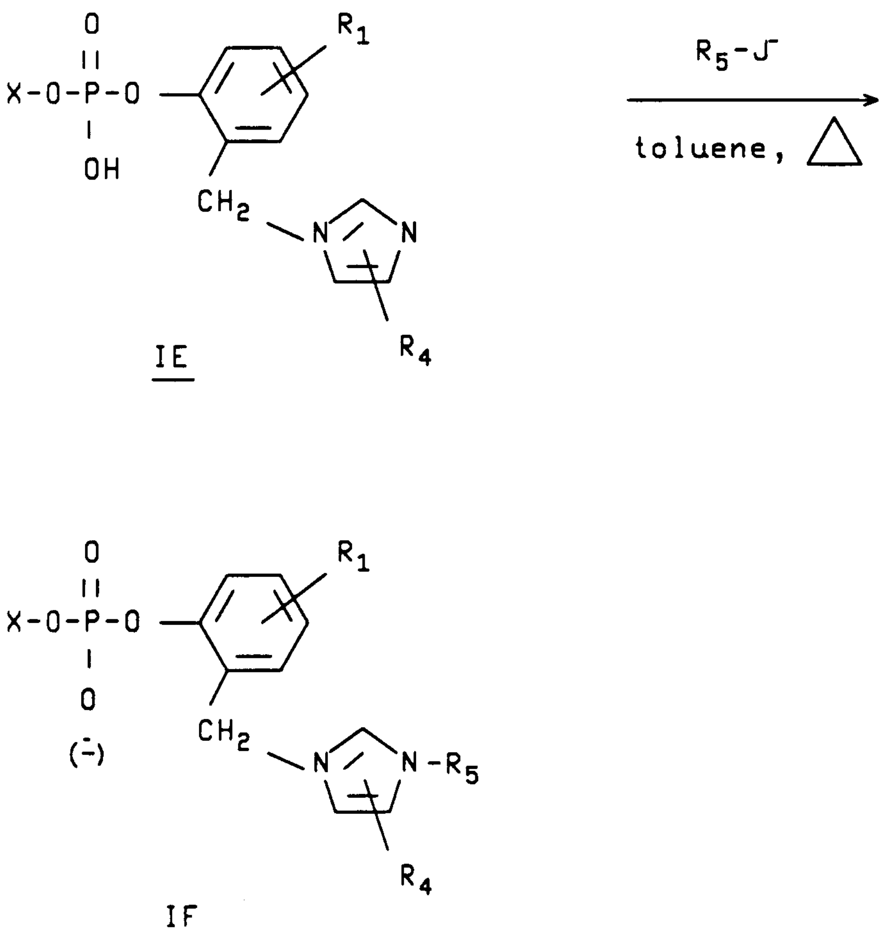


IE

IF

The phosphorus reagents represented by formulas 7a and 7b needed to prepare some of the compounds of this invention are prepared as described hereinbelow in Flowsheet C and in copending application Serial No. 679,788, filed December 10, 1984 (US-A-4 762 942) and in Serial No. 177,299 (EP-A-336 142) wherein $R_1$ is as defined hereinabove and J is chlorine or bromine.

According to the reaction outlined in Flowsheet C, the phenols 8a or 8b are reacted with phosphorus oxychloride and at least one equivalent of an amine base such as triethylamine in an inert solvent such as carbon tetrachloride to give the phosphorus reagents 7a and 7b.

## Flowsheet C

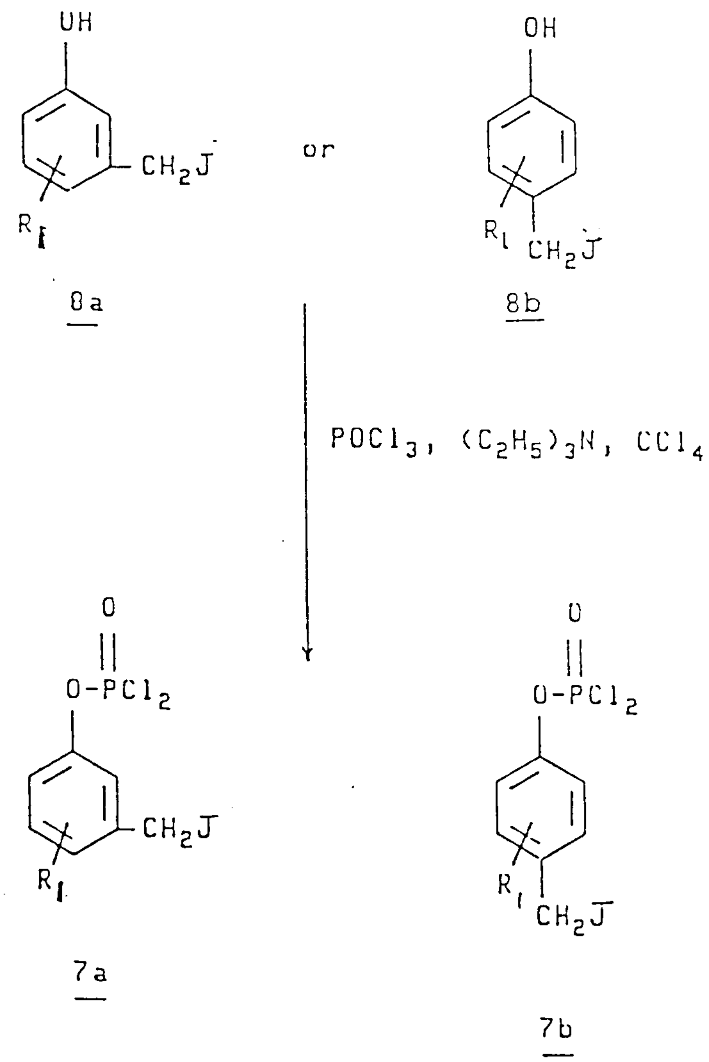


8a or 8b

7a 7b

The cyclic phosphorus reagents represented by formula 9 needed to prepare some of the compounds of this invention are prepared as described hereinbelow in Flowsheet D wherein $R_1$ is as defined hereinabove.

According to the reactions outlined in Flowsheet D, the hydroxy phenol 10 is treated with an equivalent of phosphorus trichloride in an inert solvent such as ether in the presence of at least two equivalents of pyridine to give compound 11. Oxidation of 11 with a dry stream of oxygen in benzene then furnishes the phosphorus reagent 9.

## Flowsheet D

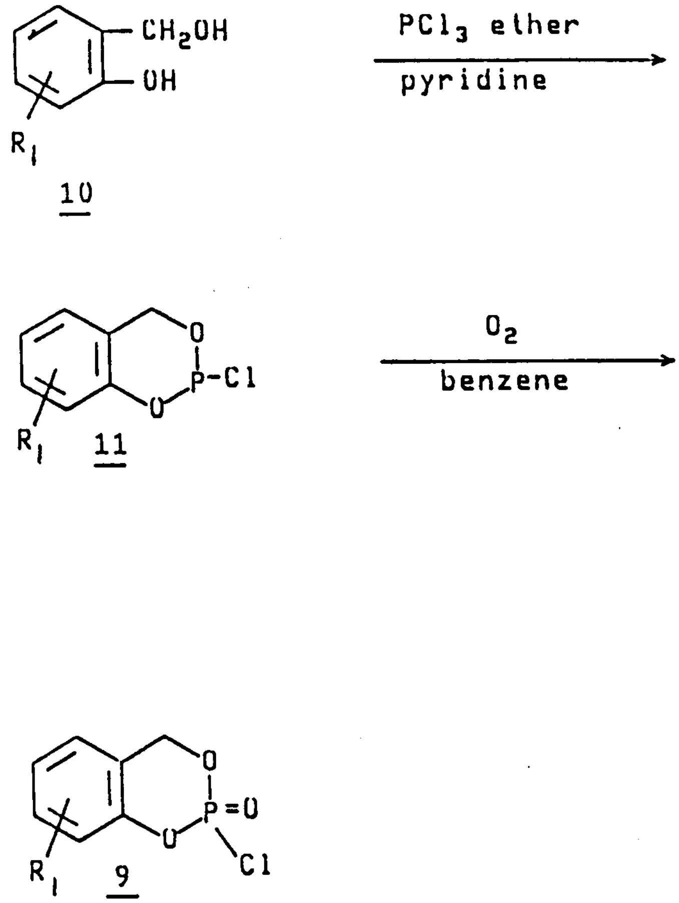


10

11

9

The substituted phenols and hydroxy naphthalenes of Formula 12 needed to prepare the compounds of this invention can be prepared as described in the following U.S. patents: 4,697,031; 4,699,990 and 4,640,913 and as described hereinbelow in Flowsheet E, wherein $R_6$ represents one or more substituents which may occupy any position and are selected from the group consisting of hydrogen, $-R_2$, trifluoromethyl, and fluorine wherein $R_2$ is as described hereinabove, and $R_7$ is $C_1$-$C_{25}$ alkyl or $C_1$-$C_{25}$ alkenyl. J′ is chlorine, bromine or iodine.

According to the sequence of reactions outlined in Flowsheet E, a substituted bromo anisol 10 is reacted with magnesium in tetrahydrofuran to form the Grignard reagent which in turn is reacted with an alkyl halide in the presence of $Li_2CuCl_4$. The resulting methyl ether 11 is cleaved to the desired phenol using boron tribromide in an inert solvent such as methylene chloride. An identical sequence of reaction can be applied to furnish substituted hydroxy naphthalenes needed to prepare some of the compounds of this invention.

Flowsheet E

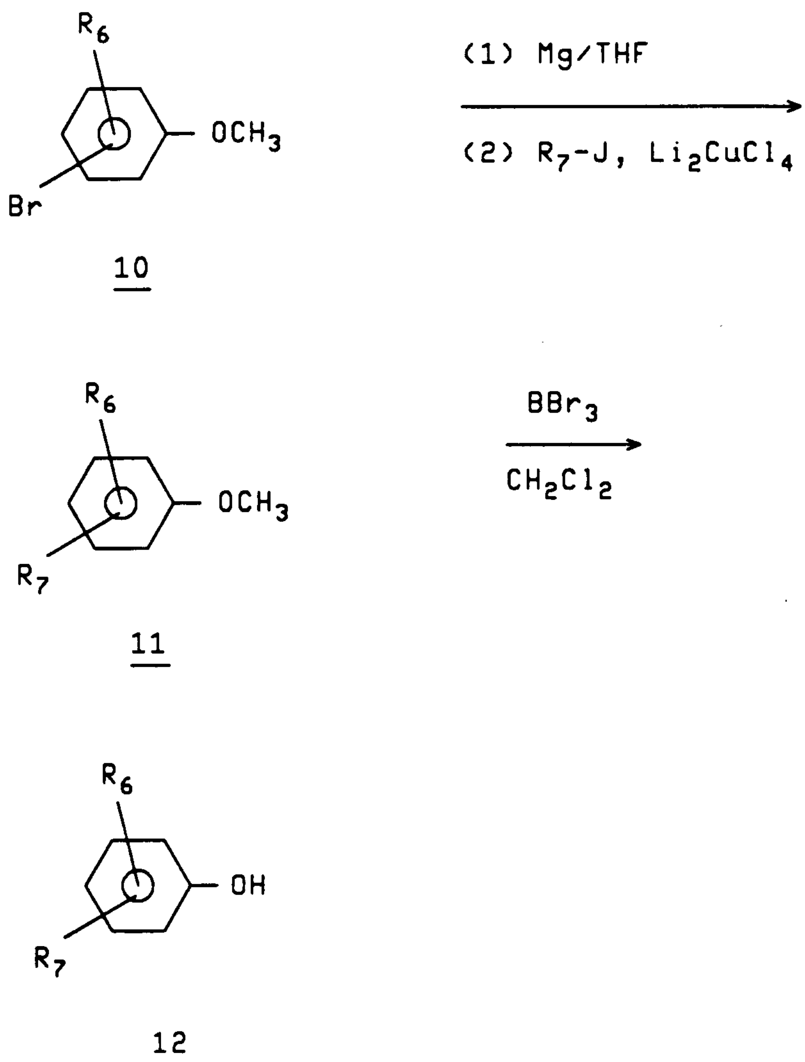


10

11

12

The substituted phenols and hydroxy naphthalenes of Formula 14 needed to prepare the compounds of this invention can be prepared as described in the following U.S. patents: 4,697,031; 4,699,990 and 4,640,913, in the following patent application Serial No. 679,792 (US-A-4 697 031), and as described hereinbelow in Flowsheet F wherein $R_2$ and J′ are as hereinabove defined and $R_7$ is $C_1$-$C_{25}$ alkyl and $C_1$-$C_{25}$ alkenyl. According to the sequence of reactions shown in Flowsheet F, treatment of the dihydroxy compound 13 with sodium hydride in an inert solvent such as dimethylformamide in the presence of an alkyl halide produces the monoalkylated product 14 which can readily be separated from the unreacted 13 and the dialkylated product by a combination of distillation and chromatography. In those cases where two isometric monoalkylated products result, they can be separated using chromatographic procedures. An identical sequence of reactions can be applied to the synthesis of the hydroxy naphthalenes needed to prepare the compounds of this invention.

Some of the phenols and hydroxy naphthalenes that can be used to prepare the compounds of this invention are listed hereinbelow in Table I.

Flowsheet F

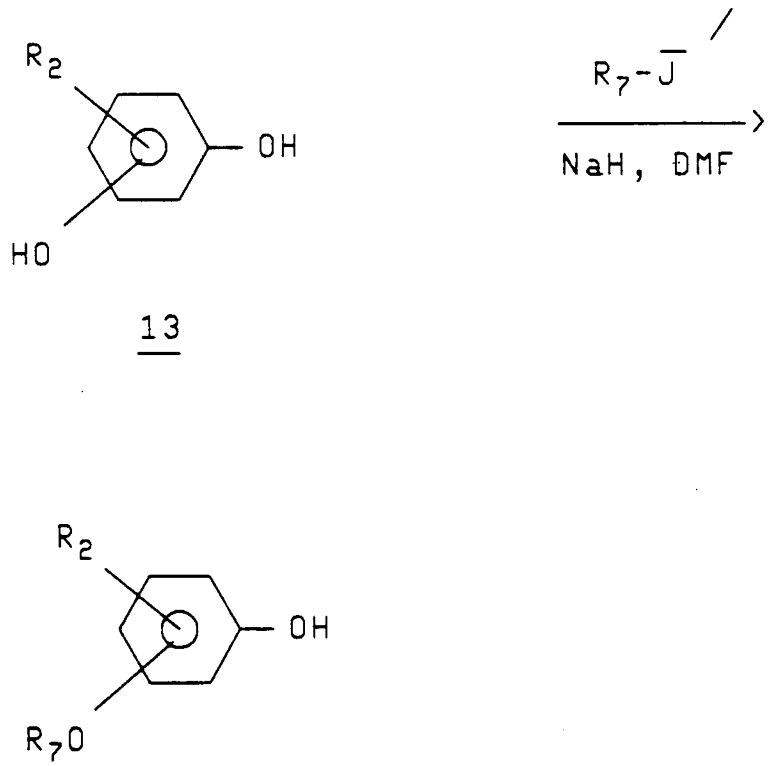


13

14

## Claims

**Claims for the following Contracting States : AT, BE, CH, DE, FR, GB, IT, LI, NL, SE**

1. Products containing, in therapeutically effective amounts, an antagonist to Platelet Activating Factor and one or more monoclonal or polyclonal antibodies directed towards either Tumor Necrosis Factor α, Interleukin-1β, Gamma Interferon, or bacterial cell wall lipopolysaccharides as a combined preparation for simultaneous, separate or sequential use in the treatment of endotoxic shock.

2. A product according to Claim 1, wherein a monoclonal antibody directed towards Tumor Necrosis Factor α is combined with an antagonist of Platelet Activating Factor of the formula

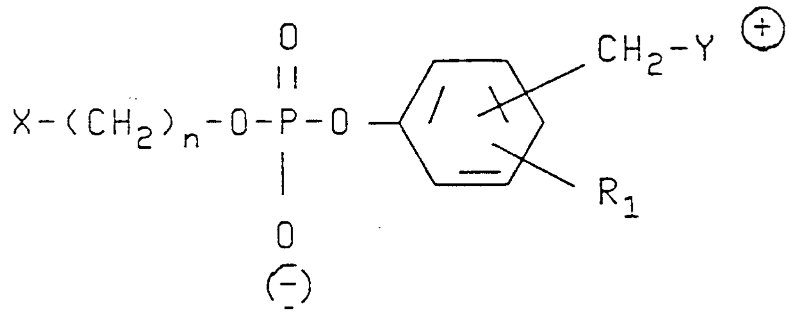

Formula I

wherein:

X is a phenyl or naphthyl ring optionally substituted in any position with one or more substituents of:

i) $-R_2$ wherein $R_2$ is $C_1$-$C_{25}$ alkyl, $C_1$-$C_{25}$ alkenyl, $C_1$-$C_{25}$ alkoxy, $C_1$-$C_{25}$ alkenyloxy, $C_1$-$C_{25}$ thioalkyl, phenyl, phenoxy, substituted phenyl or substituted phenoxy wherein the substituents are $C_1$-$C_{20}$ alkyl, $C_1$-$C_{20}$ alkoxy, halogen or trifluoromethyl;
ii) hydrogen, halogen, trifluoromethyl, cyano or nitro;
iii) $-CO_2R_3$, $-CONHR_3$, -CHO, $OCONHR_3$, or $-NHCOR_3$ wherein $R_3$ is $C_1$-$C_{25}$ alkyl, $C_1$-$C_{25}$ alkenyl, phenyl or substituted phenyl wherein the substituents are $C_1$-$C_{20}$ alkyl, $C_1$-$C_{20}$ alkoxy, halogen, or trifluoromethyl;

$R_1$ is one or more substituents of the aromatic ring which may be in any position and is hydrogen, $C_1$-$C_5$ alkyl, $C_1$-$C_5$ alkoxy, or halogen;

$-CH_2$-Y is a single substituent of the aromatic ring which may occupy any position wherein Y is a mono or bicyclic aromatic heterocycle with 5-7 membered rings containing at least one nitrogen atom which is bonded to the methylene group and optionally one or more other nitrogen or sulfur atoms; and n is the integer 0 or 1.

**3.** Products according to Claim 1, wherein, a monoclonal antibody directed towards Interleukin-1β is combined with an antagonist of Platelet Activating Factor according to Claim 2.

**4.** Products according to Claim 1, wherein a monoclonal antibody directed towards Gamma Interferon is combined with an antagonist of Platelet Activating Factor according to Claim 2.

**5.** Products according to Claim 1, wherein a monoclonal antibody directed towards bacterial cell wall lipopolysaccharide is combined with an antagonist of Platelet Activating Factor according to Claim 2.

**6.** Products according to Claim 1, wherein a monoclonal antibody directed towards Tumor Necrosis Factor α is combined with an antagonist of Platelet Activating Factor of the formula:

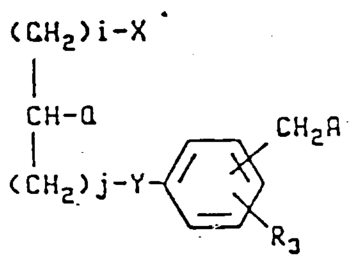

**Formula II**

wherein:

(A) X is

i) $C_1$-$C_{24}$ alkyl;
ii) $C_1$-$C_{24}$ alkoxy;
iii) $C_1$-$C_{24}$ carboamoyloxy;
iv)

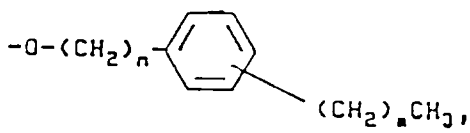 ,
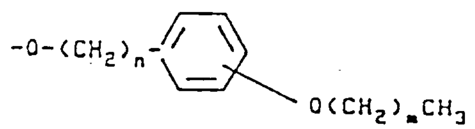 ,
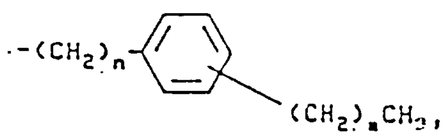 ,
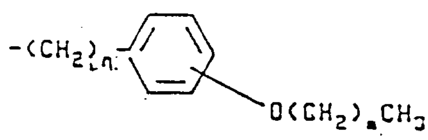
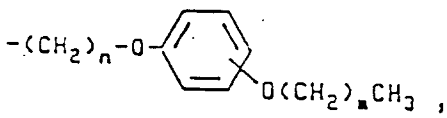 ,
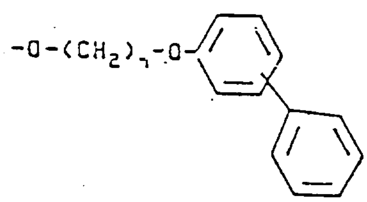

or 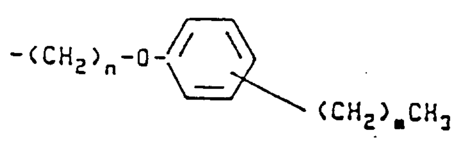

n is an integer from 1 to 25 and m is an integer from 0 to 24 and the sum of n and m is less than or equal to 25;
v) phenyl;
vi) mono- or polysubstituted phenyl substituted with $C_1$-$C_{20}$ alkyl, $C_1$-$C_{20}$ alkoxy, halogen, trifluoromethyl, phenyl, substituted phenyl or benzyloxy;
vii) phenoxy;
viii) mono- or polysubstituted phenoxy substituted with $C_1$-$C_{20}$ alkyl, $C_1$-$C_{20}$ alkoxy, halogen, trifluoromethyl, phenyl, substituted phenyl or benzyloxy;

ix) naphthaloxy;

x) mono- or polysubstituted naphthaloxy substituted with $C_1$-$C_{20}$ alkyl, $C_1$-$C_{20}$ alkoxy or halogen;

xi) -O-$(CH_2)_r$-O-$((CH_2)_pO)_t$-$(CH_2)_a$-W wherein W is methyl or phenyl optionally substituted with $C_1$-$C_3$ alkyl, $C_1$-$C_3$ alkoxy or phenyl, r, p, t and a are integers such that the expression r+(p+1)t+a is also an integer and has a value of 3 to 20; r is greater than or equal to 2; p is greater than or equal to 2; t is greater than or equal to zero; and a is greater than or equal to zero;

(B) i is an integer from 1 to 3 and j is an integer from 1 to 6;

(C) Q is $-OR_2$,

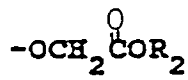

or 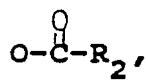

wherein $R_2$ is hydrogen, $C_1$-$C_6$ alkyl or $C_1$-$C_6$ alkenyl;

(D) Y is a divalent radical

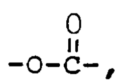

$-OCH_2$-, or

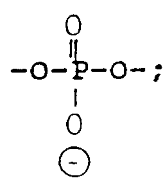

(E) $R_3$ is one or more $C_1$-$C_5$ alkyl,

$C_1$-$C_5$ alkoxy or halogen substituents of the aromatic ring;

(F) $-CH_2A$ may be in the <u>ortho</u>, <u>meta</u> or <u>para</u> position wherein A is a 5-7 member aromatic heterocyclic ring containing at least one nitrogen atom and optionally one or more other nitrogen or sulfur atoms.

**7.** Products according to Claim 1, wherein a monoclonal antibody directed towards Interleukin-1β is combined with an antagonist of Platelet Activating Factor according to Claim 6.

**8.** Products according to Claim 1, wherein a monoclonal antibody is directed towards Gamma Interferon is combined with an antagonist of Platelet Activating Factor according to Claim 6.

**9.** Products according to Claim 1, wherein a monoclonal antibody is directed towards bacterial cell wall lipopolysaccharides is combined with an antagonist of Platelet Activating Factor according to Claim 6.

**Claims for the following Contracting States : ES, GR**

**1.** A process for making a product for use in the treatment of endotoxic shock in a mammal which comprises combining a therapeutically effective amount of an antagonist to Platelet Activating Factor and one or more monoclonal or polyclonal antibodies directed towards either Tumor Necrosis Factor α and, Interleukin-1β, Gamma Interferon, or bacterial cell wall lipopolysaccharide, to provide a preparation for simultaneous, separate or sequential use.

**2.** A process as claimed in Claim 1 wherein the monoclonal or polyclonal antibody directed towards Tumor Necrosis Factor α is in combination with an antagonist of Platelet Activating Factor of the formula

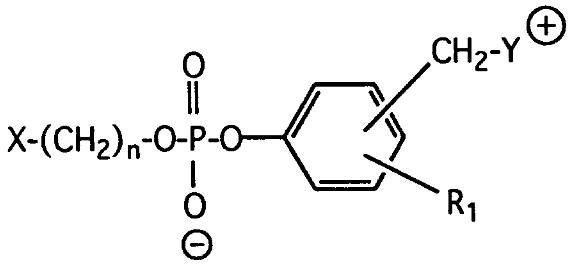

Formula I

wherein:

X is a phenyl or naphthyl ring optionally substituted in any position with one or more substituents of:

i) $-R_2$ wherein $R_2$ is $C_1$-$C_{25}$ alkyl, $C_1$-$C_{25}$ alkenyl, $C_1$-$C_{25}$ alkoxy, $C_1$-$C_{25}$ alkenyloxy, $C_1$-$C_{25}$ thioalkyl, phenyl, phenoxy, substituted phenyl or substituted phenoxy wherein the substituents are $C_1$-$C_{20}$ alkyl, $C_1$-$C_{20}$ alkoxy, halogen or trifluoromethyl;
ii) hydrogen, halogen, trifluoromethyl, cyano or nitro;
iii) $-CO_2R_3$, $-CONHR_3$, -CHO, $-OCONHR_3$, or $NHCOR_3$ wherein $R_3$ is $C_1$-$C_{25}$ alkyl, $C_1$-$C_{25}$ alkenyl, phenyl or substituted phenyl wherein the substituents are $C_1$-$C_{20}$ alkyl, $C_1$-$C_{20}$ alkoxy, halogen, or trifluoromethyl;

$R_1$ is one or more substituents of the aromatic ring which may be in any position and is hydrogen, $C_1$-$C_5$ alkyl, $C_1$-$C_5$ alkoxy, or halogen;
$-CH_2$-Y is a single substituent of the aromatic ring which may occupy any position wherein Y is a mono or bicyclic aromatic heterocycle with 5-7 membered rings containing at least one nitrogen atom which is bonded to the methylene group and optionally one or more other nitrogen or sulfur atoms;
and n is the integer 0 or 1.

**3.** A process as claimed in Claim 1 wherein the monoclonal or polyclonal antibody directed towards Interleukin-1β is in combination with an antagonist of Platelet Activating Factor of the formula

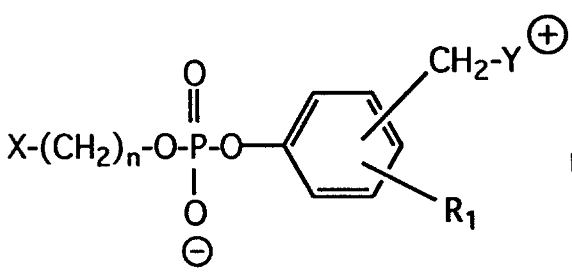

Formula I

wherein:

X is a phenyl or naphthyl ring optionally substituted in any position with one or more substituents of:

i) $-R_2$ wherein $R_2$ is $C_1$-$C_{25}$ alkyl, $C_1$-$C_{25}$ alkenyl, $C_1$-$C_{25}$ alkoxy, $C_1$-$C_{25}$ alkenyloxy, $C_1$-$C_{25}$ thioalkyl, phenyl, phenoxy, substituted phenyl or substituted phenoxy wherein the substituents are $C_1$-$C_{20}$ alkyl, $C_1$-$C_{20}$ alkoxy, halogen or trifluoromethyl;
ii) hydrogen, halogen, trifluoromethyl, cyano or nitro;
iii) $-CO_2R_3$, $-CONHR_3$, -CHO, $-OCONHR_3$, or $-NHCOR_3$ wherein $R_3$ is $C_1$-$C_{25}$ alkyl, $C_1$-$C_{25}$ alkenyl, phe-

nyl or substituted phenyl wherein the substituents are $C_1$-$C_{20}$ alkyl, $C_1$-$C_{20}$ alkoxy, halogen, or trifluoromethyl;

$R_1$ is one or more substituents of the aromatic ring which may be in any position and is hydrogen, $C_1$-$C_5$ alkyl, $C_1$-$C_5$ alkoxy, or halogen;
$-CH_2$-Y is a single substituent of the aromatic ring which may occupy any position wherein Y is a mono or bicyclic aromatic heterocycle with 5-7 membered rings containing at least one nitrogen atom which is bonded to the methylene group and optionally one or more other nitrogen or sulfur atoms;
and n is the integer 0 or 1.

**4.** A process as claimed in Claim 1 wherein the monoclonal or polyclonal antibody directed towards Gamma Interferon is in combination with an antagonist of Platelet Activating Factor of the formula

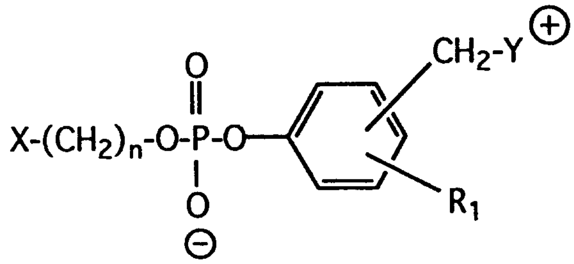

Formula I

wherein:

X is a phenyl or naphthyl ring optionally substituted in any position with one or more substituents of:

i) $-R_2$ wherein $R_2$ is $C_1$-$C_{25}$ alkyl, $C_1$-$C_{25}$ alkenyl, $C_1$-$C_{25}$ alkoxy, $C_1$-$C_{25}$ alkenyloxy, $C_1$-$C_{25}$ thioalkyl, phenyl, phenoxy, substituted phenyl or substituted phenoxy wherein the substituents are $C_1$-$C_{20}$ alkyl, $C_1$-$C_{20}$ alkoxy, halogen or trifluoromethyl;
ii) hydrogen, halogen, trifluoromethyl, cyano or nitro;
iii) $-CO_2R_3$, $-CONHR_3$, -CHO, $-OCONHR_3$, or $-NHCOR_3$ wherein $R_3$ is $C_1$-$C_{25}$ alkyl, $C_1$-$C_{25}$ alkenyl, phenyl or substituted phenyl wherein the substituents are $C_1$-$C_{20}$ alkyl, $C_1$-$C_{20}$ alkoxy, halogen, or trifluoromethyl;

$R_1$ is one or more substituents of the aromatic ring which may be in any position and is hydrogen, $C_1$-$C_5$ alkyl, $C_1$-$C_5$ alkoxy, or halogen;
$-CH_2$-Y is a single substituent of the aromatic ring which may occupy any position wherein Y is a mono or bicyclic aromatic heterocycle with 5-7 membered rings containing at least one nitrogen atom which is bonded to the methylene group and optionaily one or more other nitrogen or sulfur atoms;
and n is the integer 0 or 1.

**5.** A process as claimed in Claim 1 wherein the monoclonal or polyclonal antibody directed towards bacterial cell wall lipopolysaccharide is in combination with an antagonist of Platelet Activating Factor of the formula

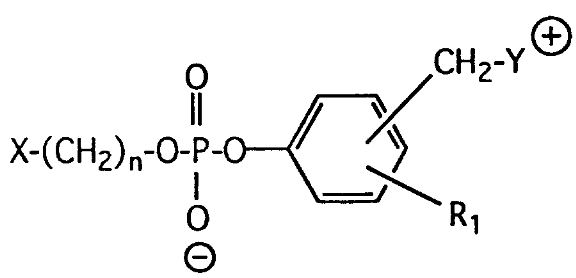

Formula I

wherein:

X is a phenyl or naphthyl ring optionally substituted in any position with one or more substituents of:

i) $-R_2$ wherein $R_2$ is $C_1$-$C_{25}$ alkyl, $C_1$-$C_{25}$ alkenyl, $C_1$-$C_{25}$ alkoxy, $C_1$-$C_{25}$ alkenyloxy, $C_1$-$C_{25}$ thioalkyl, phenyl, phenoxy, substituted phenyl or substituted phenoxy wherein the substituents are $C_1$-$C_{20}$ alkyl, $C_1$-$C_{20}$ alkoxy, halogen or trifluoromethyl;
ii) hydrogen, halogen, trifluoromethyl, cyano or nitro;
iii) $-CO_2R_3$, $-CONHR_3$, -CHO, $-OCONHR_3$, or $-NHCOR_3$ wherein $R_3$ is $C_1$-$C_{25}$ alkyl, $C_1$-$C_{25}$ alkenyl, phenyl or substituted phenyl wherein the substituents are $C_1$-$C_{20}$ alkyl, $C_1$-$C_{20}$ alkoxy, halogen, or trifluoromethyl;

$R_1$ is one or more substituents of the aromatic ring which may be in any position and is hydrogen, $C_1$-$C_5$ alkyl, $C_1$-$C_5$ alkoxy, or halogen;
$-CH_2$-Y is a single substituent of the aromatic ring which may occupy any position wherein Y is a mono or bicyclic aromatic heterocycle with 5-7 membered rings containing at least one nitrogen atom which is bonded to the methylene group and optionally one or more other nitrogen or sulfur atoms;
and n is the integer 0 or 1.

**6.** A process as claimed in Claim 1 wherein the monoclonal or polyclonal antibody directed towards Tumor Necrosis Factor $\alpha$ is in combination with an antagonist of Platelet Activating Factor of the formula:

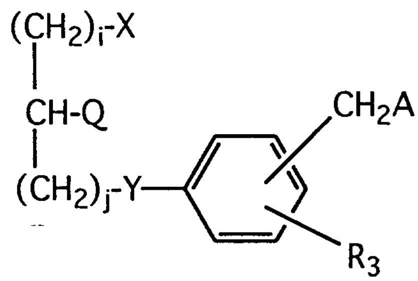

Formula II

wherein:

(A) X is

i) $C_1$-$C_{24}$ alkyl;
ii) $C_1$-$C_{24}$ alkoxy;
iii) $C_1$-$C_{24}$ carbamoyloxy;
iv)

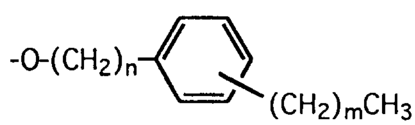 ; 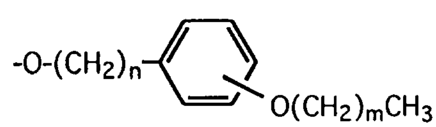 ;

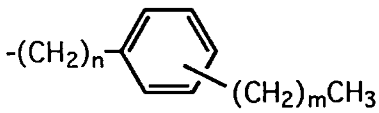 ; 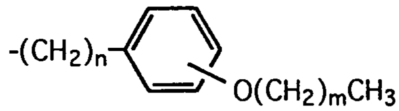 ;

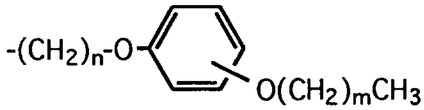 ; 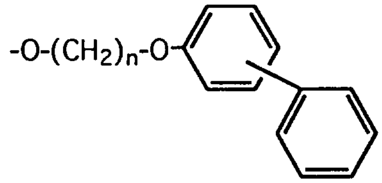

or 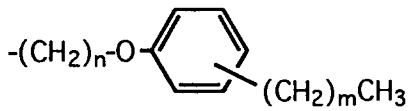 ;

n is an integer from 1 to 25 and m is an integer from 0 to 24 and the sum of n and m is less than or equal to 25;

v) phenyl;

vi) mono- or polysubstituted phenyl substituted with $C_1$-$C_{20}$ alkyl, $C_1$-$C_{20}$ alkoxy, halogen, trifluoromethyl, phenyl, substituted phenyl or benzyloxy;

vii) phenoxy;

viii) mono- or polysubstituted phenoxy substituted with $C_1$-$C_{20}$ alkyl, $C_1$-$C_{20}$ alkoxy, halogen, trifluoromethyl, phenyl, substituted phenyl or benzyloxy;

ix) naphthaloxy;

x) mono- or polysubstituted naphthaloxy substituted with $C_1$-$C_{20}$ alkyl, $C_1$-$C_{20}$ alkoxy or halogen;

xi) $-O-(CH_2)_r-O-((CH_2)_pO)_t-(CH_2)_a-W$ wherein W is methyl or phenyl optionally substituted with $C_1$-$C_3$ alkyl, $C_1$-$C_3$ alkoxy or phenyl, r, p, t and a are integers such that the expression r+(p+1)t+a is also an integer and has a value of 3 to 20; r is greater than or equal to 2; p is greater than or equal to 2; t is greater than or equal to zero; and a is greater than or equal to zero;

(B) i is an integer from 1 to 3 and j is an integer from 1 to 6;

(C) Q is $-OR_2$,

$$-OCH_2\overset{\overset{O}{\|}}{C}OR_2 \text{ or } O\text{-}\overset{\overset{O}{\|}}{C}\text{-}R_2,$$

wherein $R_2$ is hydrogen, $C_1$-$C_6$ alkyl or $C_1$-$C_6$ alkenyl;

(D) Y is a divalent radical

$$-O\text{-}\overset{\overset{O}{\|}}{C}\text{-} ,$$

$-OCH_2-$, or

$$-O\text{-}\underset{\underset{O^{\ominus}}{|}}{\overset{\overset{O}{\|}}{P}}\text{-}O\text{-} ;$$

(E) $R_3$ is one or more $C_1$-$C_5$ alkyl, $C_1$-$C_5$ alkoxy or halogen substituents of the aromatic ring;

(F) $-CH_2A$ may be in the ortho, meta or para position wherein A is a 5-7 member aromatic heterocyclic ring containing at least one nitrogen atom and optionally one or more other nitrogen or sulfur atoms.

7. A process as claimed in Claim 1 wherein the monoclonal or polyclonal antibody directed towards Interleukin-1β is in combination with an antagonist of Platelet Activating Factor of the formula:

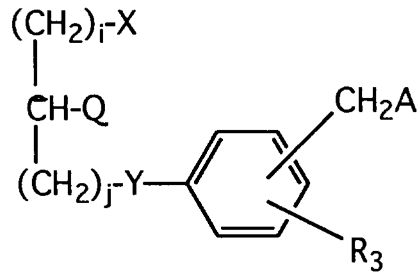

Formula II

wherein:

(A) X is

i) $C_1$-$C_{24}$ alkyl;
ii) $C_1$-$C_{24}$ alkoxy;
iii) $C_1$-$C_{24}$ carbamoyloxy;
iv)

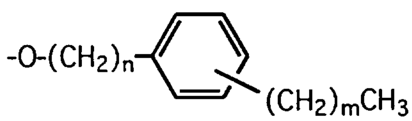 ; 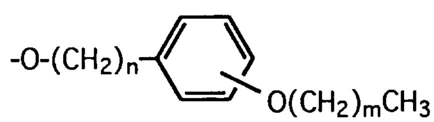 ;

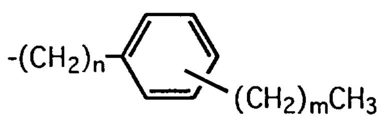 ; 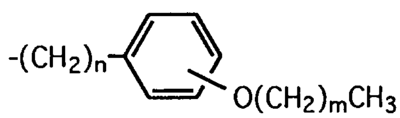 ;

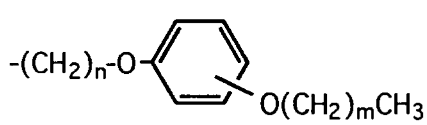 ; 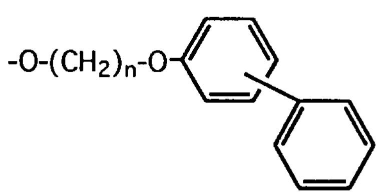

or 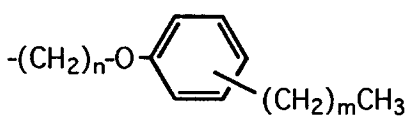 ;

n is an integer from 1 to 25 and m is an integer from 0 to 24 and the sum of n and m is less than or equal to 25;
v) phenyl;
vi) mono- or polysubstituted phenyl substituted with $C_1$-$C_{20}$ alkyl, $C_1$-$C_{20}$ alkoxy, halogen, trifluoromethyl, phenyl, substituted phenyl or benzyloxy;

vii) phenoxy;

viii) mono- or polysubstituted phenoxy substituted with $C_1$-$C_{20}$ alkyl, $C_1$-$C_{20}$ alkoxy, halogen, trifluoromethyl, phenyl, substituted phenyl or benzyloxy;

ix) naphthaloxy;

x) mono- or polysubstituted naphthaloxy substituted with $C_1$-$C_{20}$ alkyl, $C_1$-$C_{20}$ alkoxy or halogen;

xi) -O-$(CH_2)_r$-O-$((CH_2)_pO)_t$-$(CH_2)_a$-W wherein W is methyl or phenyl optionally substituted with $C_1$-$C_3$ alkyl, $C_1$-$C_3$ alkoxy or phenyl, r, p, t and a are integers such that the expression $r+(p+1)t+a$ is also an integer and has a value of 3 to 20; r is greater than or equal to 2; p is greater than or equal to 2; t is greater than or equal to zero; and a is greater than or equal to zero;

(B) i is an integer from 1 to 3 and j is an integer from 1 to 6;

(C) Q is $-OR_2$,

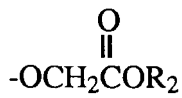 or 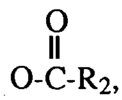

wherein $R_2$ is hydrogen, $C_1$-$C_6$ alkyl or $C_1$-$C_6$ alkenyl;

(D) Y is a divalent radical

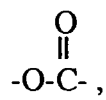

$-OCH_2-$, or

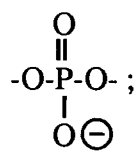

(E) $R_3$ is one or more $C_1$-$C_5$ alkyl, $C_1$-$C_5$ alkoxy or halogen substituents of the aromatic ring;

(F) $-CH_2A$ may he in the <u>ortho</u>, <u>meta</u> or <u>para</u> position wherein A is a 5-7 member aromatic heterocyclic ring containing at least one nitrogen atom and optionally one or more other nitrogen or sulfur atoms.

**8.** A process as claimed in Claim 1 wherein the monoclonal or polyclonal antibody directed towards Gamma Interferon is in combination with an antagonist of Platelet Activating Factor of the formula:

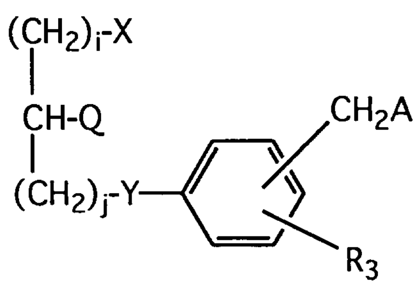

Formula II

wherein:

(A) X is

i) $C_1$-$C_{24}$ alkyl;
ii) $C_1$-$C_{24}$ alkoxy;
iii) $C_1$-$C_{24}$ carbamoyloxy;
iv)

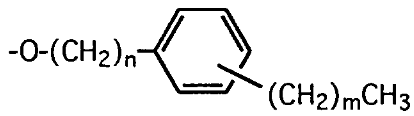 ; 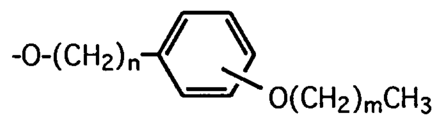 ;

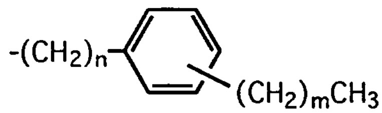 ; 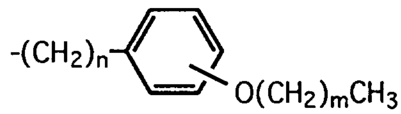 ;

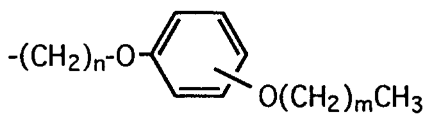 ; 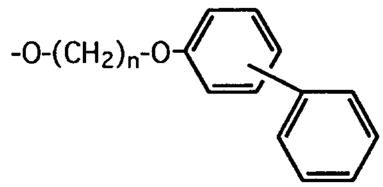

or 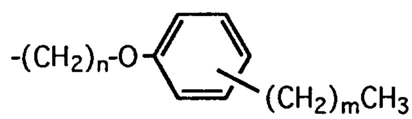 ;

n is an integer from 1 to 25 and m is an integer from 0 to 24 and the sum of n and m is less than or equal to 25;
v) phenyl;
vi) mono- or polysubstituted phenyl substituted with $C_1$-$C_{20}$ alkyl, $C_1$-$C_{20}$ alkoxy, halogen, trifluoromethyl, phenyl, substituted phenyl or benzyloxy;
vii) phenoxy;
viii) mono- or polysubstituted phenoxy substituted with $C_1$-$C_{20}$ alkyl, $C_1$-$C_{20}$ alkoxy, halogen, trifluoromethyl, phenyl, substituted phenyl or benzyloxy;
ix) naphthaloxy;
x) mono- or polysubstituted naphthaloxy substituted with $C_1$-$C_{20}$ alkyl, $C_1$-$C_{20}$ alkoxy or halogen;
xi) $-O-(CH_2)_r-O-((CH_2)_pO)_t-(CH_2)_a-W$ wherein W is methyl or phenyl optionally substituted with $C_1$-$C_3$ alkyl, $C_1$-$C_3$ alkoxy or phenyl, r, p, t and a are integers such that the expression $r+(p+1)t+a$ is also an integer and has a value of 3 to 20; r is greater than or equal to 2; p is greater than or equal to 2; t is greater than or equal to zero; and a is greater than or equal to zero;

(B) i is an integer from 1 to 3 and j is an integer from 1 to 6;

(C) Q is $-OR_2$,

$$-OCH_2\overset{\overset{\displaystyle O}{\parallel}}{C}OR_2 \text{ or } O\text{-}\overset{\overset{\displaystyle O}{\parallel}}{C}\text{-}R_2,$$

wherein $R_2$ is hydrogen, $C_1$-$C_6$ alkyl or $C_1$-$C_6$ alkenyl;

(D) Y is a divalent radical

$$-O\text{-}\overset{\overset{\displaystyle O}{\parallel}}{C}\text{-},$$

$-OCH_2-$, or

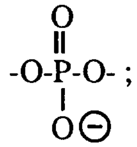

(E) $R_3$ is one or more $C_1$-$C_5$ alkyl, $C_1$-$C_5$ alkoxy or halogen substituents of the aromatic ring;

(F) $-CH_2A$ may be in the <u>ortho</u>, <u>meta</u> or <u>para</u> position wherein A is a 5-7 member aromatic heterocyclic ring containing at least one nitrogen atom and optionally one or more other nitrogen or sulfur atoms.

**9.** A process as claimed in Claim 1 wherein the monoclonal or polyclonal antibody directed towards bacterial cell wall lipopolysaccharides is in combination with an antagonist of Platelet Activating Factor of the formula:

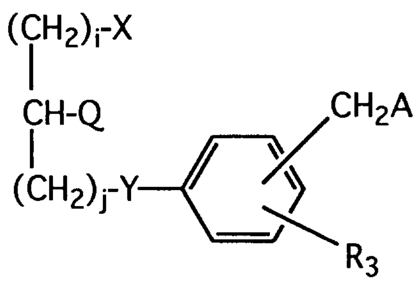

Formula II

wherein:

(A) X is

i) $C_1$-$C_{24}$ alkyl;
ii) $C_1$-$C_{24}$ alkoxy;
iii) $C_1$-$C_{24}$ carbamoyloxy;
iv)

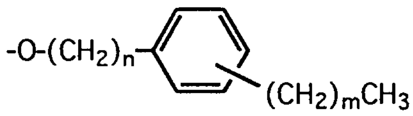 ; 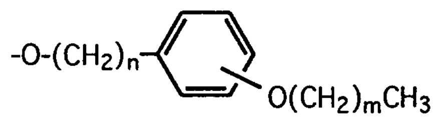 ;

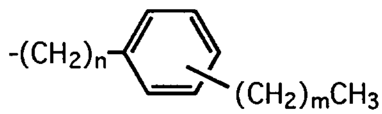 ; 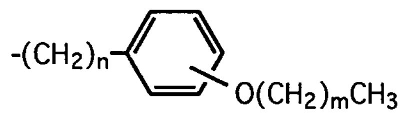 ;

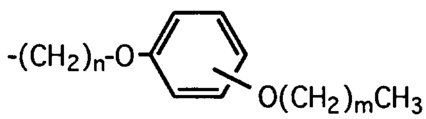 ; 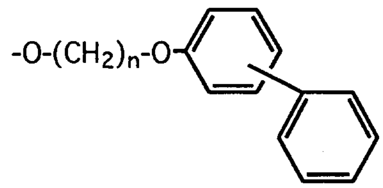

or 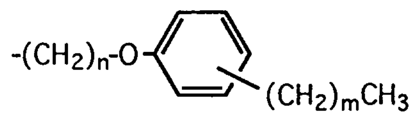 ;

n is an integer from 1 to 25 and m is an integer from 0 to 24 and the sum of n and m is less than or equal to 25;

v) phenyl;

vi) mono- or polysubstituted phenyl substituted with $C_1$-$C_{20}$ alkyl, $C_1$-$C_{20}$ alkoxy, halogen, trifluoromethyl, phenyl, substituted phenyl or benzyloxy;

vii) phenoxy;

viii) mono- or polysubstituted phenoxy substituted with $C_1$-$C_{20}$ alkyl, $C_1$-$C_{20}$ alkoxy, halogen, trifluoromethyl, phenyl, substituted phenyl or benzyloxy;

ix) naphthaloxy;

x) mono- or polysubstituted naphthaloxy substituted with $C_1$-$C_{20}$ alkyl, $C_1$-$C_{20}$ alkoxy or halogen;

xi) $-O-(CH_2)_r-O-((CH_2)_pO)_t-(CH_2)_a-W$ wherein W is methyl or phenyl optionally substituted with $C_1$-$C_3$ alkyl, $C_1$-$C_3$ alkoxy or phenyl, r, p, t and a are integers such that the expression r+(p+1)t+a is also an integer and has a value of 3 to 20; r is greater than or equal to 2; p is greater than or equal to 2; t is greater than or equal to zero; and a is greater than or equal to zero;

(B) i is an integer from 1 to 3 and j is an integer from 1 to 6;

(C) Q is $-OR_2$,

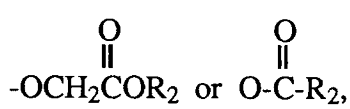

wherein $R_2$ is hydrogen, $C_1$-$C_6$ alkyl or $C_1$-$C_6$ alkenyl;

(D) Y is a divalent radical

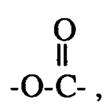

$-OCH_2-$, or

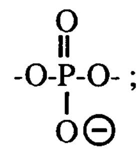

(E) $R_3$ is one or more $C_1$-$C_5$ alkyl, $C_1$-$C_5$ alkoxy or halogen substituents of the aromatic ring;

(F) $-CH_2A$ may he in the ortho, meta or para position wherein A is a 5-7 member aromatic heterocyclic ring containing at least one nitrogen atom and optionally one or more other nitrogen or sulfur atoms.

**10.** Use of a) a therapeutically effective amount of an antagonist to Platelet Activating Factor and b) one or more monoclonal or polyclonal antibodies directed towards either Tumor Necrosis Factor α, Interleukin-1β, Gamma Interferon or bacterial cell wall lipopolysaccharides in the preparation of a medicament for the treatment of endotoxic shock.

**11.** The use as claimed in Claim 10 wherein the antagonist to Platelet Activating Factor is of the formula:

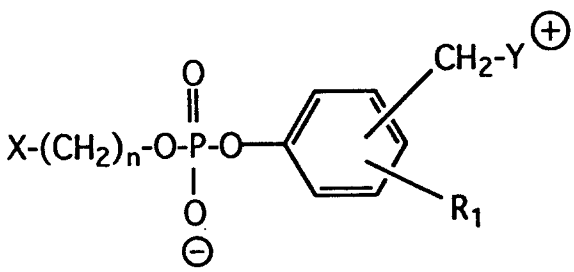

Formula I

wherein:

X is a phenyl or naphthyl ring optionally substituted in any position with one or more substituents of:

i) $-R_2$ wherein $R_2$ is $C_1$-$C_{25}$ alkyl, $C_1$-$C_{25}$ alkenyl, $C_1$-$C_{25}$ alkoxy, $C_1$-$C_{25}$ alkenyloxy, $C_1$-$C_{25}$ thioalkyl, phenyl, phenoxy, substituted phenyl or substituted phenoxy wherein the substituents are $C_1$-$C_{20}$ alkyl, $C_1$-$C_{20}$ alkoxy, halogen or trifluoromethyl;
ii) hydrogen, halogen, trifluoromethyl, cyano or nitro;
iii) $-CO_2R_3$, $-CONHR_3$, -CHO, $-OCONHR_3$, or $-NHCOR_3$ wherein $R_3$ is $C_1$-$C_{25}$ alkyl, $C_1$-$C_{25}$ alkenyl, phenyl or substituted phenyl wherein the substituents are $C_1$-$C_{20}$ alkyl, $C_1$-$C_{20}$ alkoxy, halogen, or trifluoromethyl;

$R_1$ is one or more substituents of the aromatic ring which may be in any position and is hydrogen, $C_1$-$C_5$ alkyl, $C_1$-$C_5$ alkoxy, or halogen;
$-CH_2$-Y is a single substituent of the aromatic ring which may occupy any position wherein Y is a mono or bicyclic aromatic heterocycle with 5-7 membered rings containing at least one nitrogen atom which is bonded to the methylene group and optionally one or more other nitrogen or sulfur atoms; and n is the integer 0 or 1;

and wherein the monoclonal or polyclonal antibodies are directed towards Tumor Necrosis Factor α.

**12.** The use as claimed in Claim 10 wherein the antagonist to Platelet Activating Factor is of the formula:

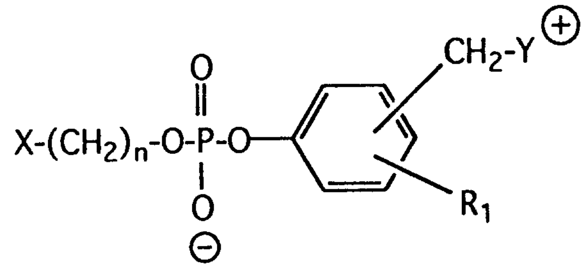

Formula I

wherein:

X is a phenyl or naphthyl ring optionally substituted in any position with one or more substituents of:

i) $-R_2$ wherein $R_2$ is $C_1$-$C_{25}$ alkyl, $C_1$-$C_{25}$ alkenyl, $C_1$-$C_{25}$ alkoxy, $C_1$-$C_{25}$ alkenyloxy, $C_1$-$C_{25}$ thioalkyl, phenyl, phenoxy, substituted phenyl or substituted phenoxy wherein the substituents are $C_1$-$C_{20}$ alkyl, $C_1$-$C_{20}$ alkoxy, halogen or trifluoromethyl;
ii) hydrogen, halogen, trifluoromethyl, cyano or nitro;
iii) $-CO_2R_3$, $-CONHR_3$, -CHO, $-OCONHR_3$, or $-NHCOR_3$ wherein $R_3$ is $C_1$-$C_{25}$ alkyl, $C_1$-$C_{25}$ alkenyl, phenyl or substituted phenyl wherein the substituents are $C_1$-$C_{20}$ alkyl, $C_1$-$C_{20}$ alkoxy, halogen, or trifluoromethyl;

$R_1$ is one or more substituents of the aromatic ring which may be in any position and is hydrogen, $C_1$-$C_5$ alkyl, $C_1$-$C_5$ alkoxy, or halogen;
$-CH_2$-Y is a single substituent of the aromatic ring which may occupy any position wherein Y is a mono or bicyclic aromatic heterocycle with 5-7 membered rings containing at least one nitrogen atom which is bonded to the methylene group and optionally one or more other nitrogen or sulfur atoms; and n is the integer 0 or 1;

and wherein the monoclonal or polyclonal antibodies are directed towards Interleukin-1β.

**13.** The use as claimed in Claim 10 wherein the antagonist to Platelet Activating Factor is of the formula:

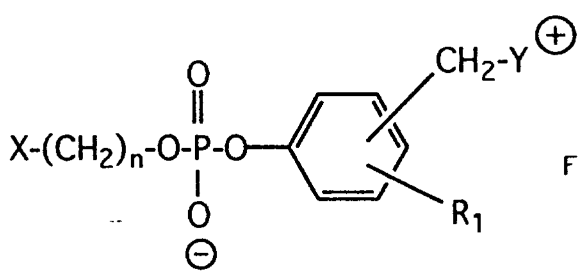

Formula I

wherein:

X is a phenyl or naphthyl ring optionally substituted in any position with one or more substituents of:

i) $-R_2$ wherein $R_2$ is $C_1$-$C_{25}$ alkyl, $C_1$-$C_{25}$ alkenyl, $C_1$-$C_{25}$ alkoxy, $C_1$-$C_{25}$ alkenyloxy, $C_1$-$C_{25}$ thioalkyl, phenyl, phenoxy, substituted phenyl or substituted phenoxy wherein the substituents are $C_1$-$C_{20}$ alkyl, $C_1$-$C_{20}$ alkoxy, halogen or trifluoromethyl;
ii) hydrogen, halogen, trifluoromethyl, cyano or nitro;
iii) $-CO_2R_3$, $-CONHR_3$, -CHO, $-OCONHR_3$, or $-NHCOR_3$ wherein $R_3$ is $C_1$-$C_{25}$ alkyl, $C_1$-$C_{25}$ alkenyl, phenyl or substituted phenyl wherein the substituents are $C_1$-$C_{20}$ alkyl, $C_1$-$C_{20}$ alkoxy, halogen, or trifluoromethyl;

$R_1$ is one or more substituents of the aromatic ring which may be in any position and is hydrogen, $C_1$-$C_5$ alkyl, $C_1$-$C_5$ alkoxy, or halogen;

$-CH_2-Y$ is a single substituent of the aromatic ring which may occupy any position wherein Y is a mono or bicyclic aromatic heterocycle with 5-7 membered rings containing at least one nitrogen atom which is bonded to the methylene group and optionally one or more other nitrogen or sulfur atoms; and n is the integer 0 or 1;

and wherein the monoclonal or polyclonal antibodies are directed towards Gamma Interferon.

**14.** The use as claimed in Claim 10 wherein the antagonist to Platelet Activating Factor is of the formula:

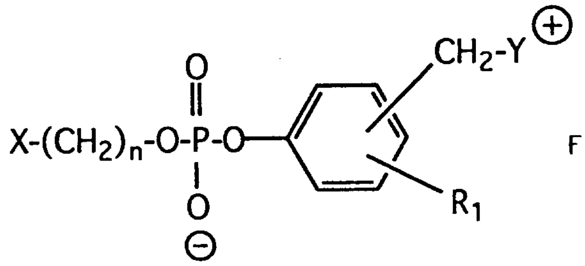

Formula I

wherein:

X is a phenyl or naphthyl ring optionally substituted in any position with one or more substituents of:

i) $-R_2$ wherein $R_2$ is $C_1$-$C_{25}$ alkyl, $C_1$-$C_{25}$ alkenyl, $C_1$-$C_{25}$ alkoxy, $C_1$-$C_{25}$ alkenyloxy, $C_1$-$C_{25}$ thioalkyl, phenyl, phenoxy, substituted phenyl or substituted phenoxy wherein the substituents are $C_1$-$C_{20}$ alkyl, $C_1$-$C_{20}$ alkoxy, halogen or trifluoromethyl;
ii) hydrogen, halogen, trifluoromethyl, cyano or nitro;
iii) $-CO_2R_3$, $-CONHR_3$, -CHO, $-OCONHR_3$, or $-NHCOR_3$ wherein $R_3$ is $C_1$-$C_{25}$ alkyl, $C_1$-$C_{25}$ alkenyl, phenyl or substituted phenyl wherein the substituents are $C_1$-$C_{20}$ alkyl, $C_1$-$C_{20}$ alkoxy, halogen, or trifluoromethyl;

$R_1$ is one or more substituents of the aromatic ring which may be in any position and is hydrogen, $C_1$-$C_5$ alkyl, $C_1$-$C_5$ alkoxy, or halogen;
$-CH_2-Y$ is a single substituent of the aromatic ring which may occupy any position wherein Y is a mono or bicyclic aromatic heterocycle with 5-7 membered rings containing at least one nitrogen atom which is bonded to the methylene group and optionally one or more other nitrogen or sulfur atoms; and n is the integer 0 or 1;

and wherein the monoclonal or polyclonal antibodies are directed towards bacterial cell wall lipopolysaccharides.

**15.** The use as claimed in Claim 10 wherein the antagonist to Platelet Activating Factor is of the formula:

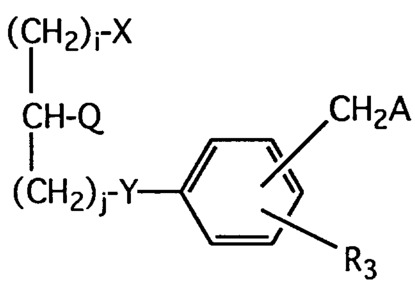

Formula II

wherein:

(A) X is

i) $C_1$-$C_{24}$ alkyl;
ii) $C_1$-$C_{24}$ alkoxy;
iii) $C_1$-$C_{24}$ carbamoyloxy;
iv)

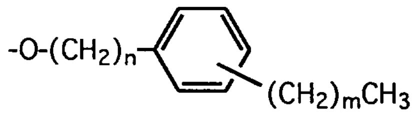 ; 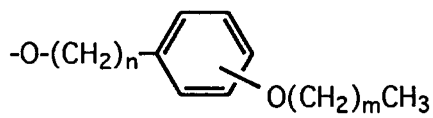 ;

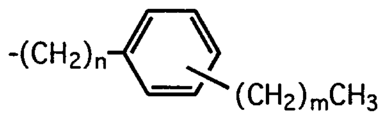 ; 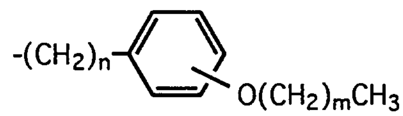 ;

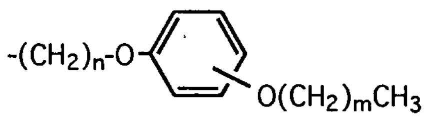 ; 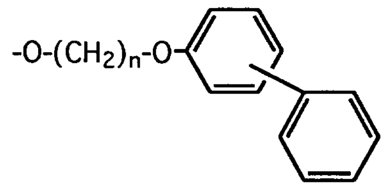

or 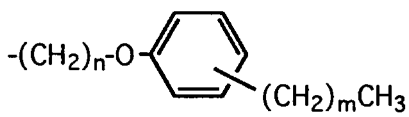 ;

n is an integer from 1 to 25 and m is an integer from 0 to 24 and the sum of n and m is less than or equal to 25;
v) phenyl;
vi) mono- or polysubstituted phenyl substituted with $C_1$-$C_{20}$ alkyl, $C_1$-$C_{20}$ alkoxy, halogen, trifluoromethyl, phenyl, substituted phenyl or benzyloxy;
vii) phenoxy;
viii) mono- or polysubstituted phenoxy substituted with $C_1$-$C_{20}$ alkyl, $C_1$-$C_{20}$ alkoxy, halogen, trifluoromethyl, phenyl, substituted phenyl or benzyloxy;
ix) naphthaloxy;
x) mono- or polysubstituted naphthaloxy substituted with $C_1$-$C_{20}$ alkyl, $C_1$-$C_{20}$ alkoxy or halogen;
xi) $-O-(CH_2)_r-O-((CH_2)_pO)_t-(CH_2)_a-W$ wherein W is methyl or phenyl optionally substituted with $C_1$-$C_3$ alkyl, $C_1$-$C_3$ alkoxy or phenyl, r, p, t and a are integers such that the expression $r+(p+1)t+a$ is also an integer and has a value of 3 to 20; r is greater than or equal to 2; p is greater than or equal to 2; t is greater than or equal to zero; and a is greater than or equal to zero;

(B) i is an integer from 1 to 3 and j is an integer from 1 to 6;

(C) Q is $-OR_2$,

$$-OCH_2\overset{\overset{O}{\|}}{C}OR_2 \text{ or } O-\overset{\overset{O}{\|}}{C}-R_2,$$

wherein $R_2$ is hydrogen, $C_1$-$C_6$ alkyl or $C_1$-$C_6$ alkenyl;

(D) Y is a divalent radical

$$-O-\overset{\overset{O}{\|}}{C}- ,$$

$-OCH_2-$, or

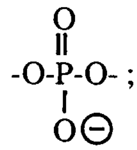

(E) $R_3$ is one or more $C_1$-$C_5$ alkyl, $C_1$-$C_5$ alkoxy or halogen substituents of the aromatic ring;

(F) $-CH_2A$ may be in the ortho, meta or para position wherein A is a 5-7 member aromatic heterocyclic ring containing at least one nitrogen atom and optionally one or more other nitrogen or sulfur atoms;

and wherein the monoclonal or polyclonal antibodies are directed towards Tumor Necrosis Factor $\alpha$.

**16.** The use as claimed in Claim 10 wherein the antagonist to Platelet Activating Factor is of the formula:

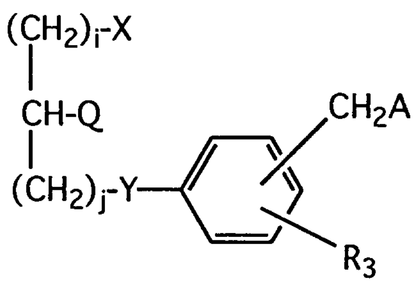

Formula II

wherein:

(A) X is

i) $C_1$-$C_{24}$ alkyl;
ii) $C_1$-$C_{24}$ alkoxy;
iii) $C_1$-$C_{24}$ carbamoyloxy;
iv)

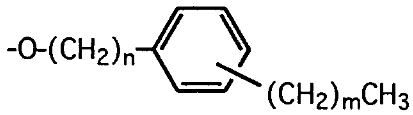 ; 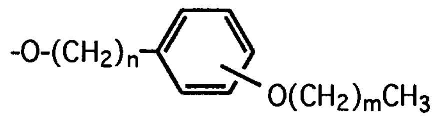 ;

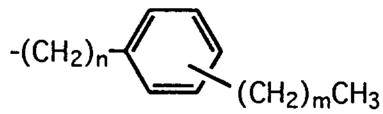 ; 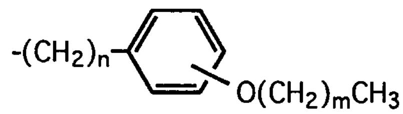 ;

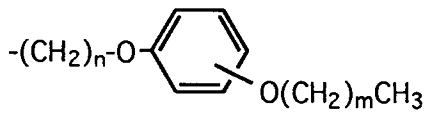 ; 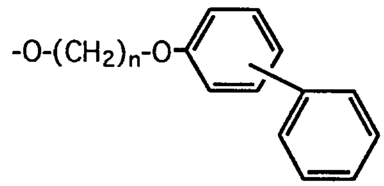

or 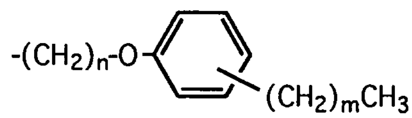 ;

n is an integer from 1 to 25 and m is an integer from 0 to 24 and the sum of n and m is less than or equal to 25;

v) phenyl;

vi) mono- or polysubstituted phenyl substituted with $C_1$-$C_{20}$ alkyl, $C_1$-$C_{20}$ alkoxy, halogen, trifluoromethyl, phenyl, substituted phenyl or benzyloxy;

vii) phenoxy;

viii) mono- or polysubstituted phenoxy substituted with $C_1$-$C_{20}$ alkyl, $C_1$-$C_{20}$ alkoxy, halogen, trifluoromethyl, phenyl, substituted phenyl or benzyloxy;

ix) naphthaloxy;

x) mono- or polysubstituted naphthaloxy substituted with $C_1$-$C_{20}$ alkyl, $C_1$-$C_{20}$ alkoxy or halogen;

xi) $-O-(CH_2)_r-O-((CH_2)_pO)_t-(CH_2)_a-W$ wherein W is methyl or phenyl optionally substituted with $C_1$-$C_3$ alkyl, $C_1$-$C_3$ alkoxy or phenyl, r, p, t and a are integers such that the expression r+(p+1)t+a is also an integer and has a value of 3 to 20; r is greater than or equal to 2; p is greater than or equal to 2; t is greater than or equal to zero; and a is greater than or equal to zero;

(B) i is an integer from 1 to 3 and j is an integer from 1 to 6;

(C) Q is $-OR_2$,

$$-OCH_2\overset{\overset{O}{\|}}{C}OR_2 \text{ or } O\text{-}\overset{\overset{O}{\|}}{C}\text{-}R_2,$$

wherein $R_2$ is hydrogen, $C_1$-$C_6$ alkyl or $C_1$-$C_6$ alkenyl;

(D) Y is a divalent radical

$$-O\text{-}\overset{\overset{O}{\|}}{C}\text{-} ,$$

$-OCH_2-$, or

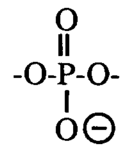 ;

(E) $R_3$ is one or more $C_1$-$C_5$ alkyl, $C_1$-$C_5$ alkoxy or halogen substituents of the aromatic ring;

(F) -$CH_2$A may be in the <u>ortho</u>, <u>meta</u> or <u>para</u> position wherein A is a 5-7 member aromatic heterocyclic ring containing at least one nitrogen atom and optionally one or more other nitrogen or sulfur atoms;

and wherein the monoclonal or polyclonal antibodies are directed towards Interleukin-1β.

**17.** The use as claimed in Claim 10 wherein the antagonist to Platelet Activating Factor is of the formula:

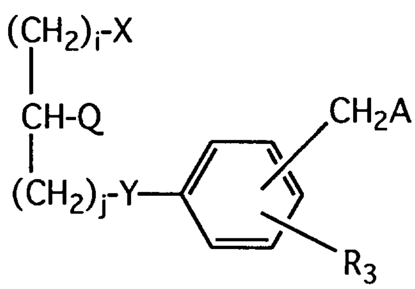

Formula II

wherein:

(A) X is

i) $C_1$-$C_{24}$ alkyl;
ii) $C_1$-$C_{24}$ alkoxy;
iii) $C_1$-$C_{24}$ carbamoyloxy;
iv)

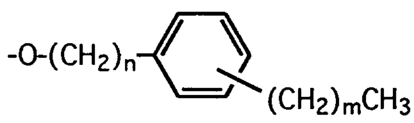 ; 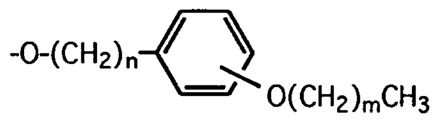 ;

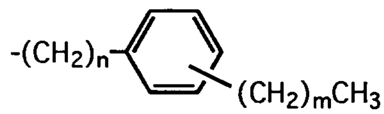 ; 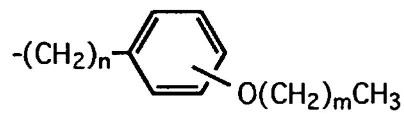 ;

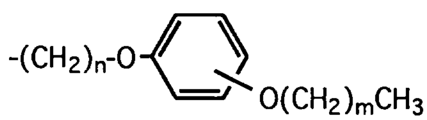 ; 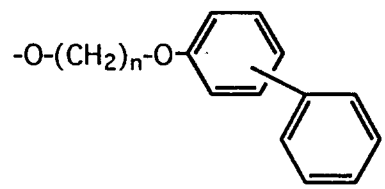

or 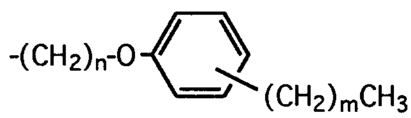 ;

n is an integer from 1 to 25 and m is an integer from 0 to 24 and the sum of n and m is less than or equal to 25;

v) phenyl;

vi) mono- or polysubstituted phenyl substituted with $C_1$-$C_{20}$ alkyl, $C_1$-$C_{20}$ alkoxy, halogen, trifluoromethyl, phenyl, substituted phenyl or benzyloxy;

vii) phenoxy;

viii) mono- or polysubstituted phenoxy substituted with $C_1$-$C_{20}$ alkyl, $C_1$-$C_{20}$ alkoxy, halogen, trifluoromethyl, phenyl, substituted phenyl or benzyloxy;

ix) naphthaloxy;

x) mono- or polysubstituted naphthaloxy substituted with $C_1$-$C_{20}$ alkyl, $C_1$-$C_{20}$ alkoxy or halogen;

xi) $-O-(CH_2)_r-O-((CH_2)_pO)_t-(CH_2)_a-W$ wherein W is methyl or phenyl optionally substituted with $C_1$-$C_3$ alkyl, $C_1$-$C_3$ alkoxy or phenyl, r, p, t and a are integers such that the expression r+(p+1)t+a is also an integer and has a value of 3 to 20; r is greater than or equal to 2; p is greater than or equal to 2; t is greater than or equal to zero; and a is greater than or equal to zero;

(B) i is an integer from 1 to 3 and j is an integer from 1 to 6;

(C) Q is $-OR_2$,

$$-OCH_2\overset{O}{\overset{\|}{C}}OR_2 \text{ or } O\text{-}\overset{O}{\overset{\|}{C}}\text{-}R_2,$$

wherein $R_2$ is hydrogen, $C_1$-$C_6$ alkyl or $C_1$-$C_6$ alkenyl;

(D) Y is a divalent radical

$$-O\text{-}\overset{O}{\overset{\|}{C}}\text{-}\ ,$$

$-OCH_2-$, or

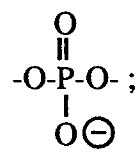

(E) $R_3$ is one or more $C_1$-$C_5$ alkyl, $C_1$-$C_5$ alkoxy or halogen substituents of the aromatic ring;

(F) -$CH_2$A may be in the ortho, meta or para position wherein A is a 5-7 member aromatic heterocyclic ring containing at least one nitrogen atom and optionally one or more other nitrogen or sulfur atoms;

and wherein the monoclonal or polyclonal antibodies are directed towards Gamma Interferon.

**18.** The use as claimed in Claim 10 wherein the antagonist to Platelet Activating Factor is of the formula:

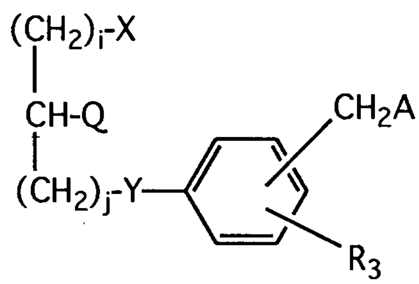

Formula II

wherein:

(A) X is

i) $C_1$-$C_{24}$ alkyl;
ii) $C_1$-$C_{24}$ alkoxy;
iii) $C_1$-$C_{24}$ carbamoyloxy;
iv)

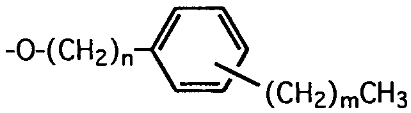 ; 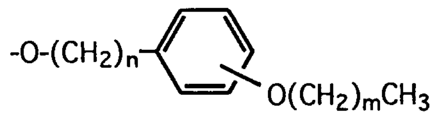 ;

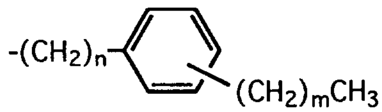 ; 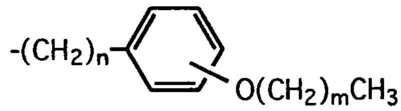 ;

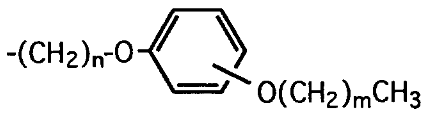 ; 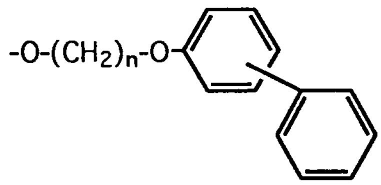

or 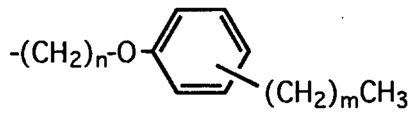 ;

n is an integer from 1 to 25 and m is an integer from 0 to 24 and the sum of n and m is less than or equal to 25;

v) phenyl;

vi) mono- or polysubstituted phenyl substituted with $C_1$-$C_{20}$ alkyl, $C_1$-$C_{20}$ alkoxy, halogen, trifluoromethyl, phenyl, substituted phenyl or benzyloxy;

vii) phenoxy;

viii) mono- or polysubstituted phenoxy substituted with $C_1$-$C_{20}$ alkyl, $C_1$-$C_{20}$ alkoxy, halogen, trifluoromethyl, phenyl, substituted phenyl or benzyloxy;

ix) naphthaloxy;

x) mono- or polysubstituted naphthaloxy substituted with $C_1$-$C_{20}$ alkyl, $C_1$-$C_{20}$ alkoxy or halogen;

xi) $-O-(CH_2)_r-O-((CH_2)_pO)_t-(CH_2)_a-W$ wherein W is methyl or phenyl optionally substituted with $C_1$-$C_3$ alkyl, $C_1$-$C_3$ alkoxy or phenyl, r, p, t and a are integers such that the expression r+(p+1)t+a is also an integer and has a value of 3 to 20; r is greater than or equal to 2; p is greater than or equal to 2; t is greater than or equal to zero; and a is greater than or equal to zero;

(B) i is an integer from 1 to 3 and j is an integer from 1 to 6;

(C) Q is $-OR_2$,

$$-OCH_2\overset{\overset{\displaystyle O}{\|}}{C}OR_2 \text{ or } O\text{-}\overset{\overset{\displaystyle O}{\|}}{C}\text{-}R_2,$$

wherein $R_2$ is hydrogen, $C_1$-$C_6$ alkyl or $C_1$-$C_6$ alkenyl;

(D) Y is a divalent radical

$$-O\text{-}\overset{\overset{\displaystyle O}{\|}}{C}\text{-}\ ,$$

$-OCH_2-$, or

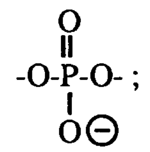

(E) $R_3$ is one or more $C_1$-$C_5$ alkyl, $C_1$-$C_5$ alkoxy or halogen substituents of the aromatic ring;
(F) -$CH_2$A may be in the ortho, meta or para position wherein A is a 5-7 member aromatic heterocyclic ring containing at least one nitrogen atom and optionally one or more other nitrogen or sulfur atoms;

and wherein the monoclonal or polyclonal antibodies are directed towards bacterial cell wall lipopolysaccharides.

**Patentansprüche**

**Patentansprüche für folgende Vertragsstaaten : AT, BE, CH, DE, FR, GB, IT, LI, NL, SE**

1. Produkte, enthaltend in therapeutisch wirksamen Mengen einen Antagonisten für den ThrombozytenAktivierungsfaktor und einen oder mehrere monoclonale oder polyclonale Antikörper, gerichtet gegen entweder Tumor-Nekrose-Faktor $\alpha$, Interleukin-1$\beta$, $\gamma$-Interferon oder bakterielle Zellwand-Lipopolysaccharide als Kombinationszubereitung zur gleichzeitigen, getrennten oder schrittweisen Verwendung bei der Behandlung von Endotoxinschock.

2. Produkt nach Anspruch 1, wobei ein gegen Tumor-Nekrose-Faktor $\alpha$ gerichteter monoclonaler Antikörper, kombiniert ist mit einem Antagonisten vom Thrombozyten-Aktivierungsfaktor der Formel

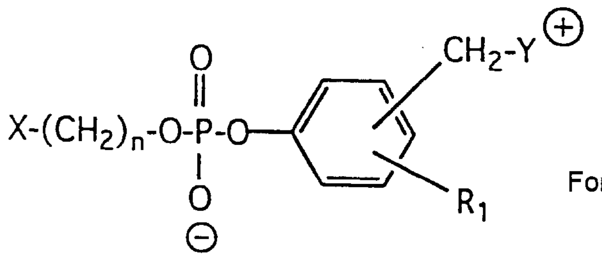

Formel I

worin X einen Phenyl- oder Naphthylring darstellt, gegebenenfalls substituiert in einer Position mit einem oder mehreren Substituenten, von:

i) -$R_2$, worin $R_2$ $C_1$-$C_{25}$-Alkyl, $C_1$-$C_{25}$-Alkenyl, $C_1$-$C_{25}$-Alkoxy, $C_1$-$C_{25}$-Alkenyloxy, $C_1$-$C_{25}$-Thioalkyl, Phenyl, Phenoxy, substituiertes Phenyl oder substituiertes Phenoxy, worin die Substituenten $C_1$-$C_{20}$-Alkyl, $C_1$-$C_{20}$-Alkoxy, Halogen oder Trifluormethyl darstellen, darstellt;
ii) Wasserstoff, Halogen, Trifluormethyl, Cyano oder Nitro;
iii) -$CO_2R_3$, -$CONHR_3$, -CHO, $OCONHR_3$ oder -$NHCOR_3$, worin $R_3$ $C_1$-$C_{25}$-Alkyl, $C_1$-$C_{25}$-Alkenyl, Phenyl oder substituiertes Phenyl, worin die Substituenten $C_1$-$C_{20}$-Alkyl, $C_1$-$C_{20}$-Alkoxy, Halogen oder Trifluormethyl darstellen, darstellt;

$R_1$ einen oder mehrere Substituenten des aromatischen Rings darstellt, die in beliebiger Position vorliegen können und Wasserstoff, $C_1$-$C_5$-Alkyl, $C_1$-$C_5$-Alkoxy oder Halogen darstellen;
-$CH_2$-Y einen einzelnen Substituenten des aromatischen Rings darstellt, der eine beliebige Position besetzen kann, worin Y einen mono- oder bicyclischen aromatischen Heterocyclus darstellt mit 5-7 gliedrigen Ringen, die mindestens ein Stickstoffatom, das an die Methylengruppe gebunden ist und gegebenenfalls eines oder mehrere andere Stickstoff- oder Schwefelatome enthalten und n die ganze Zahl 0 oder 1 ist.

3. Produkte nach Anspruch 1, wobei ein gegen Interleukin-1$\beta$ gerichteter monoclonaler Antikörper, mit einem Antagonisten vom Thrombozyten-Aktivierungsfaktor nach Anspruch 2 kombiniert ist.

4. Produkte nach Anspruch 1, wobei ein gegen $\gamma$-Interferon gerichteter monoclonaler Antikörper, mit einem Antagonisten vom Thrombozyten-Aktivierungsfaktor nach Anspruch 2 kombiniert ist.

5. Produkte nach Anspruch 1, wobei ein gegen bakterielles Zellwand-Lipopolysaccharid gerichteter monoclonaler Antikörper, mit einem Antagonisten vom Thrombozyten-Aktivierungsfaktor nach Anspruch 2 kombiniert ist.

6. Produkte nach Anspruch 1, worin ein gegen Tumor-Nekrose-Faktor α gerichteter monoclonaler Antikörper, kombiniert ist mit einem Antagonisten vom Thrombozyten-Aktivierungsfaktor der Formel:

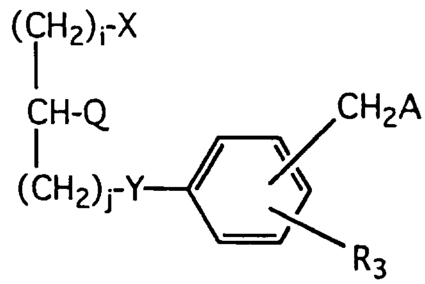

Formel II

worin:

(A) X darstellt

i) $C_1$-$C_{24}$-Alkyl;
ii) $C_1$-$C_{24}$-Alkoxy;
iii) $C_1$-$C_{24}$-Carbamoyloxy;
iv)

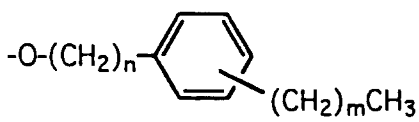 ; 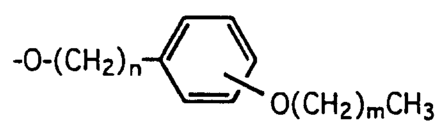 ;

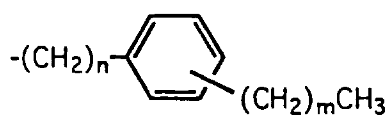 ; 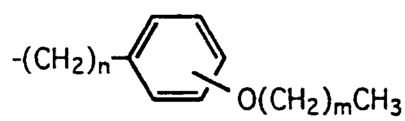 ;

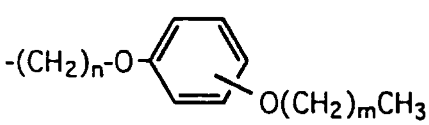 ; 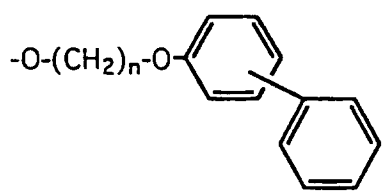

oder 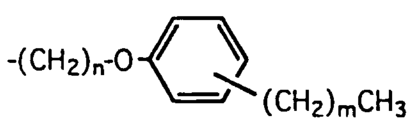 ;

n eine ganze Zahl von 1 bis 25 ist und m eine ganze Zahl von 0 bis 24 ist und die Summe von n und m geringer als oder gleich 25 ist;
v) Phenyl;
vi) mono- oder polysubstituiertes Phenyl, substituiert mit $C_1$-$C_{20}$-Alkyl, $C_1$-$C_{20}$-Alkoxy, Halogen, Trifluormethyl, Phenyl, substituiertem Phenyl oder Benzyloxy;
vii) Phenoxy;
viii) mono- oder polysubstituiertes Phenoxy, substituiert mit $C_1$-$C_{20}$-Alkyl, $C_1$-$C_{20}$-Alkoxy, Halogen, Trifluormethyl, Phenyl, substituiertem Phenyl oder Benzyloxy;

ix) Naphthaloxy;

x) mono- oder polysubstituiertes Naphthaloxy, substituiert mit $C_1$-$C_{20}$-Alkyl, $C_1$-$C_{20}$-Alkoxy oder Halogen;

xi) $-O-(CH_2)_r-O-((CH_2)_pO)_t-(CH_2)_a-W$, worin W Methyl oder Phenyl, gegebenenfalls substituiert mit $C_1$-$C_3$-Alkyl, $C_1$-$C_3$-Alkoxy oder Phenyl, darstellt, r, p, t und a ganze Zahlen sind, so daß der Ausdruck r+(p+1)t+a ebenfalls eine ganze Zahl ergibt und einen Wert von 3 bis 20 aufweist; r größer als oder gleich 2 ist; p größer als oder gleich 2 ist; t größer als oder gleich 0 ist; und a größer als oder gleich 0 ist;

(B) i eine ganze Zahl von 1 bis 3 ist und j eine ganze Zahl von 1 bis 6 ist;

(C) Q $-OR_2$,

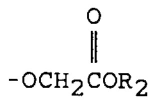 oder 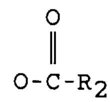

darstellt, worin $R_2$ Wasserstoff, $C_1$-$C_6$-Alkyl oder $C_1$-$C_6$-Alkenyl darstellt;

(D) Y einen zweiwertigen Rest

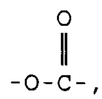

$-OCH_2-$ oder

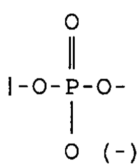

darstellt;

(E) $R_3$ einen oder mehrere $C_1$-$C_5$-Alkyl-, $C_1$-$C_5$-Alkoxy- oder Halogen-Substituenten des aromatischen Rings darstellt;

(F) $-CH_2A$ in der ortho-, meta- oder para-Position sein kann, worin A einen 5-7-gliedrigen aromatischen heterocyclischen Ring darstellt, der mindestens ein Stickstoffatom und gegebenenfalls eines oder mehrere weitere Stickstoff- oder Schwefelatome enthält.

7. Produkte nach Anspruch 1, wobei ein gegen Interleukin-1β gerichteter monoclonaler Antikörper, mit einem Antagonisten vom Thrombozyten-Aktivierungsfaktor nach Anspruch 6 kombiniert ist.

8. Produkte nach Anspruch 1, wobei ein gegen γ-Interferon gerichteter monoclonaler Antikörper, mit einem Antagonisten vom Thrombozyten-Aktivierungsfaktor nach Anspruch 6 kombiniert ist.

9. Produkte nach Anspruch 1, wobei ein gegen bakterielle Zellwand-Lipopolysaccharide gerichteter monoclonaler Antikörper, mit einem Antagonisten vom Thrombozyten-Aktivierungsfaktor nach Anspruch 6 kombiniert ist.

**Patentansprüche für folgende Vertragsstaaten : ES, GR**

1. Verfahren zur Herstellung eines Produktes zur Verwendung bei der Behandlung von Endotoxinschock in einem Säuger, umfassend Vereinigen einer therapeutisch wirksamen Menge eines Antagonisten für den Thrombozyten-Aktivierungsfaktor und eines oder mehrerer monoclonaler oder polyclonaler Antikörper, gerichtet gegen entweder Tumor-Nekrose-Faktor α und Interleukin-1β, γ-Interferon oder bakterielles Zellwand-Lipopolysaccharid unter Bereitstellung einer Zubereitung zur gleichzeitigen, getrennten oder schrittweisen Verwendung.

2. Verfahren nach Anspruch 1, wobei der monoclonale oder polyclonale Antikörper gerichtet ist gegen Tumor-Nekrose-Faktor α in Kombination mit einem Antagonisten vom Thrombozyten-Aktivierungsfaktor der Formel

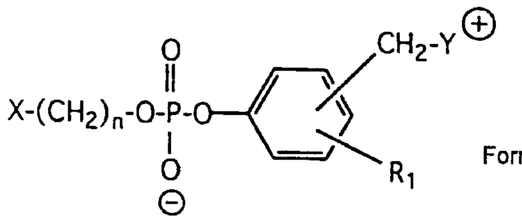

Formel I

worin X einen Phenyl- oder Naphthylring darstellt, gegebenenfalls substituiert in einer Position mit einem oder mehreren Substituenten, von:

i) $-R_2$, worin $R_2$ $C_1$-$C_{25}$-Alkyl, $C_1$-$C_{25}$-Alkenyl, $C_1$-$C_{25}$-Alkoxy, $C_1$-$C_{25}$-Alkenyloxy, $C_1$-$C_{25}$-Thioalkyl, Phenyl, Phenoxy, substituiertes Phenyl oder substituiertes Phenoxy, worin die Substituenten $C_1$-$C_{20}$-Alkyl, $C_1$-$C_{20}$-Alkoxy, Halogen oder Trifluormethyl darstellen, darstellt;
ii) Wasserstoff, Halogen, Trifluormethyl, Cyano oder Nitro;
iii) $-CO_2R_3$, $-CONHR_3$, -CHO, $OCONHR_3$ oder $-NHCOR_3$, worin $R_3$ $C_1$-$C_{25}$-Alkyl, $C_1$-$C_{25}$-Alkenyl, Phenyl oder substituiertes Phenyl, worin die Substituenten $C_1$-$C_{20}$-Alkyl, $C_1$-$C_{20}$-Alkoxy, Halogen oder Trifluormethyl darstellen, darstellt;

$R_1$ einen oder mehrere Substituenten des aromatischen Rings darstellt, die in beliebiger Position vorliegen können und Wasserstoff, $C_1$-$C_5$-Alkyl, $C_1$-$C_5$-Alkoxy oder Halogen darstellen;
$-CH_2$-Y einen einzelnen Substituenten des aromatischen Rings darstellt, der eine beliebige Position besetzen kann, worin Y einen mono- oder bicyclischen aromatischen Heterocyclus darstellt mit 5-7 gliedrigen Ringen, die mindestens ein Stickstoffatom, das an die Methylengruppe gebunden ist und gegebenenfalls eines oder mehrere andere Stickstoff- oder Schwefelatome enthalten und n die ganze Zahl 0 oder 1 ist.

3. Verfahren nach Anspruch 1, wobei der monoclonale oder polyclonale Antikörper gerichtet ist gegen Interleukin-1β in Kombination mit einem Antagonisten vom Thrombozyten-Aktivierungsfaktor der Formel

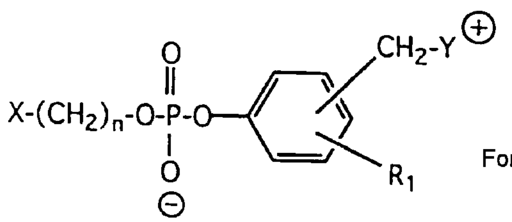

Formel I

worin X einen Phenyl- oder Naphthylring darstellt, gegebenenfalls substituiert in einer Position mit einem oder mehreren Substituenten, von:

i) $-R_2$, worin $R_2$ $C_1$-$C_{25}$-Alkyl, $C_1$-$C_{25}$-Alkenyl, $C_1$-$C_{25}$-Alkoxy, $C_1$-$C_{25}$-Alkenyloxy, $C_1$-$C_{25}$-Thioalkyl, Phenyl, Phenoxy, substituiertes Phenyl oder substituiertes Phenoxy, worin die Substituenten $C_1$-$C_{20}$-Alkyl, $C_1$-$C_{20}$-Alkoxy, Halogen oder Trifluormethyl darstellen, darstellt;
ii) Wasserstoff, Halogen, Trifluormethyl, Cyano oder Nitro;
iii) $-CO_2R_3$, $-CONHR_3$, -CHO, $OCONHR_3$ oder $-NHCOR_3$, worin $R_3$ $C_1$-$C_{25}$-Alkyl, $C_1$-$C_{25}$-Alkenyl, Phenyl oder substituiertes Phenyl, worin die Substituenten $C_1$-$C_{20}$-Alkyl, $C_1$-$C_{20}$-Alkoxy, Halogen oder Trifluormethyl darstellen, darstellt;
$R_1$ einen oder mehrere Substituenten des aromatischen Rings darstellt, die in beliebiger Position vorliegen können und Wasserstoff, $C_1$-$C_5$-Alkyl, $C_1$-$C_5$-Alkoxy oder Halogen darstellen;
$-CH_2$-Y einen einzelnen Substituenten des aromatischen Rings darstellt, der eine beliebige Position besetzen kann, worin Y einen mono- oder bicyclischen aromatischen Heterocyclus darstellt mit 5-7 gliedrigen Ringen, die mindestens ein Stickstoffatom, das an die Methylengruppe gebunden ist und gegebenenfalls eines oder mehrere andere Stickstoff- oder Schwefelatome enthalten und n die ganze Zahl 0 oder 1 ist.

4. Verfahren nach Anspruch 1, wobei der monoclonale oder polyclonale Antikörper gerichtet ist gegen γ-Interferon in Kombination mit einem Antagonisten vom Thrombozyten-Aktivierungsfaktor der Formel

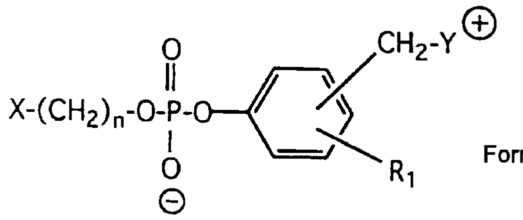

Formel I

worin X einen Phenyl- oder Naphthylring darstellt, gegebenenfalls substituiert in einer Position mit einem oder mehreren Substituenten, von:

i) $-R_2$, worin $R_2$ $C_1$-$C_{25}$-Alkyl, $C_1$-$C_{25}$-Alkenyl, $C_1$-$C_{25}$-Alkoxy, $C_1$-$C_{25}$-Alkenyloxy, $C_1$-$C_{25}$-Thioalkyl, Phenyl, Phenoxy, substituiertes Phenyl oder substituiertes Phenoxy, worin die Substituenten $C_1$-$C_{20}$-Alkyl, $C_1$-$C_{20}$-Alkoxy, Halogen oder Trifluormethyl darstellen, darstellt;
ii) Wasserstoff, Halogen, Trifluormethyl, Cyano oder Nitro;
iii) $-CO_2R_3$, $-CONHR_3$, -CHO, $OCONHR_3$ oder $-NHCOR_3$, worin $R_3$ $C_1$-$C_{25}$-Alkyl, $C_1$-$C_{25}$-Alkenyl, Phenyl oder substituiertes Phenyl, worin die Substituenten $C_1$-$C_{20}$-Alkyl, $C_1$-$C_{20}$-Alkoxy, Halogen oder Trifluormethyl darstellen, darstellt;

$R_1$ einen oder mehrere Substituenten des aromatischen Rings darstellt, die in beliebiger Position vorliegen können und Wasserstoff, $C_1$-$C_5$-Alkyl, $C_1$-$C_5$-Alkoxy oder Halogen darstellen;

$-CH_2$-Y einen einzelnen Substituenten des aromatischen Rings darstellt, der eine beliebige Position besetzen kann, worin Y einen mono- oder bicyclischen aromatischen Heterocyclus darstellt mit 5-7 gliedrigen Ringen, die mindestens ein Stickstoffatom, das an die Methylengruppe gebunden ist und gegebenenfalls eines oder mehrere andere Stickstoff- oder Schwefelatome enthalten und n die ganze Zahl 0 oder 1 ist.

**5.** Verfahren nach Anspruch 1, wobei der monoclonale oder polyclonale Antikörper gerichtet ist gegen bakterielles Zellwand-Lipopolysaccharid in Kombination mit einem Antagonisten vom Thrombozyten-Aktivierungsfaktor der Formel

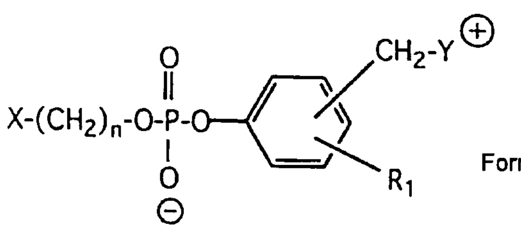

Formel I

worin X einen Phenyl- oder Naphthylring darstellt, gegebenenfalls substituiert in einer Position mit einem oder mehreren Substituenten, von:

i) $-R_2$, worin $R_2$ $C_1$-$C_{25}$-Alkyl, $C_1$-$C_{25}$-Alkenyl, $C_1$-$C_{25}$-Alkoxy, $C_1$-$C_{25}$-Alkenyloxy, $C_1$-$C_{25}$-Thioalkyl, Phenyl, Phenoxy, substituiertes Phenyl oder substituiertes Phenoxy, worin die Substituenten $C_1$-$C_{20}$-Alkyl, $C_1$-$C_{20}$-Alkoxy, Halogen oder Trifluormethyl darstellen, darstellt;
ii) Wasserstoff, Halogen, Trifluormethyl, Cyano oder Nitro;
iii) $-CO_2R_3$, $-CONHR_3$, -CHO, $OCONHR_3$ oder $-NHCOR_3$, worin $R_3$ $C_1$-$C_{25}$-Alkyl, $C_1$-$C_{25}$-Alkenyl, Phenyl oder substituiertes Phenyl, worin die Substituenten $C_1$-$C_{20}$-Alkyl, $C_1$-$C_{20}$-Alkoxy, Halogen oder Trifluormethyl darstellen, darstellt;

$R_1$ einen oder mehrere Substituenten des aromatischen Rings darstellt, die in beliebiger Position vorliegen können und Wasserstoff, $C_1$-$C_5$-Alkyl, $C_1$-$C_5$-Alkoxy oder Halogen darstellen;

$-CH_2$-Y einen einzelnen Substituenten des aromatischen Rings darstellt, der eine beliebige Position besetzen kann, worin Y einen mono- oder bicyclischen aromatischen Heterocyclus darstellt mit 5-7 gliedrigen Ringen, die mindestens ein Stickstoffatom, das an die Methylengruppe gebunden ist und gegebenenfalls eines oder mehrere andere Stickstoff- oder Schwefelatome enthalten und n die ganze Zahl 0 oder 1 ist.

**6.** Verfahren nach Anspruch 1, wobei der monoclonale oder polyclonale Antikörper gerichtet ist gegen Tumor-Nekrose-Faktor α in Kombination mit einem Antagonisten vom Thrombozyten-Aktivierungsfaktor der Formel

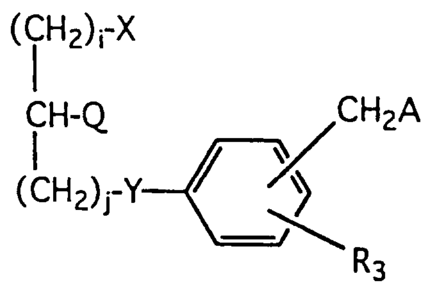

Formel II

worin:

(A) X darstellt

i) $C_1$-$C_{24}$-Alkyl;
ii) $C_1$-$C_{24}$-Alkoxy;
iii) $C_1$-$C_{24}$-Carbamoyloxy;
iv)

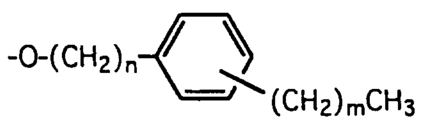 ; 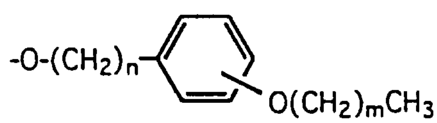 ;

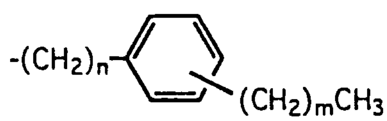 ; 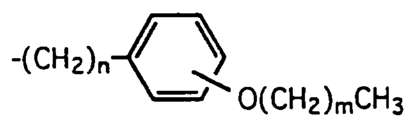 ;

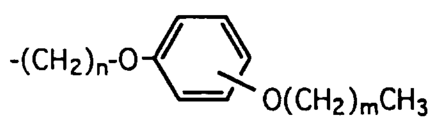 ; 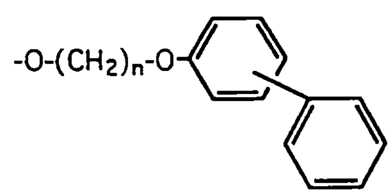

oder 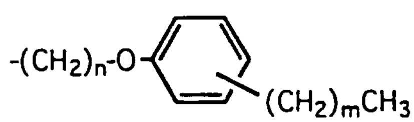 ;

n eine ganze Zahl von 1 bis 25 ist und m eine ganze Zahl von 0 bis 24 ist und die Summe von n und m geringer als oder gleich 25 ist;
v) Phenyl;
vi) mono- oder polysubstituiertes Phenyl, substituiert mit $C_1$-$C_{20}$-Alkyl, $C_1$-$C_{20}$-Alkoxy, Halogen, Trifluormethyl, Phenyl, substituiertem Phenyl oder Benzyloxy;
vii) Phenoxy;
viii) mono- oder polysubstituiertes Phenoxy, substituiert mit $C_1$-$C_{20}$-Alkyl, $C_1$-$C_{20}$-Alkoxy, Halogen, Trifluormethyl, Phenyl, substituiertem Phenyl oder Benzyloxy;
ix) Naphthaloxy;
x) mono- oder polysubstituiertes Naphthaloxy, substituiert mit $C_1$-$C_{20}$-Alkyl, $C_1$-$C_{20}$-Alkoxy oder Halogen;
xi) $-O-(CH_2)_r-O-((CH_2)_pO)_t-(CH_2)_a-W$, worin W Methyl oder Phenyl, gegebenenfalls substituiert mit $C_1$-$C_3$-Alkyl, $C_1$-$C_3$-Alkoxy oder Phenyl, darstellt, r, p, t und a ganze Zahlen sind, so daß der Ausdruck $r+(p+1)t+a$ ebenfalls eine ganze Zahl ergibt und einen Wert von 3 bis 20 aufweist; r größer als oder gleich 2 ist; p größer als oder gleich 2 ist; t größer als oder gleich 0 ist; und a größer als oder gleich 0 ist;

(B) i eine ganze Zahl von 1 bis 3 ist und j eine ganze Zahl von 1 bis 6 ist;

(C) Q $-OR_2$,

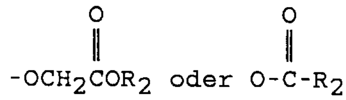

darstellt, worin $R_2$ Wasserstoff, $C_1$-$C_6$-Alkyl oder $C_1$-$C_6$-Alkenyl darstellt;

(D) Y einen zweiwertigen Rest

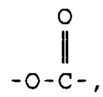

$-OCH_2-$ oder

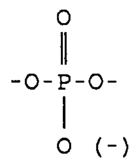

darstellt;

(E) $R_3$ einen oder mehrere $C_1$-$C_5$-Alkyl-, $C_1$-$C_5$-Alkoxy- oder Halogen-Substituenten des aromatischen Rings darstellt;

(F) $-CH_2A$ in der ortho-, meta- oder para-Position sein kann, worin A einen 5-7-gliedrigen aromatischen heterocyclischen Ring darstellt, der mindestens ein Stickstoffatom und gegebenenfalls eines oder mehrere weitere Stickstoff- oder Schwefelatome enthält.

**7.** Verfahren nach Anspruch 1, wobei der monoclonale oder polyclonale Antikörper gerichtet ist gegen Interleukin-1β in Kombination mit einem Antagonisten vom Thrombozyten-Aktivierungsfaktor der Formel

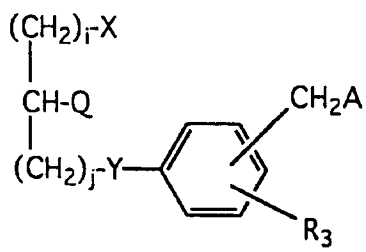

Formel II

worin:

(A) X darstellt

i) $C_1$-$C_{24}$-Alkyl;
ii) $C_1$-$C_{24}$-Alkoxy;
iii) $C_1$-$C_{24}$-Carbamoyloxy;
iv)

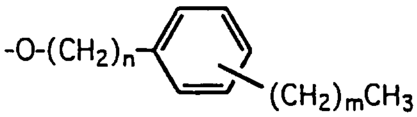 ; 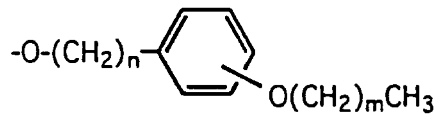 ;

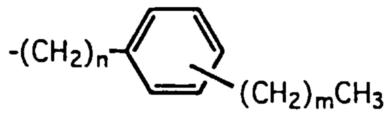 ; 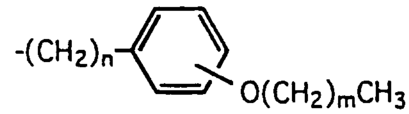 ;

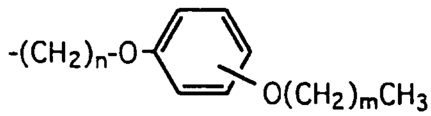 ; 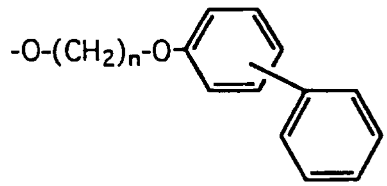

oder 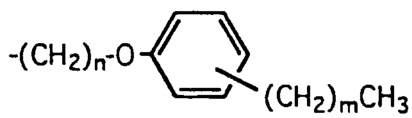 ;

n eine ganze Zahl von 1 bis 25 ist und m eine ganze Zahl von 0 bis 24 ist und die Summe von n und m geringer als oder gleich 25 ist;

v) Phenyl;

vi) mono- oder polysubstituiertes Phenyl, substituiert mit $C_1$-$C_{20}$-Alkyl, $C_1$-$C_{20}$-Alkoxy, Halogen, Trifluormethyl, Phenyl, substituiertem Phenyl oder Benzyloxy;

vii) Phenoxy;

viii) mono- oder polysubstituiertes Phenoxy, substituiert mit $C_1$-$C_{20}$-Alkyl, $C_1$-$C_{20}$-Alkoxy, Halogen, Trifluormethyl, Phenyl, substituiertem Phenyl oder Benzyloxy;

ix) Naphthaloxy;

x) mono- oder polysubstituiertes Naphthaloxy, substituiert mit $C_1$-$C_{20}$-Alkyl, $C_1$-$C_{20}$-Alkoxy oder Halogen;

xi) $-O-(CH_2)_r-O-((CH_2)_pO)_t-(CH_2)_a-W$, worin W Methyl oder Phenyl, gegebenenfalls substituiert mit $C_1$-$C_3$-Alkyl, $C_1$-$C_3$-Alkoxy oder Phenyl, darstellt, r, p, t und a ganze Zahlen sind, so daß der Ausdruck r+(p+1)t+a ebenfalls eine ganze Zahl ergibt und einen Wert von 3 bis 20 aufweist; r größer als oder gleich 2 ist; p größer als oder gleich 2 ist; t größer als oder gleich 0 ist; und a größer als oder gleich 0 ist;

(B) i eine ganze Zahl von 1 bis 3 ist und j eine ganze Zahl von 1 bis 6 ist;

(C) Q $-OR_2$,

$$-OCH_2\overset{\displaystyle O}{\overset{\|}{C}}OR_2 \text{ oder } O-\overset{\displaystyle O}{\overset{\|}{C}}-R_2$$

darstellt, worin $R_2$ Wasserstoff, $C_1$-$C_6$-Alkyl oder $C_1$-$C_6$-Alkenyl darstellt;

(D) Y einen zweiwertigen Rest

$$-O-\overset{\displaystyle O}{\overset{\|}{C}}-,$$

$-OCH_2$ - oder

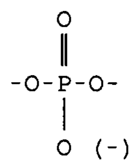

darstellt;

(E) $R_3$ einen oder mehrere $C_1$-$C_5$-Alkyl-, $C_1$-$C_5$-Alkoxy- oder Halogen-Substituenten des aromatischen Rings darstellt;

(F) -$CH_2$A in der ortho-, meta- oder para-Position sein kann, worin A einen 5-7-gliedrigen aromatischen heterocyclischen Ring darstellt, der mindestens ein Stickstoffatom und gegebenenfalls eines oder mehrere weitere Stickstoff- oder Schwefelatome enthält.

**8.** Verfahren nach Anspruch 1, wobei der monoclonale oder polyclonale Antikörper gerichtet ist gegen γ-Interferon in Kombination mit einem Antagonisten vom Thrombozyten-Aktivierungsfaktor der Formel

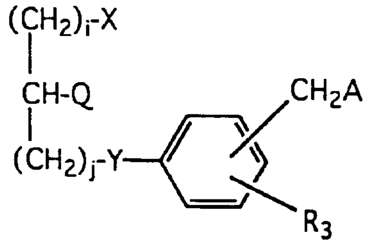

Formel II

worin:

(A) X darstellt

i) $C_1$-$C_{24}$-Alkyl;
ii) $C_1$-$C_{24}$-Alkoxy;
iii) $C_1$-$C_{24}$-Carbamoyloxy;
iv)

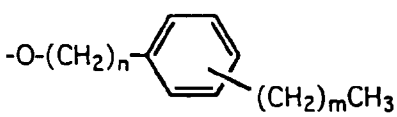 ; 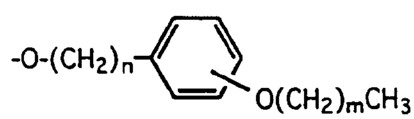 ;

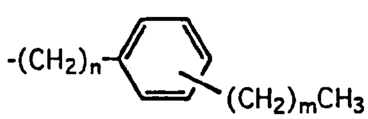 ; 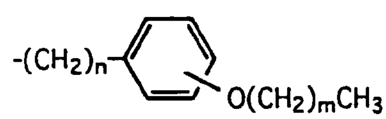 ;

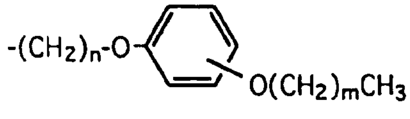 ; 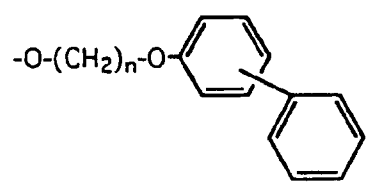

oder 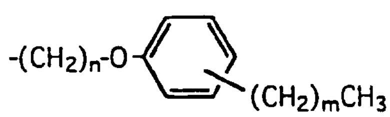 ;

n eine ganze Zahl von 1 bis 25 ist und m eine ganze Zahl von 0 bis 24 ist und die Summe von n und

m geringer als oder gleich 25 ist;

v) Phenyl;

vi) mono- oder polysubstituiertes Phenyl, substituiert mit $C_1$-$C_{20}$-Alkyl, $C_1$-$C_{20}$-Alkoxy, Halogen, Trifluormethyl, Phenyl, substituiertem Phenyl oder Benzyloxy;

vii) Phenoxy;

viii) mono- oder polysubstituiertes Phenoxy, substituiert mit $C_1$-$C_{20}$-Alkyl, $C_1$-$C_{20}$-Alkoxy, Halogen, Trifluormethyl, Phenyl, substituiertem Phenyl oder Benzyloxy;

ix) Naphthaloxy;

x) mono- oder polysubstituiertes Naphthaloxy, substituiert mit $C_1$-$C_{20}$-Alkyl, $C_1$-$C_{20}$-Alkoxy oder Halogen;

xi) $-O-(CH_2)_r-O-((CH_2)_pO)_t-(CH_2)_a-W$, worin W Methyl oder Phenyl, gegebenenfalls substituiert mit $C_1$-$C_3$-Alkyl, $C_1$-$C_3$-Alkoxy oder Phenyl, darstellt, r, p, t und a ganze Zahlen sind, so daß der Ausdruck $r+(p+1)t+a$ ebenfalls eine ganze Zahl ergibt und einen Wert von 3 bis 20 aufweist; r größer als oder gleich 2 ist; p größer als oder gleich 2 ist; t größer als oder gleich 0 ist; und a größer als oder gleich 0 ist;

(B) i eine ganze Zahl von 1 bis 3 ist und j eine ganze Zahl von 1 bis 6 ist;

(C) Q $-OR_2$,

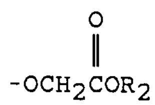
oder
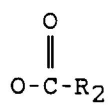

darstellt, worin $R_2$ Wasserstoff, $C_1$-$C_6$-Alkyl oder $C_1$-$C_6$-Alkenyl darstellt;

(D) Y einen zweiwertigen Rest

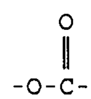,

$-OCH_2-$ oder

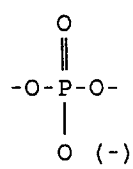

darstellt;

(E) $R_3$ einen oder mehrere $C_1$-$C_5$-Alkyl-, $C_1$-$C_5$-Alkoxy- oder Halogen-Substituenten des aromatischen Rings darstellt;

(F) $-CH_2A$ in der ortho-, meta- oder para-Position sein kann, worin A einen 5-7-gliedrigen aromatischen heterocyclischen Ring darstellt, der mindestens ein Stickstoffatom und gegebenenfalls eines oder mehrere weitere Stickstoff- oder Schwefelatome enthält.

**9.** Verfahren nach Anspruch 1, wobei der monoclonale oder polyclonale Antikörper gerichtet ist gegen bakterielle Zellwand-Lipopolysaccharide in Kombination mit einem Antagonisten vom Thrombozyten-Aktivierungsfaktor der Formel

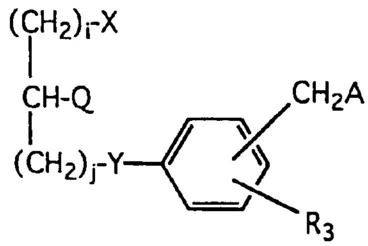

Formel II

worin:

(A) X darstellt

i) $C_1$-$C_{24}$-Alkyl;
ii) $C_1$-$C_{24}$-Alkoxy;
iii) $C_1$-$C_{24}$-Carbamoyloxy; iv)

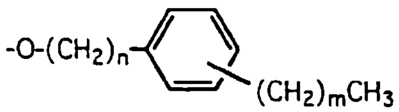 ; 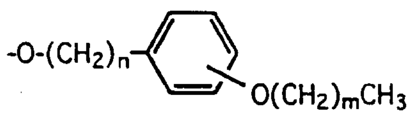 ;

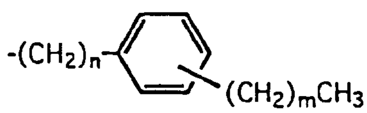 ; 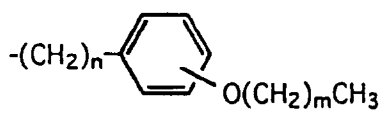 ;

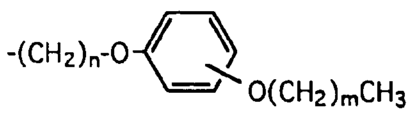 ; 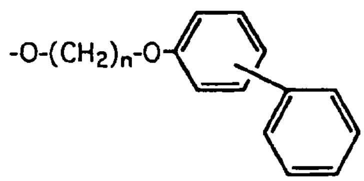

oder 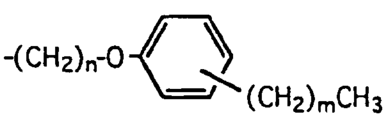 ;

n eine ganze Zahl von 1 bis 25 ist und m eine ganze Zahl von 0 bis 24 ist und die Summe von n und m geringer als oder gleich 25 ist;
v) Phenyl;
vi) mono- oder polysubstituiertes Phenyl, substituiert mit $C_1$-$C_{20}$-Alkyl, $C_1$-$C_{20}$-Alkoxy, Halogen, Trifluormethyl, Phenyl, substituiertem Phenyl oder Benzyloxy;
vii) Phenoxy;
viii) mono- oder polysubstituiertes Phenoxy, substituiert mit $C_1$-$C_{20}$-Alkyl, $C_1$-$C_{20}$-Alkoxy, Halogen, Trifluormethyl, Phenyl, substituiertem Phenyl oder Benzyloxy;
ix) Naphthaloxy;
x) mono- oder polysubstituiertes Naphthaloxy, substituiert mit $C_1$-$C_{20}$-Alkyl, $C_1$-$C_{20}$-Alkoxy oder Halogen;
xi) $-O-(CH_2)_r-O-((CH_2)_pO)_t-(CH_2)_a-W$, worin W Methyl oder Phenyl, gegebenenfalls substituiert mit $C_1$-$C_3$-Alkyl, $C_1$-$C_3$-Alkoxy oder Phenyl, darstellt, r, p, t und a ganze Zahlen sind, so daß der Ausdruck r+(p+1)t+a ebenfalls eine ganze Zahl ergibt und einen Wert von 3 bis 20 aufweist; r größer als oder gleich 2 ist; p größer als oder gleich 2 ist; t größer als oder gleich 0 ist; und a größer als oder gleich 0 ist;

(B) i eine ganze Zahl von 1 bis 3 ist und j eine ganze Zahl von 1 bis 6 ist;
(C) Q $-OR_2$,

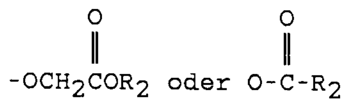

darstellt, worin $R_2$ Wasserstoff, $C_1$-$C_6$-Alkyl oder $C_1$-$C_6$-Alkenyl darstellt;

(D) Y einen zweiwertigen Rest

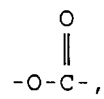

$-OCH_2-$ oder

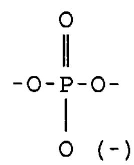

darstellt;

(E) $R_3$ einen oder mehrere $C_1$-$C_5$-Alkyl-, $C_1$-$C_5$-Alkoxy- oder Halogen-Substituenten des aromatischen Rings darstellt;

(F) $-CH_2A$ in der ortho-, meta- oder para-Position sein kann, worin A einen 5-7-gliedrigen aromatischen heterocyclischen Ring darstellt, der mindestens ein Stickstoffatom und gegebenenfalls eines oder mehrere weitere Stickstoff- oder Schwefelatome enthält.

**10.** Verwendung von a) einer therapeutisch wirksamen Menge eines Antagonisten für den Thrombozyten-Aktivierungsfaktor und b) einem oder mehreren monoclonalen oder polyclonalen Antikörpern, gerichtet gegen entweder Tumor-Nekrose-Faktor $\alpha$, Interleukin-1$\beta$, $\gamma$-Interferon oder bakterielle Zellwand-Lipopolysaccharide, bei der Zubereitung eines Arzneimittels zur Behandlung von Endotoxinschock.

**11.** Verwendung nach Anspruch 10, wobei der Antagonist für den Thrombozyten-Aktivierungsfaktor die Formel aufweist:

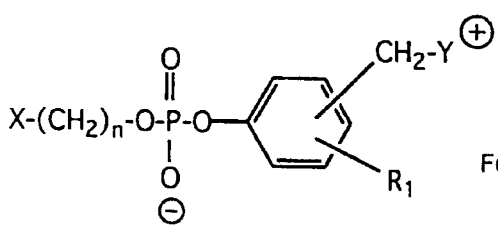

Formel I

worin X einen Phenyl- oder Naphthylring darstellt, gegebenenfalls substituiert in einer Position mit einem oder mehreren Substituenten, von:

i) $-R_2$, worin $R_2$ $C_1$-$C_{25}$-Alkyl, $C_1$-$C_{25}$-Alkenyl, $C_1$-$C_{25}$-Alkoxy, $C_1$-$C_{25}$-Alkenyloxy, $C_1$-$C_{25}$-Thioalkyl, Phenyl, Phenoxy, substituiertes Phenyl oder substituiertes Phenoxy, worin die Substituenten $C_1$-$C_{20}$-Alkyl, $C_1$-$C_{20}$-Alkoxy, Halogen oder Trifluormethyl darstellen, darstellt;

ii) Wasserstoff, Halogen, Trifluormethyl, Cyano oder Nitro;

iii) $-CO_2R_3$, $-CONHR_3$, -CHO, $OCONHR_3$ oder $-NHCOR_3$, worin $R_3$ $C_1$-$C_{25}$-Alkyl, $C_1$-$C_{25}$-Alkenyl, Phenyl oder substituiertes Phenyl, worin die Substituenten $C_1$-$C_{20}$-Alkyl, $C_1$-$C_{20}$-Alkoxy, Halogen oder Trifluormethyl darstellen, darstellt;

$R_1$ einen oder mehrere Substituenten des aromatischen Rings darstellt, die in beliebiger Position vorlie-

gen können und Wasserstoff, $C_1$-$C_5$-Alkyl, $C_1$-$C_5$-Alkoxy oder Halogen darstellen;

$-CH_2$-Y einen einzelnen Substituenten des aromatischen Rings darstellt, der eine beliebige Position besetzen kann, worin Y einen mono- oder bicyclischen aromatischen Heterocyclus darstellt mit 5-7 gliedrigen Ringen, die mindestens ein Stickstoffatom, das an die Methylengruppe gebunden ist und gegebenenfalls eines oder mehrere andere Stickstoff- oder Schwefelatome enthalten und n die ganze Zahl 0 oder 1 ist;

und wobei die monoclonalen oder polyclonalen Antikörper gerichtet sind gegen Tumor-Nekrose-Faktor α.

**12.** Verwendung nach Anspruch 10, worin der Antagonist für den Thrombozyten-Aktivierungsfaktor die Formel aufweist:

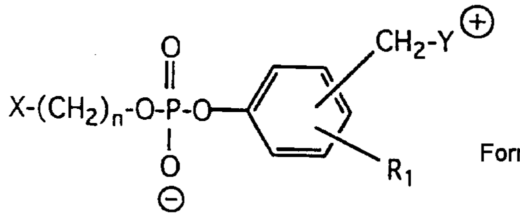

Formel I

worin X einen Phenyl- oder Naphthylring darstellt, gegebenenfalls substituiert in einer Position mit einem oder mehreren Substituenten, von:

i) $-R_2$, worin $R_2$ $C_1$-$C_{25}$-Alkyl, $C_1$-$C_{25}$-Alkenyl, $C_1$-$C_{25}$-Alkoxy, $C_1$-$C_{25}$-Alkenyloxy, $C_1$-$C_{25}$-Thioalkyl, Phenyl, Phenoxy, substituiertes Phenyl oder substituiertes Phenoxy, worin die Substituenten $C_1$-$C_{20}$-Alkyl, $C_1$-$C_{20}$-Alkoxy, Halogen oder Trifluormethyl darstellen, darstellt;
ii) Wasserstoff, Halogen, Trifluormethyl, Cyano oder Nitro;
iii) $-CO_2R_3$, $-CONHR_3$, -CHO, $OCONHR_3$ oder $-NHCOR_3$, worin $R_3$ $C_1$-$C_{25}$-Alkyl, $C_1$-$C_{25}$-Alkenyl, Phenyl oder substituiertes Phenyl, worin die Substituenten $C_1$-$C_{20}$-Alkyl, $C_1$-$C_{20}$-Alkoxy, Halogen oder Trifluormethyl darstellen, darstellt;

$R_1$ einen oder mehrere Substituenten des aromatischen Rings darstellt, die in beliebiger Position vorliegen können und Wasserstoff, $C_1$-$C_5$-Alkyl, $C_1$-$C_5$-Alkoxy oder Halogen darstellen;

$-CH_2$-Y einen einzelnen Substituenten des aromatischen Rings darstellt, der eine beliebige Position besetzen kann, worin Y einen mono- oder bicyclischen aromatischen Heterocyclus darstellt mit 5-7 gliedrigen Ringen, die mindestens ein Stickstoffatom, das an die Methylengruppe gebunden ist und gegebenenfalls eines oder mehrere andere Stickstoff- oder Schwefelatome enthalten und n die ganze Zahl 0 oder 1 ist;

und wobei die monoclonalen oder polyclonalen Antikörper gegen Interleukin-1β gerichtet sind.

**13.** Verwendung nach Anspruch 10, wobei der Antagonist für den Thrombozyten-Aktivierungsfaktor die Formel aufweist:

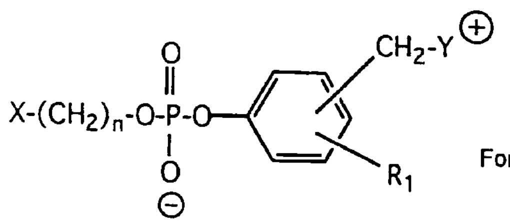

Formel I

worin X einen Phenyl- oder Naphthylring darstellt, gegebenenfalls substituiert in einer Position mit einem oder mehreren Substituenten, von:

i) $-R_2$, worin $R_2$ $C_1$-$C_{25}$-Alkyl, $C_1$-$C_{25}$-Alkenyl, $C_1$-$C_{25}$-Alkoxy, $C_1$-$C_{25}$-Alkenyloxy, $C_1$-$C_{25}$-Thioalkyl, Phenyl, Phenoxy, substituiertes Phenyl oder substituiertes Phenoxy, worin die Substituenten $C_1$-$C_{20}$-Alkyl, $C_1$-$C_{20}$-Alkoxy, Halogen oder Trifluormethyl darstellen, darstellt;
ii) Wasserstoff, Halogen, Trifluormethyl, Cyano oder Nitro;
iii) $-CO_2R_3$, $-CONHR_3$, -CHO, $OCONHR_3$ oder $-NHCOR_3$, worin $R_3$ $C_1$-$C_{25}$-Alkyl, $C_1$-$C_{25}$-Alkenyl, Phenyl oder substituiertes Phenyl, worin die Substituenten $C_1$-$C_{20}$-Alkyl, $C_1$-$C_{20}$-Alkoxy, Halogen oder Trifluormethyl dar-

stellen, darstellt;

$R_1$ einen oder mehrere Substituenten des aromatischen Rings darstellt, die in beliebiger Position vorliegen können und Wasserstoff, $C_1$-$C_5$-Alkyl, $C_1$-$C_5$-Alkoxy oder Halogen darstellen;

-$CH_2$-Y einen einzelnen Substituenten des aromatischen Rings darstellt, der eine beliebige Position besetzen kann, worin Y einen mono- oder bicyclischen aromatischen Heterocyclus darstellt mit 5-7 gliedrigen Ringen, die mindestens ein Stickstoffatom, das an die Methylengruppe gebunden ist und gegebenenfalls eines oder mehrere andere Stickstoff- oder Schwefelatome enthalten und n die ganze Zahl 0 oder 1 ist;

und wobei die monoclonalen oder polyclonalen Antikörper gegen $\gamma$-Interferon gerichtet sind.

**14.** Verwendung nach Anspruch 10, wobei der Antagonist für den Thrombozyten-Aktivierungsfaktor die Formel aufweist:

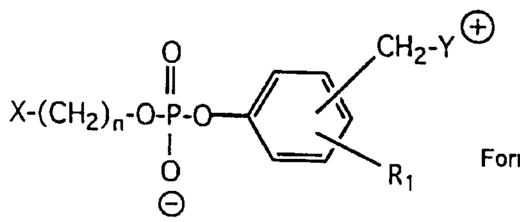

Formel I

worin X einen Phenyl- oder Naphthylring darstellt, gegebenenfalls substituiert in einer Position mit einem oder mehreren Substituenten, von:

i) -$R_2$, worin $R_2$ $C_1$-$C_{25}$-Alkyl, $C_1$-$C_{25}$-Alkenyl, $C_1$-$C_{25}$-Alkoxy, $C_1$-$C_{25}$-Alkenyloxy, $C_1$-$C_{25}$-Thioalkyl, Phenyl, Phenoxy, substituiertes Phenyl oder substituiertes Phenoxy, worin die Substituenten $C_1$-$C_{20}$-Alkyl, $C_1$-$C_{20}$-Alkoxy, Halogen oder Trifluormethyl darstellen, darstellt;

ii) Wasserstoff, Halogen, Trifluormethyl, Cyano oder Nitro;

iii) -$CO_2R_3$, -$CONHR_3$, -CHO, $OCONHR_3$ oder -$NHCOR_3$, worin $R_3$ $C_1$-$C_{25}$-Alkyl, $C_1$-$C_{25}$-Alkenyl, Phenyl oder substituiertes Phenyl, worin die Substituenten $C_1$-$C_{20}$-Alkyl, $C_1$-$C_{20}$-Alkoxy, Halogen oder Trifluormethyl darstellen, darstellt;

$R_1$ einen oder mehrere Substituenten des aromatischen Rings darstellt, die in beliebiger Position vorliegen können und Wasserstoff, $C_1$-$C_5$-Alkyl, $C_1$-$C_5$-Alkoxy oder Halogen darstellen;

-$CH_2$-Y einen einzelnen Substituenten des aromatischen Rings darstellt, der eine beliebige Position besetzen kann, worin Y einen mono- oder bicyclischen aromatischen Heterocyclus darstellt mit 5-7 gliedrigen Ringen, die mindestens ein Stickstoffatom, das an die Methylengruppe gebunden ist und gegebenenfalls eines oder mehrere andere Stickstoff- oder Schwefelatome enthalten und n die ganze Zahl 0 oder 1 ist;

und wobei die monoclonalen oder polyclonalen Antikörper gegen bakterielle Zellwand-Lipopolysaccharide gerichtet sind.

**15.** Verwendung nach Anspruch 10, wobei der Antagonist für den Thrombozyten-Aktivierungsfaktor die Formel aufweist:

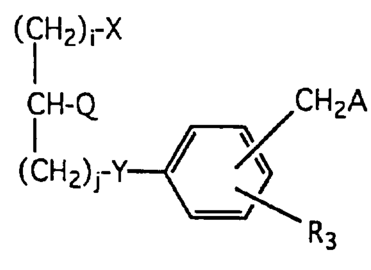

Formel II

worin:

(A) X darstellt

i) $C_1$-$C_{24}$-Alkyl;
ii) $C_1$-$C_{24}$-Alkoxy;
iii) $C_1$-$C_{24}$-Carbamoyloxy;
iv)

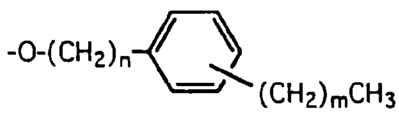 ; 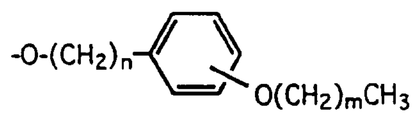 ;

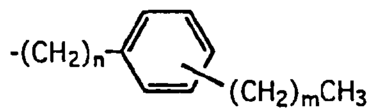 ; 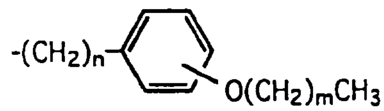 ;

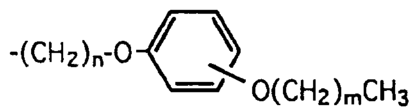 ; 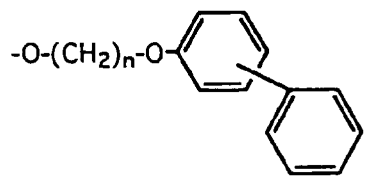

oder 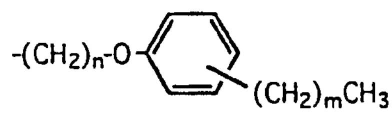 ;

n eine ganze Zahl von 1 bis 25 ist und m eine ganze Zahl von 0 bis 24 ist und die Summe von n und m geringer als oder gleich 25 ist;
v) Phenyl;
vi) mono- oder polysubstituiertes Phenyl, substituiert mit $C_1$-$C_{20}$-Alkyl, $C_1$-$C_{20}$-Alkoxy, Halogen, Trifluormethyl, Phenyl, substituiertem Phenyl oder Benzyloxy;
vii) Phenoxy;
viii) mono- oder polysubstituiertes Phenoxy, substituiert mit $C_1$-$C_{20}$-Alkyl, $C_1$-$C_{20}$-Alkoxy, Halogen, Trifluormethyl, Phenyl, substituiertem Phenyl oder Benzyloxy;
ix) Naphthaloxy;
x) mono- oder polysubstituiertes Naphthaloxy, substituiert mit $C_1$-$C_{20}$-Alkyl, $C_1$-$C_{20}$-Alkoxy oder Halogen;
xi) $-O-(CH_2)_r-O-((CH_2)_pO)_t-(CH_2)_a-W$, worin W Methyl oder Phenyl, gegebenenfalls substituiert mit $C_1$-$C_3$-Alkyl, $C_1$-$C_3$-Alkoxy oder Phenyl, darstellt, r, p, t und a ganze Zahlen sind, so daß der Ausdruck r+(p+1)t+a ebenfalls eine ganze Zahl ergibt und einen Wert von 3 bis 20 aufweist; r größer als oder gleich 2 ist; p größer als oder gleich 2 ist; t größer als oder gleich 0 ist; und a größer als oder gleich 0 ist;

(B) i eine ganze Zahl von 1 bis 3 ist und j eine ganze Zahl von 1 bis 6 ist;
(C) Q $-OR_2$,

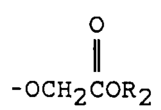 oder 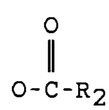

darstellt, worin $R_2$ Wasserstoff, $C_1$-$C_6$-Alkyl oder $C_1$-$C_6$-Alkenyl darstellt;
(D) Y einen zweiwertigen Rest

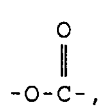

$-OCH_2$- oder

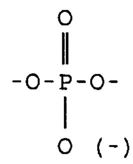

darstellt;

(E) $R_3$ einen oder mehrere $C_1$-$C_5$-Alkyl-, $C_1$-$C_5$-Alkoxy- oder Halogen-Substituenten des aromatischen Rings darstellt;

(F) -$CH_2$A in der ortho-, meta- oder para-Position sein kann, worin A einen 5-7-gliedrigen aromatischen heterocyclischen Ring darstellt, der mindestens ein Stickstoffatom und gegebenenfalls eines oder mehrere weitere Stickstoff- oder Schwefelatome enthält;

und wobei die monoclonalen oder polyclonalen Antikörper gegen Tumor-Nekrose-Faktor $\alpha$ gerichtet sind.

**16.** Verwendung nach Anspruch 10, wobei der Antagonist für den Thrombozyten-Aktivierungsfaktor die Formel aufweist:

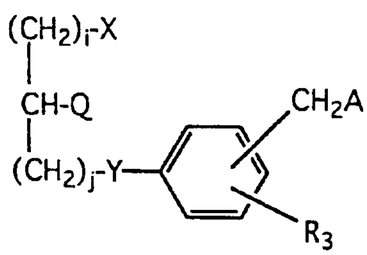

Formel II

worin:

(A) X darstellt

i) $C_1$-$C_{24}$-Alkyl;
ii) $C_1$-$C_{24}$-Alkoxy;
iii) $C_1$-$C_{24}$-Carbamoyloxy;
iv)

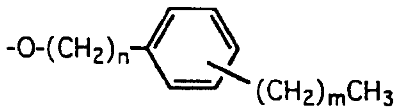 ; 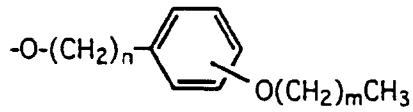 ;

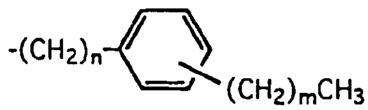 ; 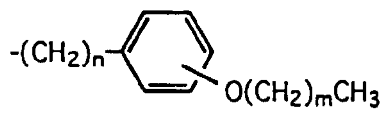 ;

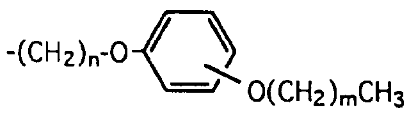 ; 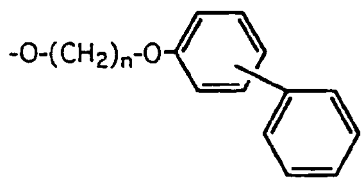

oder 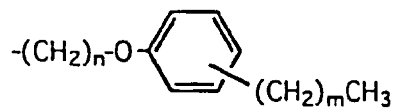 ;

n eine ganze Zahl von 1 bis 25 ist und m eine ganze Zahl von 0 bis 24 ist und die Summe von n und m geringer als oder gleich 25 ist;

v) Phenyl;

vi) mono- oder polysubstituiertes Phenyl, substituiert mit $C_1$-$C_{20}$-Alkyl, $C_1$-$C_{20}$-Alkoxy, Halogen, Trifluormethyl, Phenyl, substituiertem Phenyl oder Benzyloxy;

vii) Phenoxy;

viii) mono- oder polysubstituiertes Phenoxy, substituiert mit $C_1$-$C_{20}$-Alkyl, $C_1$-$C_{20}$-Alkoxy, Halogen, Trifluormethyl, Phenyl, substituiertem Phenyl oder Benzyloxy;

ix) Naphthaloxy;

x) mono- oder polysubstituiertes Naphthaloxy, substituiert mit $C_1$-$C_{20}$-Alkyl, $C_1$-$C_{20}$-Alkoxy oder Halogen;

xi) $-O-(CH_2)_r-O-((CH_2)_pO)_t-(CH_2)_a-W$, worin W Methyl oder Phenyl, gegebenenfalls substituiert mit $C_1$-$C_3$-Alkyl, $C_1$-$C_3$-Alkoxy oder Phenyl, darstellt, r, p, t und a ganze Zahlen sind, so daß der Ausdruck r+(p+1)t+a ebenfalls eine ganze Zahl ergibt und einen Wert von 3 bis 20 aufweist; r größer als oder gleich 2 ist; p größer als oder gleich 2 ist; t größer als oder gleich 0 ist; und a größer als oder gleich 0 ist;

(B) i eine ganze Zahl von 1 bis 3 ist und j eine ganze Zahl von 1 bis 6 ist;

(C) Q $-OR_2$,

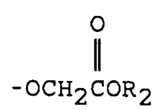 oder 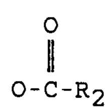

darstellt, worin $R_2$ Wasserstoff, $C_1$-$C_6$-Alkyl oder $C_1$-$C_6$-Alkenyl darstellt;

(D) Y einen zweiwertigen Rest

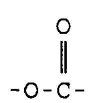 ,

$-OCH_2-$ oder

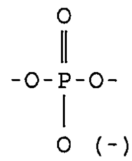

darstellt;

(E) $R_3$ einen oder mehrere $C_1$-$C_5$-Alkyl-, $C_1$-$C_5$-Alkoxy- oder Halogen-Substituenten des aromatischen Rings darstellt;

(F) -$CH_2$A in der ortho-, meta- oder para-Position sein kann, worin A einen 5-7-gliedrigen aromatischen heterocyclischen Ring darstellt, der mindestens ein Stickstoffatom und gegebenenfalls eines oder mehrere weitere Stickstoff- oder Schwefelatome enthält;

und wobei die monoclonalen oder polyclonalen Antikörper gegen Interleukin-1β gerichtet sind.

**17.** Verwendung nach Anspruch 10, wobei der Antagonist für den Thrombozyten-Aktivierungsfaktor die Formel aufweist:

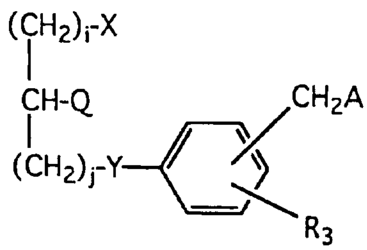

Formel II

worin:

(A) X darstellt

i) $C_1$-$C_{24}$-Alkyl;
ii) $C_1$-$C_{24}$-Alkoxy;
iii) $C_1$-$C_{24}$-Carbamoyloxy;
iv)

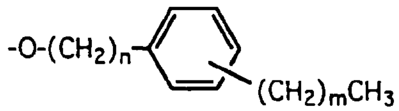 ; 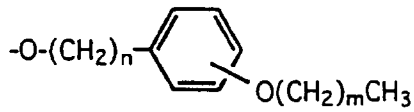 ;

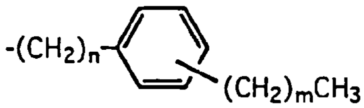 ; 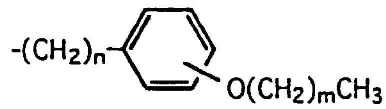 ;

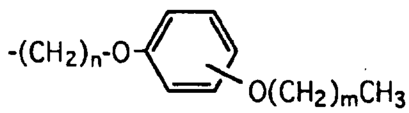 ; 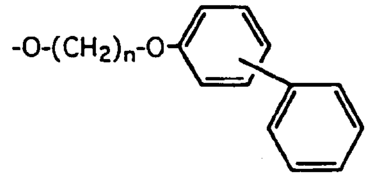

oder 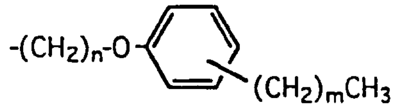 ;

n eine ganze Zahl von 1 bis 25 ist und m eine ganze Zahl von 0 bis 24 ist und die Summe von n und m geringer als oder gleich 25 ist;

v) Phenyl;

vi) mono- oder polysubstituiertes Phenyl, substituiert mit $C_1$-$C_{20}$-Alkyl, $C_1$-$C_{20}$-Alkoxy, Halogen, Trifluormethyl, Phenyl, substituiertem Phenyl oder Benzyloxy;

vii) Phenoxy;

viii) mono- oder polysubstituiertes Phenoxy, substituiert mit $C_1$-$C_{20}$-Alkyl, $C_1$-$C_{20}$-Alkoxy, Halogen, Trifluormethyl, Phenyl, substituiertem Phenyl oder Benzyloxy;

ix) Naphthaloxy;

x) mono- oder polysubstituiertes Naphthaloxy, substituiert mit $C_1$-$C_{20}$-Alkyl, $C_1$-$C_{20}$-Alkoxy oder Halogen;

xi) -O-$(CH_2)_r$-O-$((CH_2)_pO)_t$-$(CH_2)_a$-W, worin W Methyl oder Phenyl, gegebenenfalls substituiert mit $C_1$-$C_3$-Alkyl, $C_1$-$C_3$-Alkoxy oder Phenyl, darstellt, r, p, t und a ganze Zahlen sind, so daß der Ausdruck r+(p+1)t+a ebenfalls eine ganze Zahl ergibt und einen Wert von 3 bis 20 aufweist; r größer als oder gleich 2 ist; p größer als oder gleich 2 ist; t größer als oder gleich 0 ist; und a größer als oder gleich 0 ist;

(B) i eine ganze Zahl von 1 bis 3 ist und j eine ganze Zahl von 1 bis 6 ist;

(C) Q $-OR_2$,

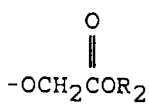 oder 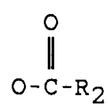

darstellt, worin $R_2$ Wasserstoff, $C_1$-$C_6$-Alkyl oder $C_1$-$C_6$-Alkenyl darstellt;

(D) Y einen zweiwertigen Rest

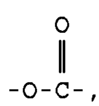

$-OCH_2-$ oder

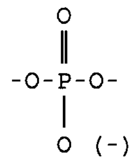

darstellt;

(E) $R_3$ einen oder mehrere $C_1$-$C_5$-Alkyl-, $C_1$-$C_5$-Alkoxy- oder Halogen-Substituenten des aromatischen Rings darstellt;

(F) -$CH_2$A in der ortho-, meta- oder para-Position sein kann, worin A einen 5-7-gliedrigen aromatischen heterocyclischen Ring darstellt, der mindestens ein Stickstoffatom und gegebenenfalls eines oder mehrere weitere Stickstoff- oder Schwefelatome enthält;

und wobei die monoclonalen oder polyclonalen Antikörper gegen γ-Interferon gerichtet sind.

**18.** Verwendung nach Anspruch 10, wobei der Antagonist für den Thrombozyten-Aktivierungsfaktor die Formel aufweist:

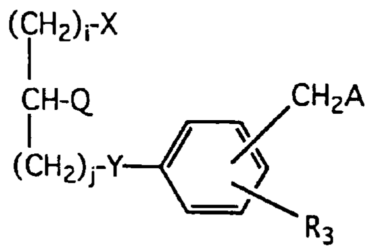

Formel II

worin:

(A) X darstellt

i) $C_1$-$C_{24}$-Alkyl;
ii) $C_1$-$C_{24}$-Alkoxy;
iii) $C_1$-$C_{24}$-Carbamoyloxy;
iv)

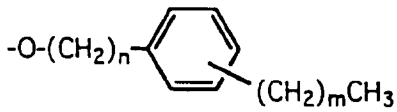 ; 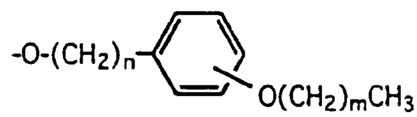 ;

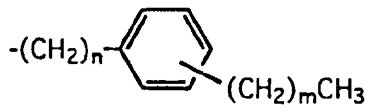 ; 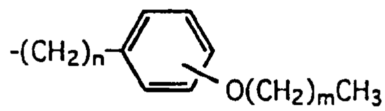 ;

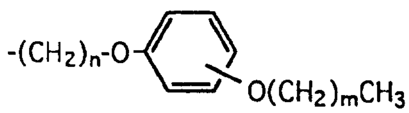 ; 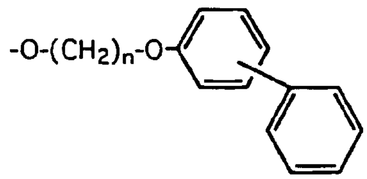

oder 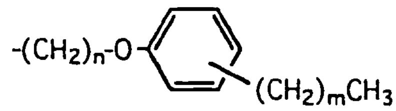 ;

n eine ganze Zahl von 1 bis 25 ist und m eine ganze Zahl von 0 bis 24 ist und die Summe von n und m geringer als oder gleich 25 ist;

v) Phenyl;

vi) mono- oder polysubstituiertes Phenyl, substituiert mit $C_1$-$C_{20}$-Alkyl, $C_1$-$C_{20}$-Alkoxy, Halogen, Trifluormethyl, Phenyl, substituiertem Phenyl oder Benzyloxy;

vii) Phenoxy;

viii) mono- oder polysubstituiertes Phenoxy, substituiert mit $C_1$-$C_{20}$-Alkyl, $C_1$-$C_{20}$-Alkoxy, Halogen, Trifluormethyl, Phenyl, substituiertem Phenyl oder Benzyloxy;

ix) Naphthaloxy;

x) mono- oder polysubstituiertes Naphthaloxy, substituiert mit $C_1$-$C_{20}$-Alkyl, $C_1$-$C_{20}$-Alkoxy oder Halogen;

xi) $-O-(CH_2)_r-O-((CH_2)_pO)_t-(CH_2)_a-W$, worin W Methyl oder Phenyl, gegebenenfalls substituiert mit $C_1$-$C_3$-Alkyl, $C_1$-$C_3$-Alkoxy oder Phenyl, darstellt, r, p, t und a ganze Zahlen sind, so daß der Ausdruck r+(p+1)t+a ebenfalls eine ganze Zahl ergibt und einen Wert von 3 bis 20 aufweist; r größer als oder gleich 2 ist; p größer als oder gleich 2 ist; t größer als oder gleich 0 ist; und a größer als oder gleich 0 ist;

(B) i eine ganze Zahl von 1 bis 3 ist und j eine ganze Zahl von 1 bis 6 ist;

(C) Q $-OR_2$,

$$-OCH_2\overset{O}{\overset{\|}{C}}OR_2 \quad \text{oder} \quad O-\overset{O}{\overset{\|}{C}}-R_2$$

darstellt, worin $R_2$ Wasserstoff, $C_1$-$C_6$-Alkyl oder $C_1$-$C_6$-Alkenyl darstellt;

(D) Y einen zweiwertigen Rest

$$-O-\overset{O}{\overset{\|}{C}}-,$$

$-OCH_2-$ oder

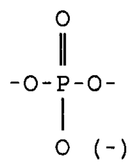

darstellt;

(E) $R_3$ einen oder mehrere $C_1$-$C_5$-Alkyl-, $C_1$-$C_5$-Alkoxy- oder Halogen-Substituenten des aromatischen Rings darstellt;

(F) $-CH_2A$ in der ortho-, meta- oder para-Position sein kann, worin A einen 5-7-gliedrigen aromatischen heterocyclischen Ring darstellt, der mindestens ein Stickstoffatom und gegebenenfalls eines oder mehrere weitere Stickstoff- oder Schwefelatome enthält;

und wobei die monoclonalen oder polyclonalen Antikörper gegen bakterielle Zellwand-Lipopolysaccharide gerichtet sind.

## Revendications

### Revendications pour les Etats contractants suivants : AT, BE, CH, DE, FR, GB, IT, LI, NL, SE

1. Produits contenant, en quantités efficaces du point de vue thérapeutique, un antagoniste du facteur d'activation des plaquettes et un ou plusieurs anticorps monoclonaux ou polyclonaux contre le facteur de nécrose tumorale $\alpha$, l'interleukine-1$\beta$, l'interféron gamma, ou les lipopolysaccharides de paroi cellulaire bactérienne sous forme de préparation combinée pour une utilisation simultanée, séparée ou séquentielle dans le traitement du choc endotoxique.

2. Produit selon la revendication 1, dans lequel un anticorps monoclonal contre le facteur de nécrose tumorale $\alpha$ est associé à un antagoniste du facteur d'activation des plaquettes de formule

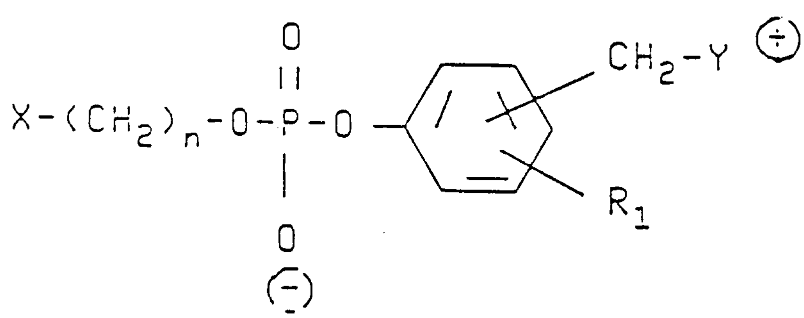

Formule I

dans lequel :

X est un cycle phényle ou naphtyle éventuellement substitué en une position quelconque avec un ou plusieurs des substituants de :

i) $-R_2$ dans lequel $R_2$ représente un groupe alkyle en $C_1$-$C_{25}$, un groupe alkényle en $C_1$-$C_{25}$, un groupe alcoxy en $C_1$-$C_{25}$, un groupe alkényloxy en $C_1$-$C_{25}$, un groupe thioalkyle en $C_1$-$C_{25}$, un groupe phényle, un groupe phénoxy, un groupe phényle substitué ou un groupe phénoxy substitué dans lesquels les substituants sont un groupe alkyle en $C_1$-$C_{20}$, un groupe alcoxy en $C_1$-$C_{20}$, un atome d'halogène ou un groupe trifluorométhyle ;

ii) un atome d'hydrogène, un atome d'halogène, un groupe trifluorométhyle, un groupe cyano ou un groupe nitro ;

iii) $-CO_2R_3$, $-CONHR_3$, -CHO, $OCONHR_3$, ou $-NHCOR_3$ dans lesquels $R_3$ représente un groupe alkyle en

$C_1$-$C_{25}$, un groupe alkényle en $C_1$-$C_{25}$, un groupe phényle ou un groupe phényle substitué dans lequel les substituants sont un groupe alkyle en $C_1$-$C_{25}$, un groupe alcoxy en $C_1$-$C_{20}$, un atome d'halogène ou un groupe trifluorométhyle ;

$R_1$ représente un ou plusieurs substituants du cycle aromatique qui peuvent se trouver en une position quelconque et sont un atome d'hydrogène, un groupe alkyle en $C_1$-$C_5$, un groupe alcoxy en $C_1$-$C_5$, ou un atome d'halogène ;
$-CH_2$-Y est un substituant unique du cycle aromatique qui peut occuper toute position quelconque, dans lequel Y est un hétérocycle aromatique mono- ou bicyclique comprenant des cycles de 5 à 7 éléments contenant au moins un atome d'azote qui est lié au groupe méthylène et éventuellement un ou plusieurs atomes d'azote ou de soufre ; et n représente un nombre entier de valeur 0 ou 1.

**3.** Produits selon la revendication 1, dans lesquels un anticorps monoclonal contre l'interleukine-1β est associé à un antagoniste du facteur d'activation des plaquettes selon la revendication 2.

**4.** Produits selon la revendication 1, dans lesquels un anticorps monoclonal contre l'interféron gamma est associé à un antagoniste du facteur d'activation des plaquettes selon la revendication 2.

**5.** Produits selon la revendication 1, dans lesquels un anticorps monoclonal contre des lipopolysaccharides de paroi cellulaire bactérienne est associé à un antagoniste du facteur d'activation des plaquettes selon la revendication 2.

**6.** Produits selon la revendication 1, dans lesquels un anticorps monoclonal contre le facteur de nécrose tumorale α est associé à un antagoniste du facteur d'activation des plaquettes de formule :

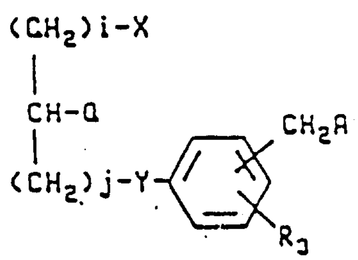

Formule II

dans laquelle :

(A) X est choisi dans le groupe constitué de

(i) groupe alkyle en $C_1$-$C_{24}$ ;
(ii) groupe alcoxy en $C_1$-$C_{24}$ ;
(iii) groupe carboamoyloxy en $C_1$-$C_{24}$ ;
iv)

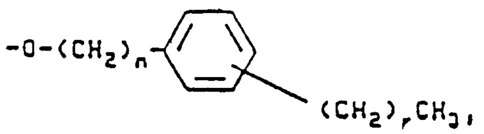 ,
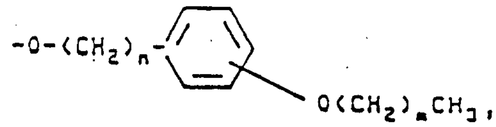 ,

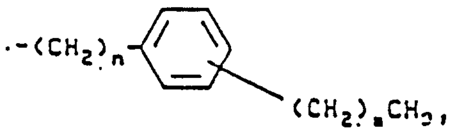 ,
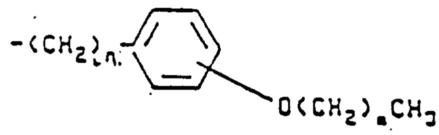

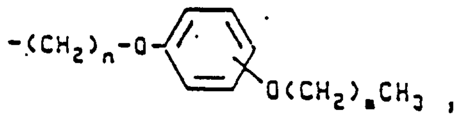 ,
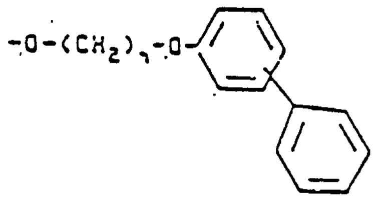

ou 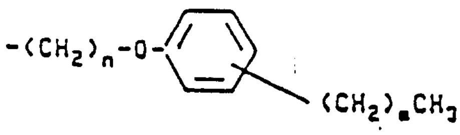

dans lesquelles n est un nombre entier compris entre 1 et 25 et m est un nombre entier compris entre 0 et 24 et la somme de n et de m est inférieure ou égale à 25 ;

(v) groupe phényle ;

(vi) groupe phényle mono- ou polysubstitué substitué par un groupe alkyle en $C_1$-$C_{20}$, un groupe alcoxy en $C_1$-$C_{20}$, un atome d'halogène, un groupe trifluorométhyle, un groupe phényle, un groupe phényle substitué et un groupe benzyloxy ;

(vii) groupe phénoxy ;

(viii) groupe phénoxy mono- ou polysubstitué susbstitué, par un groupe alkyle en $C_1$-$C_{20}$, un groupe alcoxy en $C_1$-$C_{20}$, un atome d'halogène, un groupe trifluorométhyle, un groupe phényle, un groupe phényle substitué et un groupe benzyloxy ;

(ix) groupe naphtaloxy ;

(x) groupe naphtaloxy mono- ou polysubstitué substitué par un groupe alkyle en $C_1$-$C_{20}$, un groupe alcoxy en $C_1$-$C_{20}$ et un atome d'halogène ;

(xi) $-O-(CH_2)_r-O-((CH_2)_pO)_t-(CH_2)_a-W$ dans lequel W représente un groupe méthyle ou phényle éventuellement substitués par un groupe alkyle en $C_1$-$C_3$ un groupe alcoxy en $C_1$-$C_3$ ou un groupe phényle, r, p, t et a représentent des nombres entiers tels que l'expression r+(p+1)t+a donne également un nombre entier dont la valeur est comprise entre 3 et 20 ; r est supérieur ou égal à 2 ; p est supérieur ou égal à 2 ; t représente supérieur ou égal à zéro ; et a est supérieur ou égal à zéro ;

(B) i est un nombre entier compris entre 1 et 3 et j représente un nombre entier compris entre 1 et 6 ;

(C) Q représente $-OR_2$,

$$-OCH_2\overset{\overset{O}{\|}}{C}OR_2,$$

et

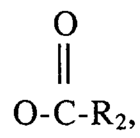

dans lesquels $R_2$ est un atome d'hydrogène, un groupe alkyle en $C_1$-$C_6$ ou un groupe alkényle en $C_1$-$C_6$ ;
(D) Y représente un radical divalent

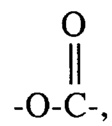

$-OCH_2-$, et

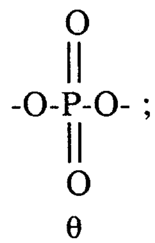

(E) $R_3$ représente un ou plusieurs goupes alkyle en $C_1$-$C_5$, groupes alcoxy en $C_1$-$C_5$ ou des substituants halogènes du cycle aromatique ;
(F) $-CH_2A$ peut être en position ortho, meta ou para, où A est un noyau hétérocyclique aromatique à 5 à 7 éléments contenant au moins un atome d'azote et éventuellement un ou plusieurs autres atomes d'azote ou de soufre.

**7.** Produits selon la revendication 1, dans lesquels un anticorps monoclonal contre l'interleukine-1β est associé à un antagoniste du facteur d'activation des plaquettes selon la revendication 6.

**8.** Produits selon la revendication 1, dans lesquels un anticorps monoclonal contre l'interféron gamma est associé à un antagoniste du facteur d'activation des plaquettes selon la revendication 6.

**9.** Produits selon la revendication 1, dans lesquels un anticorps monoclonal contre les lipopolysaccharides de paroi cellulaire bactérienne est associé à un antagoniste du facteur d'activation des plaquettes selon la revendication 6.

**Revendications pour les Etats contractants suivants : ES, GR**

**1.** Procédé de préparation d'un produit destiné à être utilisé dans le traitement du choc endotoxique chez un mammifère qui comprend la combinaison d'une quantité efficace du point de vue thérapeutique d'un antagoniste du facteur d'activation des plaquettes et d'un ou plusieurs anticorps monoclonaux ou polyclonaux dirigés contre le facteur de nécrose tumorale $\alpha$, et l'interleukine-1$\beta$, l'interféron $\gamma$ ou les lipopolysaccharides de paroi cellulaire bactérienne pour obtenir une préparation pour une utilisation simultanée, séparée ou séquentielle.

**2.** Procédé selon la revendication 1, dans lequel l'anticorps monoclonal ou polyclonal dirigé contre le facteur de nécrose tumorale $\alpha$ est en combinaison avec un antagoniste du facteur d'activation des plaquettes de formule

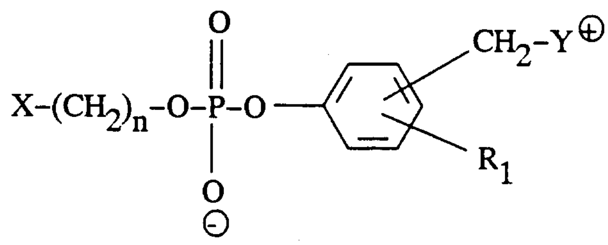

Formule I

dans lequel

X est un cycle phényle ou naphtyle éventuellement substitué en une position quelconque par un ou plusieurs des substituants :

i) -$R_2$ dans lequel $R_2$ représente un groupe alkyle en $C_1$-$C_{25}$, un groupe alcényle en $C_1$-$C_{25}$, un groupe alcoxy en $C_1$-$C_{25}$, un groupe alcényloxy en $C_1$-$C_{25}$, un groupe thioalkyle en $C_1$-$C_{25}$, un groupe phényle, un groupe phénoxy, un groupe phényle substitué ou un groupe phénoxy substitué dans lesquels les substituants sont un groupe alkyle en $C_1$-$C_{20}$, un groupe alcoxy en $C_1$-$C_{20}$, un atome d'halogène ou un groupe trifluorométhyle ;
ii) un atome d'hydrogène, un atome d'halogène, un groupe trifluorométhyle, un groupe cyano ou un groupe nitro ;
iii) -$CO_2R_3$, -$CONHR_3$, -CHO, $OCONHR_3$, ou -$NHCOR_3$ dans lesquels $R_3$ représente un groupe alkyle en $C_1$-$C_{25}$, un groupe alcényle en $C_1$-$C_{25}$, un groupe phényle ou un groupe phényle substitué dans lequel les substituants sont un groupe alkyle en $C_1$-$C_{20}$, un groupe alcoxy en $C_1$-$C_{20}$, un atome d'halogène ou un groupe trifluorométhyle ;

$R_1$ représente un ou plusieurs substituants du cycle aromatique qui peuvent se trouver en une position quelconque et sont un atome d'hydrogène, un groupe alkyle en $C_1$-$C_5$, un groupe alcoxy en $C_1$-$C_5$, ou un atome d'halogène ;
-$CH_2$-Y est un substituant unique du cycle aromatique qui peut occuper une position quelconque, dans lequel Y est un hétérocycle aromatique mono- ou bicyclique comprenant des cycles de 5 à 7 éléments contenant au moins un atome d'azote qui est lié au groupe méthylène et éventuellement un ou plusieurs autres atomes d'azote ou de soufre ; et n représente un nombre entier de valeur 0 ou 1.

3. Procédé selon la revendication 1, dans lequel l'anticorps monoclonal ou polyclonal dirigé contre l'interleukine-1β est en combinaison avec un antagoniste du facteur d'activation des plaquettes de formule

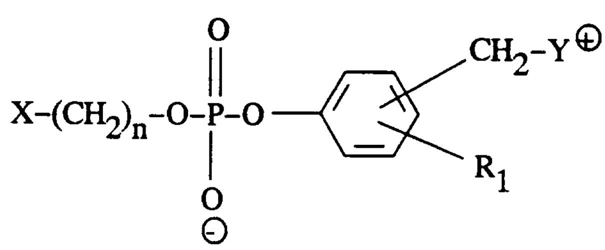

Formule I

dans lequel

X est un cycle phényle ou naphtyle éventuellement substitué en une position quelconque par un ou plusieurs des substituants :

i) $-R_2$ dans lequel $R_2$ représente un groupe alkyle en $C_1$-$C_{25}$, un groupe alcényle en $C_1$-$C_{25}$, un groupe alcoxy en $C_1$-$C_{25}$, un groupe alcényloxy en $C_1$-$C_{25}$, un groupe thioalkyle en $C_1$-$C_{25}$, un groupe phényle, un groupe phénoxy, un groupe phényle substitué ou un groupe phénoxy substitué dans lesquels les substituants sont un groupe alkyle en $C_1$-$C_{20}$, un groupe alcoxy en $C_1$-$C_{20}$, un atome d'halogène ou un groupe trifluorométhyle ;
ii) un atome d'hydrogène, un atome d'halogène, un groupe trifluorométhyle, un groupe cyano ou un groupe nitro ;
iii) $-CO_2R_3$, $-CONHR_3$, -CHO, $OCONHR_3$, ou $-NHCOR_3$ dans lesquels $R_3$ représente un groupe alkyle en $C_1$-$C_{25}$, un groupe alcényle en $C_1$-$C_{25}$, un groupe phényle ou un groupe phényle substitué dans lequel les substituants sont un groupe alkyle en $C_1$-$C_{20}$, un groupe alcoxy en $C_1$-$C_{20}$, un atome d'halogène ou un groupe trifluorométhyle ;

$R_1$ représente un ou plusieurs substituants du cycle aromatique qui peuvent se trouver en une position quelconque et sont un atome d'hydrogène, un groupe alkyle en $C_1$-$C_5$, un groupe alcoxy en $C_1$-$C_5$, ou un atome d'halogène ;
$-CH_2$-Y est un substituant unique du cycle aromatique qui peut occuper une position quelconque, dans lequel Y est un hétérocycle aromatique mono- ou bicyclique comprenant des cycles de 5 à 7 éléments contenant au moins un atome d'azote qui est lié au groupe méthylène et éventuellement un ou plusieurs autres atomes d'azote ou de soufre ; et n représente un nombre entier de valeur 0 ou 1.

**4.** Procédé selon la revendication 1, dans lequel l'anticorps monoclonal ou polyclonal dirigé contre l'interféron $\gamma$ est en combinaison avec un antagoniste du facteur d'activation des plaquettes de formule

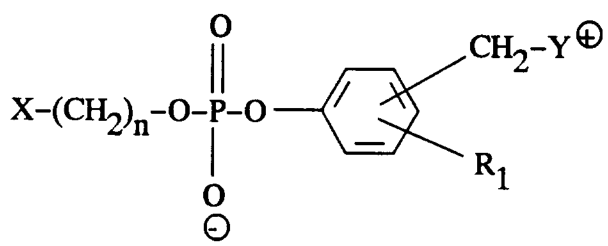

Formule I

dans lequel

X est un cycle phényle ou naphtyle éventuellement substitué en une position quelconque par un ou plusieurs des substituants :

i) $-R_2$ dans lequel $R_2$ représente un groupe alkyle en $C_1$-$C_{25}$, un groupe alcényle en $C_1$-$C_{25}$, un groupe alcoxy en $C_1$-$C_{25}$, un groupe alcényloxy en $C_1$-$C_{25}$, un groupe thioalkyle en $C_1$-$C_{25}$, un groupe phényle, un groupe phénoxy, un groupe phényle substitué ou un groupe phénoxy substitué dans lesquels les substituants sont un groupe alkyle en $C_1$-$C_{20}$, un groupe alcoxy en $C_1$-$C_{20}$, un atome d'halogène ou un groupe trifluorométhyle ;
ii) un atome d'hydrogène, un atome d'halogène, un groupe trifluorométhyle, un groupe cyano ou un groupe nitro ;
iii) $-CO_2R_3$, $-CONHR_3$, -CHO, $OCONHR_3$, ou $-NHCOR_3$ dans lesquels $R_3$ représente un groupe alkyle en $C_1$-$C_{25}$, un groupe alcényle en $C_1$-$C_{25}$, un groupe phényle ou un groupe phényle substitué dans lequel les substituants sont un groupe alkyle en $C_1$-$C_{20}$, un groupe alcoxy en $C_1$-$C_{20}$, un atome d'halogène ou un groupe trifluorométhyle ;

$R_1$ représente un ou plusieurs substituants du cycle aromatique qui peuvent se trouver en une position quelconque et sont un atome d'hydrogène, un groupe alkyle en $C_1$-$C_5$, un groupe alcoxy en $C_1$-$C_5$, ou un atome

d'halogène ;

$-CH_2-Y$ est un substituant unique du cycle aromatique qui peut occuper une position quelconque, dans lequel Y est un hétérocycle aromatique mono- ou bicyclique comprenant des cycles de 5 à 7 éléments contenant au moins un atome d'azote qui est lié au groupe méthylène et éventuellement un ou plusieurs autres atomes d'azote ou de soufre; et n représente un nombre entier de valeur 0 ou 1.

5. Procédé selon la revendication 1, dans lequel l'anticorps monoclonal ou polyclonal dirigé contre les lipopolysaccharides de paroi cellulaire bactérienne est en combinaison avec un antagoniste du facteur d'activation des plaquettes de formule

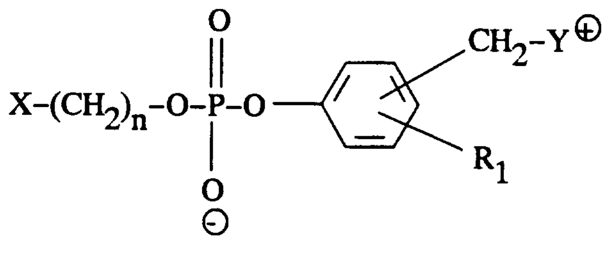

Formule I

dans lequel

X est un cycle phényle ou naphtyle éventuellement substitué en une position quelconque par un ou plusieurs des substituants :

i) $-R_2$ dans lequel $R_2$ représente un groupe alkyle en $C_1$-$C_{25}$, un groupe alcényle en $C_1$-$C_{25}$, un groupe alcoxy en $C_1$-$C_{25}$, un groupe alcényloxy en $C_1$-$C_{25}$, un groupe thioalkyle en $C_1$-$C_{25}$, un groupe phényle, un groupe phénoxy, un groupe phényle substitué ou un groupe phénoxy substitué dans lesquels les substituants sont un groupe alkyle en $C_1$-$C_{20}$, un groupe alcoxy en $C_1$-$C_{20}$, un atome d'halogène ou un groupe trifluorométhyle ;
ii) un atome d'hydrogène, un atome d'halogène, un groupe trifluorométhyle, un groupe cyano ou un groupe nitro ;
iii) $-CO_2R_3$, $-CONHR_3$, -CHO, $OCONHR_3$, ou $-NHCOR_3$ dans lesquels $R_3$ représente un groupe alkyle en $C_1$-$C_{25}$, un groupe alcényle en $C_1$-$C_{25}$, un groupe phényle ou un groupe phényle substitué dans lequel les substituants sont un groupe alkyle en $C_1$-$C_{20}$, un groupe alcoxy en $C_1$-$C_{20}$, un atome d'halogène ou un groupe trifluorométhyle ;

$R_1$ représente un ou plusieurs substituants du cycle aromatique qui peuvent se trouver en une position quelconque et sont un atome d'hydrogène, un groupe alkyle en $C_1$-$C_5$, un groupe alcoxy en $C_1$-$C_5$, ou un atome d'halogène ;
$-CH_2-Y$ est un substituant unique du cycle aromatique qui peut occuper une position quelconque, dans lequel Y est un hétérocycle aromatique mono- ou bicyclique comprenant des cycles de 5 à 7 éléments contenant au moins un atome d'azote qui est lié au groupe méthylène et éventuellement un ou plusieurs autres atomes d'azote ou de soufre ; et n représente un nombre entier de valeur 0 ou 1.

6. Procédé selon la revendication 1, dans lequel l'anticorps monoclonal ou polyclonal dirigé contre le facteur de nécrose tumorale α est en combinaison avec un antagoniste du facteur d'activation des plaquettes de formule :

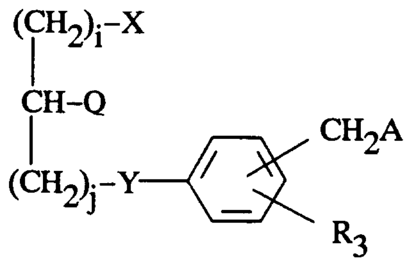

Formule II

dans laquelle

(A) X est

(i) alkyle en $C_1$-$C_{24}$ ;
(ii) alcoxy en $C_1$-$C_{24}$ ;
(iii) carbamoyloxy en $C_1$-$C_{24}$ ;
(iv)

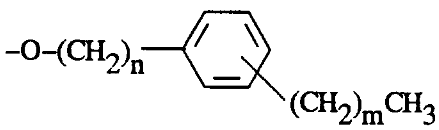 ; 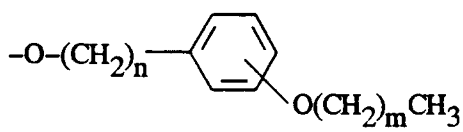 ;

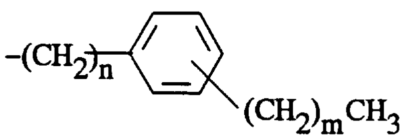 ; 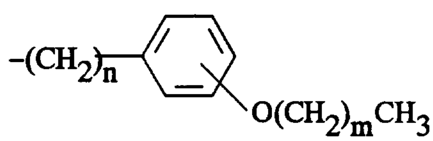 ;

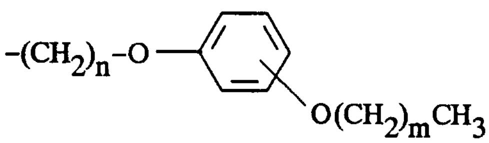 ; 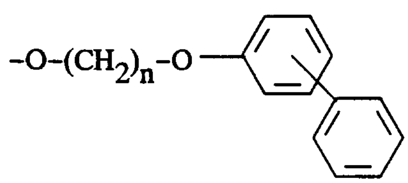 ;

ou

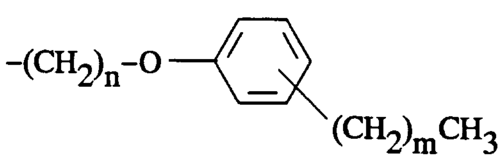 ;

où n est un nombre entier compris entre 1 et 25 et m est un nombre entier compris entre 0 et 24 et la somme de n et de m est inférieure ou égale à 25 ;
(v) phényle ;
(vi) phényle mono- ou polysubstitué par un groupe alkyle en $C_1$-$C_{20}$, un groupe alcoxy en $C_1$-$C_{20}$, un atome d'halogène, un groupe trifluorométhyle, un groupe phényle, un groupe phényle substitué ou un groupe benzyloxy ;
(vii) phénoxy ;
(viii) phénoxy mono- ou polysubstitué substitué par un groupe alkyle en $C_1$-$C_{20}$, un groupe alcoxy en $C_1$-$C_{20}$, un atome d'halogène, un groupe trifluorométhyle, un groupe phényle, un groupe phényle substitué ou

un groupe benzyloxy ;

(ix) naphtyloxy ;

(x) naphtyloxy mono- ou polysubstitué substitué par un groupe alkyle en $C_1$-$C_{20}$, un groupe alcoxy en $C_1$-$C_{20}$ ou un atome d'halogène ;

(xi) $-O-(CH_2)_r-O-((CH_2)_pO)_t-(CH_2)_a-W$ où W représente un groupe méthyle ou phényle éventuellement substitué par un groupe alkyle en $C_1$-$C_3$, un groupe alcoxy en $C_1$-$C_3$ ou un groupe phényle, r, p, t et a représentent des nombres entiers tels que l'expression r+(p+1)t+a donne également un nombre entier dont la valeur est comprise entre 3 et 20 ; r est supérieur ou égal à 2 ; p est supérieur ou égal à 2 ; t est supérieur ou égal à zéro ; et a est supérieur ou égal à zéro ;

(B) i est un nombre entier compris entre 1 et 3 et j est un nombre entier compris entre 1 et 6 ;

(C) Q représente $-OR_2$,

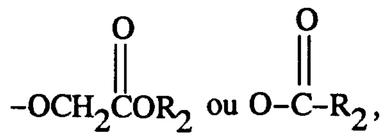

où $R_2$ est un atome d'hydrogène, un groupe alkyle en $C_1$-$C_6$ ou un groupe alcényle en $C_1$-$C_6$ ;

(D) Y représente un radical divalent

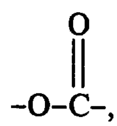

$-OCH_2-$ ou

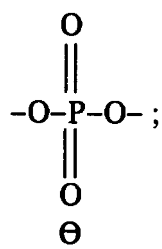

(E) $R_3$ représente un ou plusieurs substituants alkyle en $C_1$-$C_5$, alcoxy en $C_1$-$C_5$ ou halogènes du cycle aromatique ;

(F) $-CH_2A$ peut être en position <u>ortho</u> <u>méta</u> ou <u>para</u>, où A est un noyau hétérocyclique aromatique à 5 à 7 éléments contenant au moins un atome d'azote et éventuellement un ou plusieurs autres atomes d'azote ou de soufre.

**7.** Procédé selon la revendication 1, dans lequel l'anticorps monoclonal ou polyclonal dirigé contre l'interleukine-1β est en combinaison avec un antagoniste du facteur d'activation des plaquettes de formule :

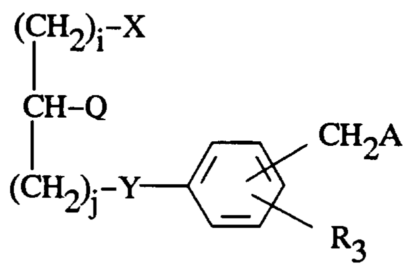

Formule II

dans laquelle

(A) X est

(i) alkyle en $C_1$-$C_{24}$ ;
(ii) alcoxy en $C_1$-$C_{24}$ ;
(iii) carbamoyloxy en $C_1$-$C_{24}$ ;
(iv)

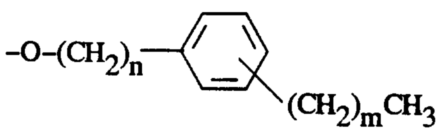 ; 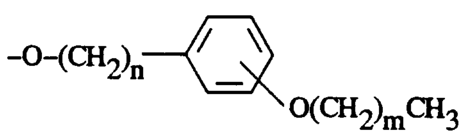 ;

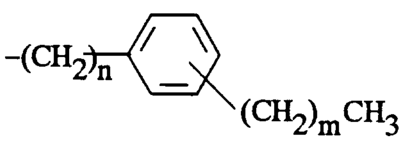 ; 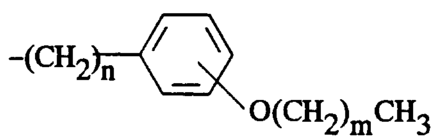 ;

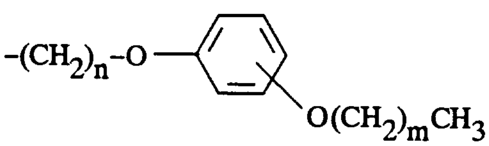 ; 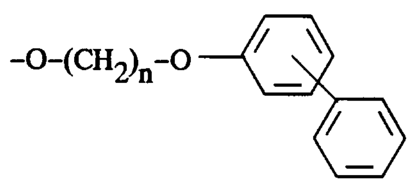 ;

ou

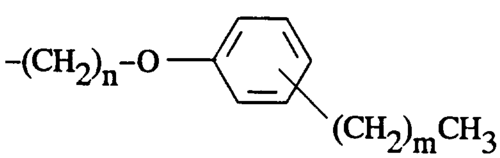 ;

où n est un nombre entier compris entre 1 et 25 et m est un nombre entier compris entre 0 et 24 et la somme de n et de m est inférieure ou égale à 25 ;
(v) phényle ;
(vi) phényle mono- ou polysubstitué par un groupe alkyle en $C_1$-$C_{20}$, un groupe alcoxy en $C_1$-$C_{20}$, un atome d'halogène, un groupe trifluorométhyle, un groupe phényle, un groupe phényle substitué ou un groupe benzyloxy ;
(vii) phénoxy ;

(viii) phénoxy mono- ou polysubstitué substitué par un groupe alkyle en $C_1$-$C_{20}$, un groupe alcoxy en $C_1$-$C_{20}$, un atome d'halogène, un groupe trifluorométhyle, un groupe phényle, un groupe phényle substitué ou un groupe benzyloxy ;

(ix) naphtyloxy ;

(x) naphtyloxy mono- ou polysubstitué substitué par un groupe alkyle en $C_1$-$C_{20}$, un groupe alcoxy en $C_1$-$C_{20}$ ou un atome d'halogène ;

(xi) $-O-(CH_2)_r-O-((CH_2)_pO)_t-(CH_2)_a-W$ où W représente un groupe méthyle ou phényle éventuellement substitué par un groupe alkyle en $C_1$-$C_3$, un groupe alcoxy en $C_1$-$C_3$ ou un groupe phényle, r, p, t et a représentent des nombres entiers tels que l'expression r+(p+1)t+a donne également un nombre entier dont la valeur est comprise entre 3 et 20 ; r est supérieur ou égal à 2 ; p est supérieur ou égal à 2 ; t est supérieur ou égal à zéro; et a est supérieur ou égal à zéro ;

(B) i est un nombre entier compris entre 1 et 3 et j est un nombre entier compris entre 1 et 6 ;

(C) Q représente $-OR_2$,

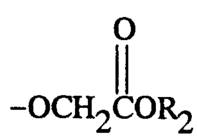 ou 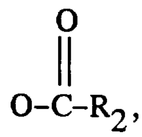

où $R_2$ est un atome d'hydrogène, un groupe alkyle en $C_1$-$C_6$ ou un groupe alcényle en $C_1$-$C_6$ ;

(D) Y représente un radical divalent

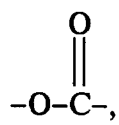

$-OCH_2-$ ou

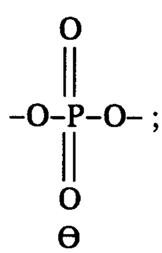

(E) $R_3$ représente un ou plusieurs substituants alkyle en $C_1$-$C_5$, alcoxy en $C_1$-$C_5$ ou halogènes du cycle aromatique ;

(F) $-CH_2A$ peut être en position ortho, méta ou para, où A est un noyau hétérocyclique aromatique à 5 à 7 éléments contenant au moins un atome d'azote et éventuellement un ou plusieurs autres atomes d'azote ou de soufre.

**8.** Procédé selon la revendication 1, dans lequel l'anticorps monoclonal ou polyclonal dirigé contre l'interféron γ est en combinaison avec un antagoniste du facteur d'activation des plaquettes de formule :

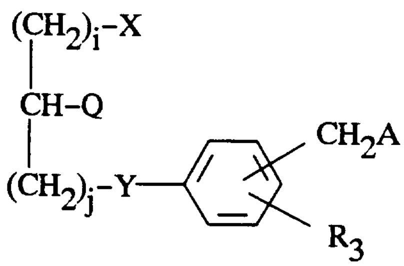

Formule II

dans laquelle

(A) X est

(i) alkyle en $C_1$-$C_{24}$ ;
(ii) alcoxy en $C_1$-$C_{24}$ ;
(iii) carbamoyloxy en $C_1$-$C_{24}$ ;
(iv)

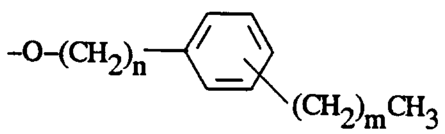 ; 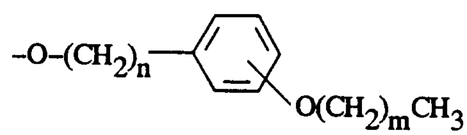 ;

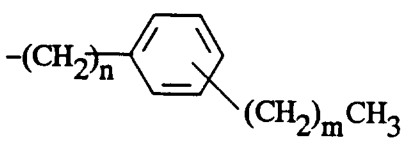 ; 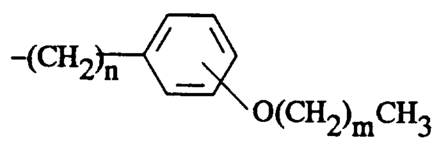 ;

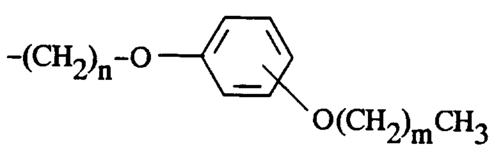 ; 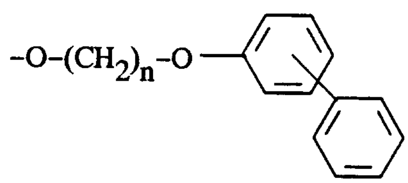 ;

ou

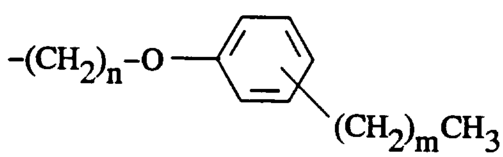 ;

où n est un nombre entier compris entre 1 et 25 et m est un nombre entier compris entre 0 et 24 et la somme de n et de m est inférieure ou égale à 25 ;
(v) phényle ;
(vi) phényle mono- ou polysubstitué par un groupe alkyle en $C_1$-$C_{20}$, un groupe alcoxy en $C_1$-$C_{20}$, un atome d'halogène, un groupe trifluorométhyle, un groupe phényle, un groupe phényle substitué ou un groupe benzyloxy ;
(vii) phénoxy ;

(viii) phénoxy mono- ou polysubstitué substitué par un groupe alkyle en $C_1$-$C_{20}$, un groupe alcoxy en $C_1$-$C_{20}$, un atome d'halogène, un groupe trifluorométhyle, un groupe phényle, un groupe phényle substitué ou un groupe benzyloxy ;

(ix) naphtyloxy ;

(x) naphtyloxy mono- ou polysubstitué substitué par un groupe alkyle en $C_1$-$C_{20}$, un groupe alcoxy en $C_1$-$C_{20}$ ou un atome d'halogène ;

(xi) $-O-(CH_2)_r-O-((CH_2)_pO)_t-(CH_2)_a-W$ où W représente un groupe méthyle ou phényle éventuellement substitué par un groupe alkyle en $C_1$-$C_3$, un groupe alcoxy en $C_1$-$C_3$ ou un groupe phényle, r, p, t et a représentent des nombres entiers tels que l'expression r+(p+1)t+a donne également un nombre entier dont la valeur est comprise entre 3 et 20 ; r est supérieur ou égal à 2 ; p est supérieur ou égal à 2 ; t est supérieur ou égal à zéro ; et a est supérieur ou égal à zéro ;

(B) i est un nombre entier compris entre 1 et 3 et j est un nombre entier compris entre 1 et 6 ;

(C) Q représente $-OR_2$,

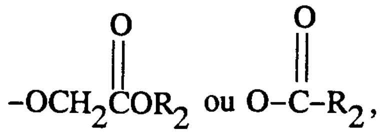

où $R_2$ est un atome d'hydrogène, un groupe alkyle en $C_1$-$C_6$ ou un groupe alcényle en $C_1$-$C_6$ ;

(D) Y représente un radical divalent

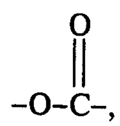

$-OCH_2-$ ou

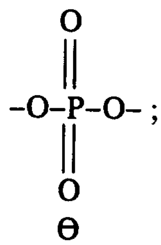

(E) $R_3$ représente un ou plusieurs substituants alkyle en $C_1$-$C_5$, alcoxy en $C_1$-$C_5$ ou halogènes du cycle aromatique ;

(F) $-CH_2A$ peut être en position ortho, méta ou para, où A est un noyau hétérocyclique aromatique à 5 à 7 éléments contenant au moins un atome d'azote et éventuellement un ou plusieurs autres atomes d'azote ou de soufre.

**9.** Procédé selon la revendication 1, dans lequel l'anticorps monoclonal ou polyclonal dirigé contre les lipopolysaccharides de paroi cellulaire bactérienne est en combinaison avec un antagoniste du facteur d'activation des plaquettes de formule :

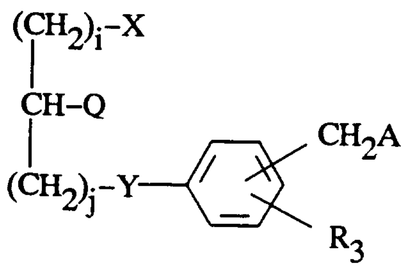

Formule II

dans laquelle

(A) X est

(i) alkyle en $C_1$-$C_{24}$ ;
(ii) alcoxy en $C_1$-$C_{24}$ ;
(iii) carbamoyloxy en $C_1$-$C_{24}$ ;
(iv)

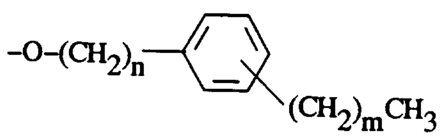 ; 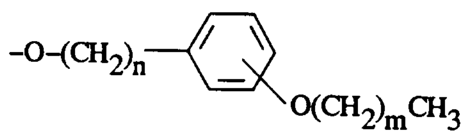 ;

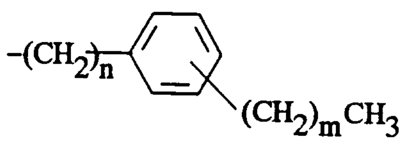 ; 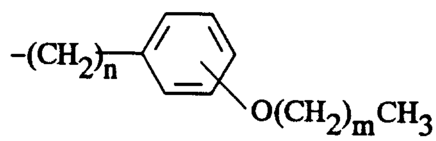 ;

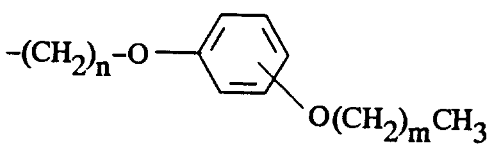 ; 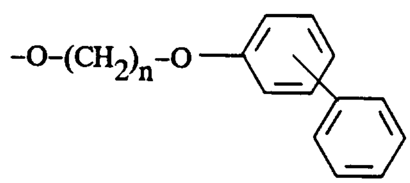 ;

ou

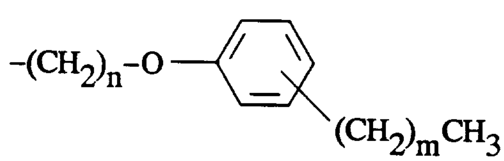 ;

où n est un nombre entier compris entre 1 et 25 et m est un nombre entier compris entre 0 et 24 et la somme de n et de m est inférieure ou égale à 25 ;
(v) phényle ;
(vi) phényle mono- ou polysubstitué par un groupe alkyle en $C_1$-$C_{20}$, un groupe alcoxy en $C_1$-$C_{20}$, un atome d'halogène, un groupe trifluorométhyle, un groupe phényle, un groupe phényle substitué ou un

groupe benzyloxy ;
(vii) phénoxy ;
(viii) phénoxy mono- ou polysubstitué substitué par un groupe alkyle en $C_1$-$C_{20}$, un groupe alcoxy en $C_1$-$C_{20}$, un atome d'halogène, un groupe trifluorométhyle, un groupe phényle, un groupe phényle substitué ou un groupe benzyloxy ;
(ix) naphtyloxy ;
(x) naphtyloxy mono- ou polysubstitué substitué par un groupe alkyle en $C_1$-$C_{20}$, un groupe alcoxy en $C_1$-$C_{20}$ ou un atome d'halogène ;
(xi) $-O-(CH_2)_r-O-((CH_2)_pO)_t-(CH_2)_a-W$ où W représente un groupe méthyle ou phényle éventuellement substitué par un groupe alkyle en $C_1$-$C_3$, un groupe alcoxy en $C_1$-$C_3$ ou un groupe phényle, r, p, t et a représentent des nombres entiers tels que l'expression r+(p+1)t+a donne également un nombre entier dont la valeur est comprise entre 3 et 20 ; r est supérieur ou égal à 2 ; p est supérieur ou égal à 2 ; t est supérieur ou égal à zéro ; et a est supérieur ou égal à zéro ;

(B) i est un nombre entier compris entre 1 et 3 et j est un nombre entier compris entre 1 et 6 ;
(C) Q représente $-OR_2$,

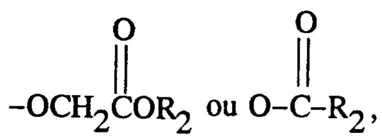

où $R_2$ est un atome d'hydrogène, un groupe alkyle en $C_1$-$C_6$ ou un groupe alcényle en $C_1$-$C_6$ ;
(D) Y représente un radical divalent

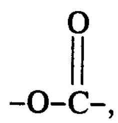

$-OCH_2-$ ou

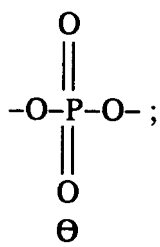

(E) $R_3$ représente un ou plusieurs substituants alkyle en $C_1$-$C_5$, alcoxy en $C_1$-$C_5$ ou halogènes du cycle aromatique ;
(F) $-CH_2A$ peut être en position <u>ortho</u>, <u>méta</u> ou <u>para</u>, où A est un noyau hétérocyclique aromatique à 5 à 7 éléments contenant au moins un atome d'azote et éventuellement un ou plusieurs autres atomes d'azote ou de soufre.

**10.** Utilisation de a) une quantité efficace du point de vue thérapeutique d'un antagoniste du facteur d'activation des plaquettes et de b) un ou plusieurs anticorps monoclonaux ou polyclonaux dirigés contre le facteur de nécrose tumorale α, l'interleukine-1β, l'interféron γ ou les lipopolysaccharides de paroi cellulaire bactérienne dans la préparation d'un médicament pour le traitement du choc endotoxique.

**11.** Utilisation selon la revendication 10, dans laquelle l'antagoniste du facteur d'activation des plaquettes est de formule :

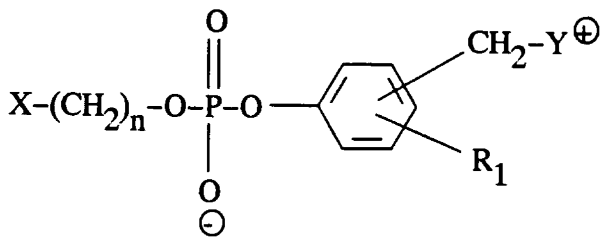

Formule I

dans lequel

X est un cycle phényle ou naphtyle éventuellement substitué en une position quelconque par un ou plusieurs des substituants :

i) $-R_2$ dans lequel $R_2$ représente un groupe alkyle en $C_1$-$C_{25}$, un groupe alcényle en $C_1$-$C_{25}$, un groupe alcoxy en $C_1$-$C_{25}$, un groupe alcényloxy en $C_1$-$C_{25}$, un groupe thioalkyle en $C_1$-$C_{25}$, un groupe phényle, un groupe phénoxy, un groupe phényle substitué ou un groupe phénoxy substitué dans lesquels les substituants sont un groupe alkyle en $C_1$-$C_{20}$, un groupe alcoxy en $C_1$-$C_{20}$, un atome d'halogène ou un groupe trifluorométhyle ;
ii) un atome d'hydrogène, un atome d'halogène, un groupe trifluorométhyle, un groupe cyano ou un groupe nitro ;
iii) $-CO_2R_3$, $-CONHR_3$, -CHO, $OCONHR_3$, ou $-NHCOR_3$ dans lesquels $R_3$ représente un groupe alkyle en $C_1$-$C_{25}$, un groupe alcényle en $C_1$-$C_{25}$, un groupe phényle ou un groupe phényle substitué dans lequel les substituants sont un groupe alkyle en $C_1$-$C_{20}$, un groupe alcoxy en $C_1$-$C_{20}$, un atome d'halogène ou un groupe trifluorométhyle ;

$R_1$ représente un ou plusieurs substituants du cycle aromatique qui peuvent se trouver en une position quelconque et sont un atome d'hydrogène, un groupe alkyle en $C_1$-$C_5$, un groupe alcoxy en $C_1$-$C_5$, ou un atome d'halogène ;
$-CH_2-Y$ est un substituant unique du cycle aromatique qui peut occuper une position quelconque, dans lequel Y est un hétérocycle aromatique mono- ou bicyclique comprenant des cycles de 5 à 7 éléments contenant au moins un atome d'azote qui est lié au groupe méthylène et éventuellement un ou plusieurs autres atomes d'azote ou de soufre ; et n représente un nombre entier de valeur 0 ou 1 ;
et où les anticorps monoclonaux ou polyclonaux sont dirigés contre le facteur de nécrose tumorale $\alpha$.

**12.** Utilisation selon la revendication 10, dans laquelle l'antagoniste du facteur d'activation des plaquettes est de formule :

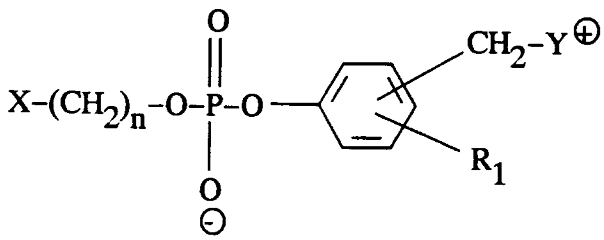

Formule I

dans lequel

X est un cycle phényle ou naphtyle éventuellement substitué en une position quelconque par un ou plusieurs des substituants :

i) $-R_2$ dans lequel $R_2$ représente un groupe alkyle en $C_1$-$C_{25}$, un groupe alcényle en $C_1$-$C_{25}$, un groupe alcoxy en $C_1$-$C_{25}$, un groupe alcényloxy en $C_1$-$C_{25}$, un groupe thioalkyle en $C_1$-$C_{25}$, un groupe phényle, un groupe phénoxy, un groupe phényle substitué ou un groupe phénoxy substitué dans lesquels les substituants sont un groupe alkyle en $C_1$-$C_{20}$, un groupe alcoxy en $C_1$-$C_{20}$, un atome d'halogène ou un groupe trifluorométhyle ;
ii) un atome d'hydrogène, un atome d'halogène, un groupe trifluorométhyle, un groupe cyano ou un groupe nitro ;
iii) $-CO_2R_3$, $-CONHR_3$, -CHO, $OCONHR_3$, ou $-NHCOR_3$ dans lesquels $R_3$ représente un groupe alkyle en $C_1$-$C_{25}$, un groupe alcényle en $C_1$-$C_{25}$, un groupe phényle ou un groupe phényle substitué dans lequel les substituants sont un groupe alkyle en $C_1$-$C_{20}$, un groupe alcoxy en $C_1$-$C_{20}$, un atome d'halogène ou un groupe trifluorométhyle ;

$R_1$ représente un ou plusieurs substituants du cycle aromatique qui peuvent se trouver en une position quelconque et sont un atome d'hydrogène, un groupe alkyle en $C_1$-$C_5$, un groupe alcoxy en $C_1$-$C_5$, ou un atome d'halogène ;
$-CH_2$-Y est un substituant unique du cycle aromatique qui peut occuper une position quelconque, dans lequel Y est un hétérocycle aromatique mono- ou bicyclique comprenant des cycles de 5 à 7 éléments contenant au moins un atome d'azote qui est lié au groupe méthylène et éventuellement un ou plusieurs autres atomes d'azote ou de soufre ; et n représente un nombre entier de valeur 0 ou 1 ;
et où les anticorps monoclonaux ou polyclonaux sont dirigés contre l'interleukine-1β.

13. Utilisation selon la revendication 10, dans laquelle l'antagoniste du facteur d'activation des plaquettes est de formule :

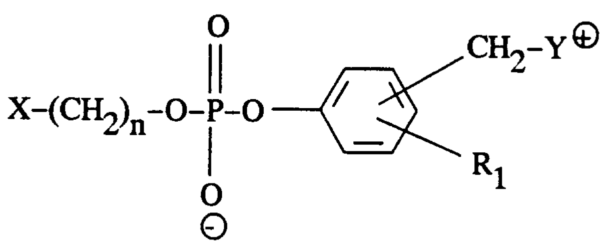

Formule I

dans lequel

X est un cycle phényle ou naphtyle éventuellement substitué en une position quelconque par un ou plusieurs des substituants :

i) $-R_2$ dans lequel $R_2$ représente un groupe alkyle en $C_1$-$C_{25}$, un groupe alcényle en $C_1$-$C_{25}$, un groupe alcoxy en $C_1$-$C_{25}$, un groupe alcényloxy en $C_1$-$C_{25}$, un groupe thioalkyle en $C_1$-$C_{25}$, un groupe phényle, un groupe phénoxy, un groupe phényle substitué ou un groupe phénoxy substitué dans lesquels les substituants sont un groupe alkyle en $C_1$-$C_{20}$, un groupe alcoxy en $C_1$-$C_{20}$, un atome d'halogène ou un groupe trifluorométhyle ;
ii) un atome d'hydrogène, un atome d'halogène, un groupe trifluorométhyle, un groupe cyano ou un groupe nitro ;
iii) $-CO_2R_3$, $-CONHR_3$, -CHO, $OCONHR_3$, ou $-NHCOR_3$ dans lesquels $R_3$ représente un groupe alkyle en $C_1$-$C_{25}$, un groupe alcényle en $C_1$-$C_{25}$, un groupe phényle ou un groupe phényle substitué dans lequel les substituants sont un groupe alkyle en $C_1$-$C_{20}$, un groupe alcoxy en $C_1$-$C_{20}$, un atome d'halogène ou un groupe trifluorométhyle ;

$R_1$ représente un ou plusieurs substituants du cycle aromatique qui peuvent se trouver en une position quelconque et sont un atome d'hydrogène, un groupe alkyle en $C_1$-$C_5$, un groupe alcoxy en $C_1$-$C_5$, ou un atome d'halogène ;

-$CH_2$-Y est un substituant unique du cycle aromatique qui peut occuper une position quelconque, dans lequel Y est un hétérocycle aromatique mono- ou bicyclique comprenant des cycles de 5 à 7 éléments contenant au moins un atome d'azote qui est lié au groupe méthylène et éventuellement un ou plusieurs autres atomes d'azote ou de soufre ; et n représente un nombre entier de valeur 0 ou 1 ;

et où les anticorps monoclonaux ou polyclonaux sont dirigés contre l'interféron $\gamma$.

**14.** Utilisation selon la revendication 10, dans laquelle l'antagoniste du facteur d'activation des plaquettes est de formule :

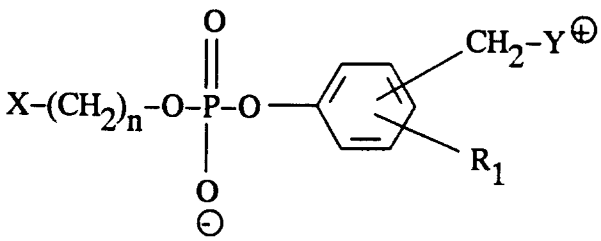

Formule I

dans lequel

X est un cycle phényle ou naphtyle éventuellement substitué en une position quelconque par un ou plusieurs des substituants :

i) -$R_2$ dans lequel $R_2$ représente un groupe alkyle en $C_1$-$C_{25}$, un groupe alcényle en $C_1$-$C_{25}$, un groupe alcoxy en $C_1$-$C_{25}$, un groupe alcényloxy en $C_1$-$C_{25}$, un groupe thioalkyle en $C_1$-$C_{25}$, un groupe phényle, un groupe phénoxy, un groupe phényle substitué ou un groupe phénoxy substitué dans lesquels les substituants sont un groupe alkyle en $C_1$-$C_{20}$, un groupe alcoxy en $C_1$-$C_{20}$, un atome d'halogène ou un groupe trifluorométhyle ;

ii) un atome d'hydrogène, un atome d'halogène, un groupe trifluorométhyle, un groupe cyano ou un groupe nitro ;

iii) -$CO_2R_3$, -$CONHR_3$, -CHO, $OCONHR_3$, ou -$NHCOR_3$ dans lesquels $R_3$ représente un groupe alkyle en $C_1$-$C_{25}$, un groupe alcényle en $C_1$-$C_{25}$, un groupe phényle ou un groupe phényle substitué dans lequel les substituants sont un groupe alkyle en $C_1$-$C_{20}$, un groupe alcoxy en $C_1$-$C_{20}$, un atome d'halogène ou un groupe trifluorométhyle ;

$R_1$ représente un ou plusieurs substituants du cycle aromatique qui peuvent se trouver en une position quelconque et sont un atome d'hydrogène, un groupe alkyle en $C_1$-$C_5$, un groupe alcoxy en $C_1$-$C_5$, ou un atome d'halogène ;

-$CH_2$-Y est un substituant unique du cycle aromatique qui peut occuper une position quelconque, dans lequel Y est un hétérocycle aromatique mono- ou bicyclique comprenant des cycles de 5 à 7 éléments contenant au moins un atome d'azote qui est lié au groupe méthylène et éventuellement un ou plusieurs autres atomes d'azote ou de soufre ; et n représente un nombre entier de valeur 0 ou 1 ;

et où les anticorps monoclonaux ou polyclonaux sont dirigés contre les lipopolysaccharides de paroi cellulaire bactérienne.

**15.** Utilisation selon la revendication 10, dans laquelle l'antagoniste du facteur d'activation des plaquettes est de formule :

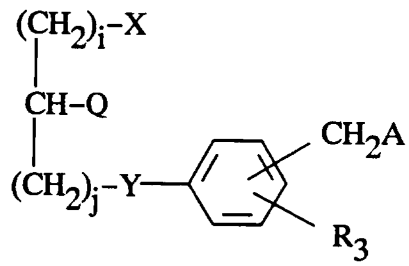

Formule II

dans laquelle

(A) X est

(i) alkyle en $C_1$-$C_{24}$ ;
(ii) alcoxy en $C_1$-$C_{24}$ ;
(iii) carbamoyloxy en $C_1$-$C_{24}$ ;
(iv)

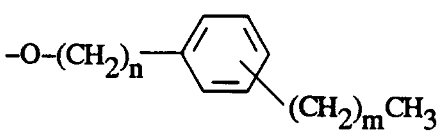 ; 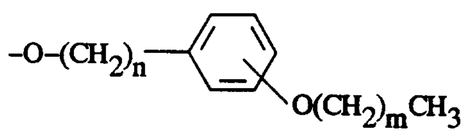 ;

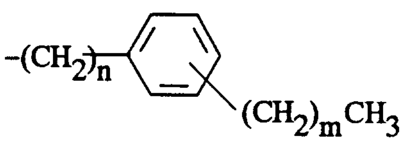 ; 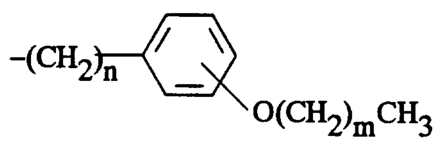 ;

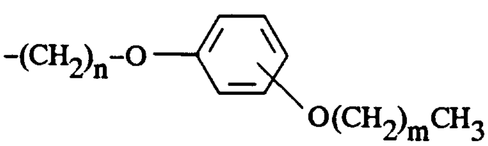 ; 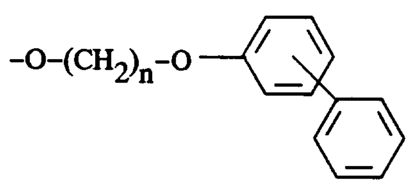 ;

ou

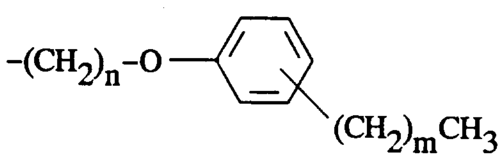 ;

où n est un nombre entier compris entre 1 et 25 et m est un nombre entier compris entre 0 et 24 et la somme de n et de m est inférieure ou égale à 25 ;
(v) phényle ;
(vi) phényle mono- ou polysubstitué par un groupe alkyle en $C_1$-$C_{20}$, un groupe alcoxy en $C_1$-$C_{20}$, un atome d'halogène, un groupe trifluorométhyle, un groupe phényle, un groupe phényle substitué ou un groupe benzyloxy ;
(vii) phénoxy ;

(viii) phénoxy mono- ou polysubstitué substitué par un groupe alkyle en $C_1$-$C_{20}$, un groupe alcoxy en $C_1$-$C_{20}$, un atome d'halogène, un groupe trifluorométhyle, un groupe phényle, un groupe phényle substitué ou un groupe benzyloxy ;

(ix) naphtyloxy ;

(x) naphtyloxy mono- ou polysubstitué substitué par un groupe alkyle en $C_1$-$C_{20}$, un groupe alcoxy en $C_1$-$C_{20}$ ou un atome d'halogène ;

(xi) $-O-(CH_2)_r-O-((CH_2)_pO)_t-(CH_2)_a-W$ où W représente un groupe méthyle ou phényle éventuellement substitué par un groupe alkyle en $C_1$-$C_3$, un groupe alcoxy en $C_1$-$C_3$ ou un groupe phényle, r, p, t et a représentent des nombres entiers tels que l'expression r+(p+1)t+a donne également un nombre entier dont la valeur est comprise entre 3 et 20 ; r est supérieur ou égal à 2 ; p est supérieur ou égal à 2 ; t est supérieur ou égal à zéro ; et a est supérieur ou égal à zéro ;

(B) i est un nombre entier compris entre 1 et 3 et j est un nombre entier compris entre 1 et 6 ;

(C) Q représente $-OR_2$,

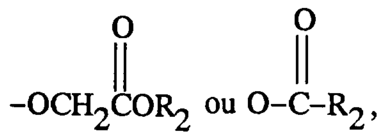

où $R_2$ est un atome d'hydrogène, un groupe alkyle en $C_1$-$C_6$ ou un groupe alcényle en $C_1$-$C_6$ ;

(D) Y représente un radical divalent

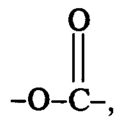

$-OCH_2-$ ou

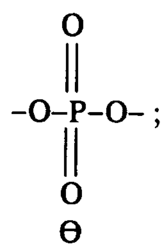

(E) $R_3$ représente un ou plusieurs substituants alkyle en $C_1$-$C_5$, alcoxy en $C_1$-$C_5$ ou halogènes du cycle aromatique ;

(F) $-CH_2A$ peut être en position ortho, méta ou para, où A est un noyau hétérocyclique aromatique à 5 à 7 éléments contenant au moins un atome d'azote et éventuellement un ou plusieurs autres atomes d'azote ou de soufre ;

et où les anticorps monoclonaux ou polyclonaux sont dirigés contre le facteur de nécrose tumorale $\alpha$.

**16.** Utilisation selon la revendication 10, dans laquelle l'antagoniste du facteur d'activation des plaquettes est de formule :

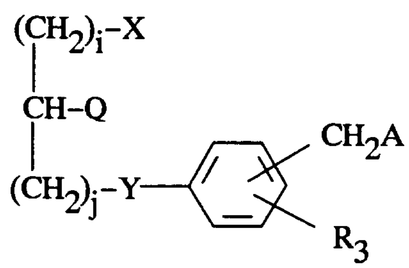

Formule II

dans laquelle

(A) X est

(i) alkyle en $C_1$-$C_{24}$ ;
(ii) alcoxy en $C_1$-$C_{24}$ ;
(iii) carbamoyloxy en $C_1$-$C_{24}$ ;
(iv)

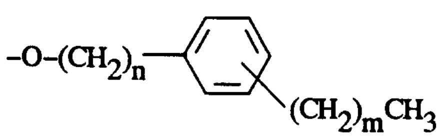 ; 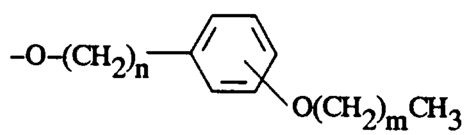 ;

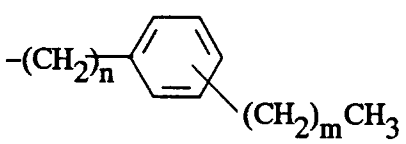 ; 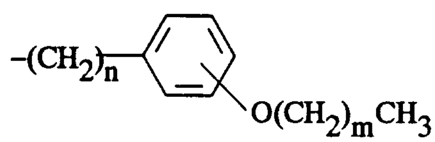 ;

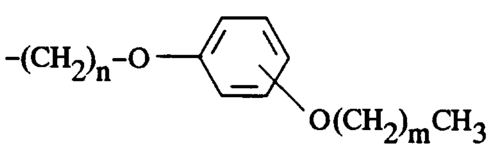 ; 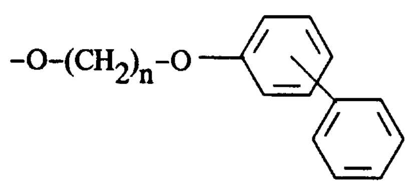 ;

ou

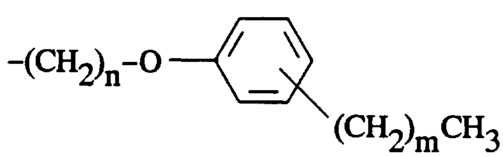 ;

où n est un nombre entier compris entre 1 et 25 et m est un nombre entier compris entre 0 et 24 et la somme de n et de m est inférieure ou égale à 25 ;
(v) phényle ;
(vi) phényle mono- ou polysubstitué par un groupe alkyle en $C_1$-$C_{20}$, un groupe alcoxy en $C_1$-$C_{20}$, un atome d'halogène, un groupe trifluorométhyle, un groupe phényle, un groupe phényle substitué ou un groupe benzyloxy ;
(vii) phénoxy ;

(viii) phénoxy mono- ou polysubstitué substitué par un groupe alkyle en $C_1$-$C_{20}$, un groupe alcoxy en $C_1$-$C_{20}$, un atome d'halogène, un groupe trifluorométhyle, un groupe phényle, un groupe phényle substitué ou un groupe benzyloxy ;

(ix) naphtyloxy ;

(x) naphtyloxy mono- ou polysubstitué substitué par un groupe alkyle en $C_1$-$C_{20}$, un groupe alcoxy en $C_1$-$C_{20}$ ou un atome d'halogène ;

(xi) $-O-(CH_2)_r-O-((CH_2)_pO)_t-(CH_2)_a-W$ où W représente un groupe méthyle ou phényle éventuellement substitué par un groupe alkyle en $C_1$-$C_3$, un groupe alcoxy en $C_1$-$C_3$ ou un groupe phényle, r, p, t et a représentent des nombres entiers tels que l'expression r+(p+1)t+a donne également un nombre entier dont la valeur est comprise entre 3 et 20 ; r est supérieur ou égal à 2 ; p est supérieur ou égal à 2 ; t est supérieur ou égal à zéro; et a est supérieur ou égal à zéro ;

(B) i est un nombre entier compris entre 1 et 3 et j est un nombre entier compris entre 1 et 6 ;

(C) Q représente $-OR_2$,

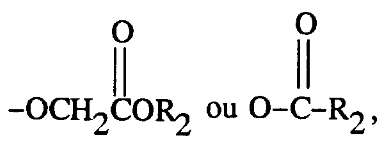

où $R_2$ est un atome d'hydrogène, un groupe alkyle en $C_1$-$C_6$ ou un groupe alcényle en $C_1$-$C_6$ ;

(D) Y représente un radical divalent

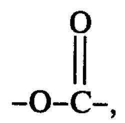

$-OCH_2-$ ou

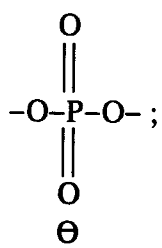

(E) $R_3$ représente un ou plusieurs substituants alkyle en $C_1$-$C_5$, alcoxy en $C_1$-$C_5$ ou halogènes du cycle aromatique ;

(F) $-CH_2A$ peut être en position ortho, méta ou para, où A est un noyau hétérocyclique aromatique à 5 à 7 éléments contenant au moins un atome d'azote et éventuellement un ou plusieurs autres atomes d'azote ou de soufre ; et où les anticorps monoclonaux ou polyclonaux sont dirigés contre l'interleukine-1β.

**17.** Utilisation selon la revendication 10, dans laquelle l'antagoniste du facteur d'activation des plaquettes est de formule :

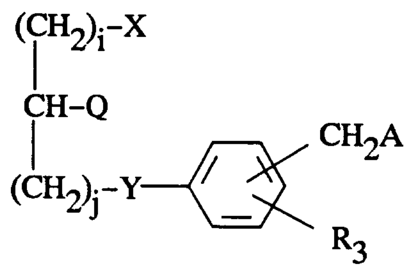

Formule II

dans laquelle

(A) X est

(i) alkyle en $C_1$-$C_{24}$ ;
(ii) alcoxy en $C_1$-$C_{24}$ ;
(iii) carbamoyloxy en $C_1$-$C_{24}$ ;
(iv)

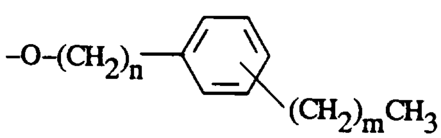 ; 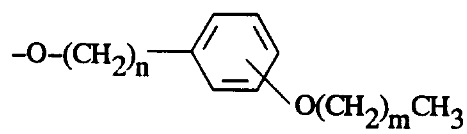 ;

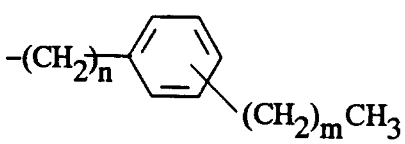 ; 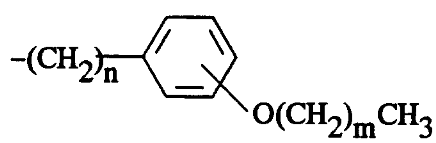 ;

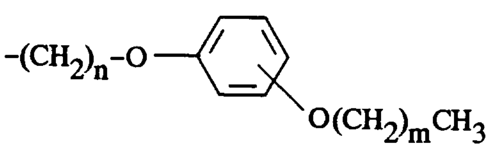 ; 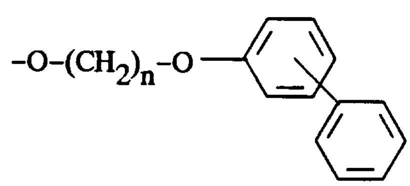 ;

ou

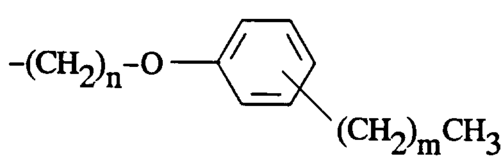 ;

où n est un nombre entier compris entre 1 et 25 et m est un nombre entier compris entre 0 et 24 et la somme de n et de m est inférieure ou égale à 25 ;
(v) phényle ;
(vi) phényle mono- ou polysubstitué par un groupe alkyle en $C_1$-$C_{20}$, un groupe alcoxy en $C_1$-$C_{20}$, un atome d'halogène, un groupe trifluorométhyle, un groupe phényle, un groupe phényle substitué ou un

groupe benzyloxy ;

(vii) phénoxy ;

(viii) phénoxy mono- ou polysubstitué substitué par un groupe alkyle en $C_1$-$C_{20}$, un groupe alcoxy en $C_1$-$C_{20}$, un atome d'halogène, un groupe trifluorométhyle, un groupe phényle, un groupe phényle substitué ou un groupe benzyloxy ;

(ix) naphtyloxy ;

(x) naphtyloxy mono- ou polysubstitué substitué par un groupe alkyle en $C_1$-$C_{20}$, un groupe alcoxy en $C_1$-$C_{20}$ ou un atome d'halogène ;

(xi) $-O-(CH_2)_r-O-((CH_2)_pO)_t-(CH_2)_a-W$ où W représente un groupe méthyle ou phényle éventuellement substitué par un groupe alkyle en $C_1$-$C_3$, un groupe alcoxy en $C_1$-$C_3$ ou un groupe phényle, r, p, t et a représentent des nombres entiers tels que l'expression r+(p+1)t+a donne également un nombre entier dont la valeur est comprise entre 3 et 20 ; r est supérieur ou égal à 2 ; p est supérieur ou égal à 2 ; t est supérieur ou égal à zéro ; et a est supérieur ou égal à zéro ;

(B) i est un nombre entier compris entre 1 et 3 et j est un nombre entier compris entre 1 et 6 ;

(C) Q représente $-OR_2$,

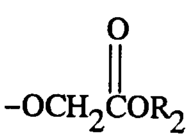 ou 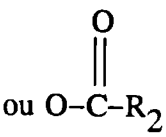,

où $R_2$ est un atome d'hydrogène, un groupe alkyle en $C_1$-$C_6$ ou un groupe alcényle en $C_1$-$C_6$ ;

(D) Y représente un radical divalent

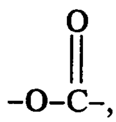,

$-OCH_2-$ ou

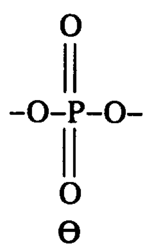 ;

(E) $R_3$ représente un ou plusieurs substituants alkyle en $C_1$-$C_5$, alcoxy en $C_1$-$C_5$ ou halogènes du cycle aromatique ;

(F) $-CH_2A$ peut être en position <u>ortho</u>, <u>méta</u> ou <u>para</u>, où A est un noyau hétérocyclique aromatique à 5 à 7 éléments contenant au moins un atome d'azote et éventuellement un ou plusieurs autres atomes d'azote ou de soufre ;

et où les anticorps monoclonaux ou polyclonaux sont dirigés contre l'interféron $\gamma$.

**18.** Utilisation selon la revendication 10, dans laquelle l'antagoniste du facteur d'activation des plaquettes est de formule :

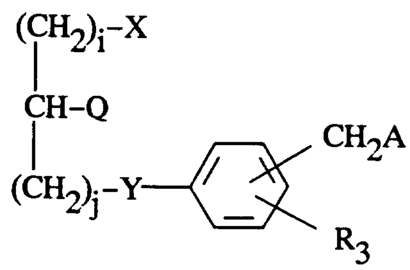

Formule II

dans laquelle

(A) X est

(i) alkyle en $C_1$-$C_{24}$ ;
(ii) alcoxy en $C_1$-$C_{24}$ ;
(iii) carbamoyloxy en $C_1$-$C_{24}$ ;
(iv)

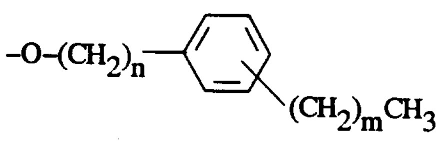 ; 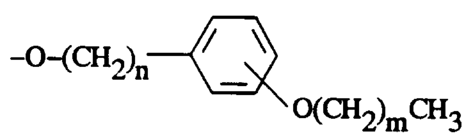 ;

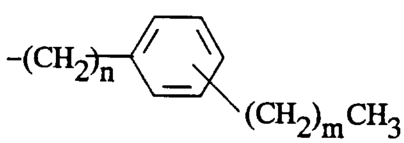 ; 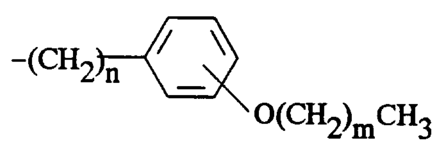 ;

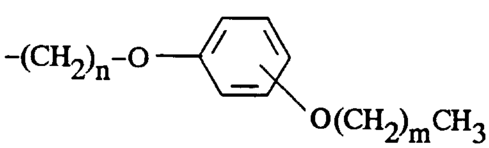 ; 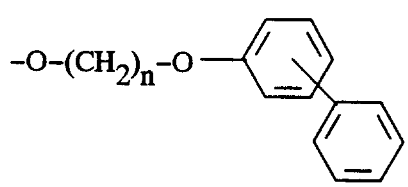 ;

ou

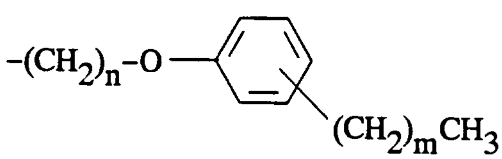 ;

où n est un nombre entier compris entre 1 et 25 et m est un nombre entier compris entre 0 et 24 et la somme de n et de m est inférieure ou égale à 25 ;
(v) phényle ;
(vi) phényle mono- ou polysubstitué par un groupe alkyle en $C_1$-$C_{20}$, un groupe alcoxy en $C_1$-$C_{20}$, un atome d'halogène, un groupe trifluorométhyle, un groupe phényle, un groupe phényle substitué ou un

groupe benzyloxy ;

(vii) phénoxy ;

(viii) phénoxy mono- ou polysubstitué substitué par un groupe alkyle en $C_1$-$C_{20}$, un groupe alcoxy en $C_1$-$C_{20}$, un atome d'halogène, un groupe trifluorométhyle, un groupe phényle, un groupe phényle substitué ou un groupe benzyloxy ;

(ix) naphtyloxy ;

(x) naphtyloxy mono- ou polysubstitué substitué par un groupe alkyle en $C_1$-$C_{20}$, un groupe alcoxy en $C_1$-$C_{20}$ ou un atome d'halogène ;

(xi) -O-$(CH_2)_r$-O-$((CH_2)_pO)_t$-$(CH_2)_a$-W où W représente un groupe méthyle ou phényle éventuellement substitué par un groupe alkyle en $C_1$-$C_3$, un groupe alcoxy en $C_1$-$C_3$ ou un groupe phényle, r, p, t et a représentent des nombres entiers tels que l'expression r+(p+1)t+a donne également un nombre entier dont la valeur est comprise entre 3 et 20 ; r est supérieur ou égal à 2 ; p est supérieur ou égal à 2 ; t est supérieur ou égal à zéro ; et a est supérieur ou égal à zéro ;

(B) i est un nombre entier compris entre 1 et 3 et j est un nombre entier compris entre 1 et 6 ;

(C) Q représente -$OR_2$,

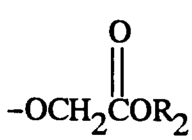 ou 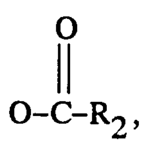

où $R_2$ est un atome d'hydrogène, un groupe alkyle en $C_1$-$C_6$ ou un groupe alcényle en $C_1$-$C_6$ ;

(D) Y représente un radical divalent

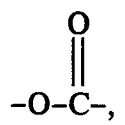

-$OCH_2$- ou

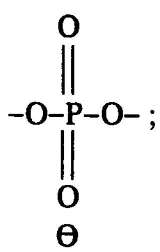

(E) $R_3$ représente un ou plusieurs substituants alkyle en $C_1$-$C_5$, alcoxy en $C_1$-$C_5$ ou halogènes du cycle aromatique ;

(F) -$CH_2$A peut être en position <u>ortho</u>, <u>méta</u> ou <u>para</u>, où A est un noyau hétérocyclique aromatique à 5 à 7 éléments contenant au moins un atome d'azote et éventuellement un ou plusieurs autres atomes d'azote ou de soufre ;

et où les anticorps monoclonaux ou polyclonaux sont dirigés contre les lipopolysaccharides de paroi cellulaire bactérienne.